(19) Europäisches Patentamt
European Patent Office
Office européen des brevets

(11) **EP 4 269 415 A1**

(12) **EUROPEAN PATENT APPLICATION**
published in accordance with Art. 153(4) EPC

(43) Date of publication:
**01.11.2023 Bulletin 2023/44**

(21) Application number: **21910951.9**

(22) Date of filing: **23.12.2021**

(51) International Patent Classification (IPC):
***C07D 495/04*** *(2006.01)* ***A61K 31/519*** *(2006.01)*
***A61K 31/5377*** *(2006.01)* ***A61K 31/5383*** *(2006.01)*
***A61P 35/00*** *(2006.01)* ***C07D 519/00*** *(2006.01)*

(52) Cooperative Patent Classification (CPC):
**A61K 31/519; A61K 31/5377; A61K 31/5383; A61P 35/00; C07D 495/04; C07D 519/00**

(86) International application number:
**PCT/JP2021/047849**

(87) International publication number:
**WO 2022/138812 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.12.2020 JP 2020214655**

(71) Applicant: **Modulus Discovery, Inc.**
**Tokyo 102-0074 (JP)**

(72) Inventors:
- **TAKAHASHI Taisuke**
  **Tokyo 102-0074 (JP)**
- **SHIMIZU Takafumi**
  **Tokyo 102-0074 (JP)**
- **TERADA Yoh**
  **Tokyo 102-0074 (JP)**
- **URAKAMI Takeo**
  **Tokyo 102-0074 (JP)**
- **SINKO William**
  **Dover, Kent County, Delaware 19901 (US)**

(74) Representative: **Hoffmann Eitle**
**Patent- und Rechtsanwälte PartmbB**
**Arabellastraße 30**
**81925 München (DE)**

(54) **TETRAHYDRO THIENOPYRIDINE SULFONAMIDE COMPOUND**

(57) Provided is a tetrahydrothienopyrimidinesulfonamide compound having a PARG inhibitory action useful as an active ingredient of a pharmaceutical composition for treating cancer, a pharmaceutical composition for enhancing an effect of an anticancer agent, and/or a pharmaceutical composition for enhancing an effect of radiotherapy. The tetrahydrothienopyrimidinesulfonamide compound of the present invention is a compound of Formula (I) or a salt thereof.

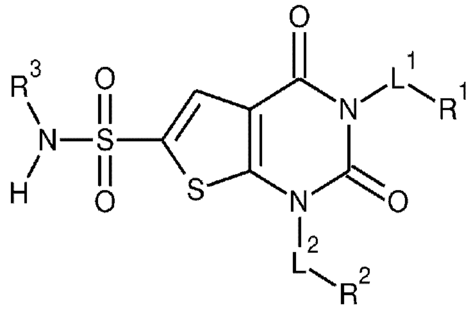

(I)

(In Formula, $L^1$ is methylene or the like, $L^2$ is methylene or the like, $R^1$ is an aromatic heterocyclic group which may have a substituent or the like, $R^2$ is a non-aromatic heterocyclic group which may have a substituent, an aromatic heterocyclic group which may have a substituent, or the like, and $R^3$ is 1-methylcyclopropyl or the like.)

EP 4 269 415 A1

## Description

Technical Field

The present invention relates to a tetrahydrothienopyrimidinesulfonamide compound useful as an active ingredient of a pharmaceutical composition, for example, a pharmaceutical composition for treating cancer or a pharmaceutical composition for enhancing an effect of an anticancer agent.

Related Art

DNA repair mechanism of cells contributes to gene stabilization and suppression of tumorigenesis. Mutations in tumor suppressor genes that control DNA repair, such as BRCA1, ATM, CHK2, etc., are often found in cancer cells. For example, in a case where DNA single-strand breaks occur due to oxidative stress, poly (ADP-ribose) polymerase (PARP) promotes poly ADP-ribosylation (PARylation) to recruit DNA repair proteins such as XRCC1, etc..

PARP inhibitors are known to induce apoptosis by suppressing PARylation in cancer cells with mutations in the DNA repair mechanism, and exhibit anticancer effects. In recent years, it has been also clinically confirmed that PARP inhibitors are effective as anticancer agents and effect enhancers of anticancer agents.

On the other hand, poly (ADP-ribose) glycohydrolase (PARG), which is an enzyme that specifically decomposes poly (ADP-ribose), plays a part of the DNA repair mechanism by functioning in conjunction with PARP. The PARG inhibitors are also expected to be utilized as anticancer agents and effect enhancers of anticancer agents (Patent Documents 1 and 2).

In addition, some tetrahydrothienopyrimidine compounds are known as compounds having an acetyl-CoA carboxylase (ACC) inhibitory action (Patent Documents 3, 4, and 5).

Prior Art Document

Patent Document

Patent Document 1: PCT International Publication No. WO 2018/237296
Patent Document 2: PCT International Publication No. WO 2016/092326
Patent Document 3: PCT International Publication No. WO 2017/075056
Patent Document 4: PCT International Publication No. WO 2017/147161
Patent Document 5: PCT International Publication No. WO 2018/171698

Disclosure of the Invention

Problems to be Solved by the Invention

The present invention is to provide a tetrahydrothienopyrimidinesulfonamide compound having a PARG inhibitory action useful as an active ingredient of a pharmaceutical composition for treating cancer, a pharmaceutical composition for enhancing an effect of an anticancer agent, and/or a pharmaceutical composition for enhancing an effect of radiotherapy.

Means for Solving the Problem

The present invention has the following aspects.

[1] A compound of Formula (1) or a salt thereof.

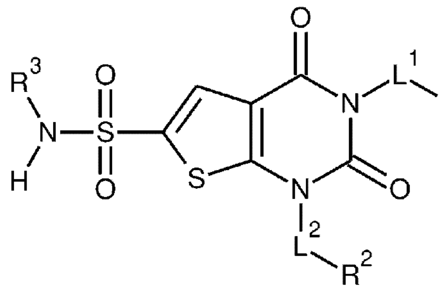

(I)

(In Formula,

$L^1$ is $C_{1-4}$ alkylene;
$L^2$ is a single bond, $C_{1-4}$ alkylene, $C_{2-4}$ alkenylene, $C_{2-4}$ alkynylene, or $C_{1-4}$ alkylene-O-;
$R^1$ is an aromatic heterocyclic group which may have a substituent, or a non-aromatic heterocyclic group which may have a substituent;
$R^2$ is -H, amino, $C_{3-6}$ cycloalkyl which may have a substituent, an aryl which may have a substituent, a non-aromatic heterocyclic group which may have a substituent, or an aromatic heterocyclic group which may have a substituent; and
$R^3$ is $C_{3-6}$ cycloalkyl which may be substituted with one or more groups selected from the group consisting of $C_{1-4}$ alkyl and cyano, or $C_{1-4}$ alkyl.)

[2] The compound or a salt thereof according to [1], wherein $R^1$ is an aromatic heterocyclic group which may have a substituent, and
$R^3$ is $C_{3-6}$ cycloalkyl which may be substituted with $C_{1-4}$ alkyl, or $C_{1-4}$ alkyl.
[3] The compound or a salt thereof according to [1] or [2], wherein $R^3$ is 1-methylcyclopropyl.
[4] The compound or a salt thereof according to any one of [1] to [3], wherein $L^1$ is methylene.
[5] The compound or a salt thereof according to any one of [1] to [4], wherein $L^2$ is methylene.
[6] The compound or a salt thereof according to any one of [1] to [5], wherein $R^1$ is 1-methylpyrazol-4-yl or 2-methylthiazol-5-yl.
[7] The compound or a salt thereof according to [6], wherein $R^1$ is 1-methylpyrazol-4-yl.
[8] The compound or a salt thereof according to any one of [1] to [7], wherein $R^2$ is a non-aromatic heterocyclic group which may have a substituent or an aromatic heterocyclic group which may have a substituent.
[9] 1-((2,4-Dimethylthiazol-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,

1-((1,3-Dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,
1,3-Bis((1-methyl-1H-pyrazol-4-yl)methyl)-N-(1 -methylcyclopropyl)-2,4-dioxo-1,2,3,4-tetrahydrothieno[2,3-d]pyrimidine-6-sulfonamide or a salt thereof,
1-((1H-Indazol-6-yl)methyl)-3-((1-methyl-1H-pyrazol-4-yl)methyl)-N-(1- methylcyclopropyl)-2,4-dioxo-1,2,3,4-tetrahydrothieno[2,3-d]pyrimidine-6-sulfonamide or a salt thereof,
1-((Imidazo[1,5-a]pyridin-6-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,
1-((2-(Methoxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno [2,3-d] pyrimidine-6-sulfonamide or a salt thereof, or
1-((2-(hydroxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof.

[10] A pharmaceutical composition comprising the compound or a salt thereof according to [1] or [9], and a pharmaceutically acceptable excipient.
[11] The pharmaceutical composition according to [10], wherein the pharmaceutical composition is a pharmaceutical composition for treating cancer, a pharmaceutical composition for enhancing an effect of an anticancer agent, or a pharmaceutical composition for enhancing an effect of radiotherapy.
[12] Use of the compound or a salt thereof according to [1] or [9] for manufacturing a pharmaceutical composition for treating cancer, a pharmaceutical composition for enhancing an effect of an anticancer agent, or a pharmaceutical composition for enhancing an effect of radiotherapy.

[13] Use of the compound or a salt thereof according to [1] or [9] for treating cancer, enhancing an effect of an anticancer agent, or enhancing an effect of radiotherapy.

[14] The compound or a salt thereof according to [1] or [9] for treating cancer, enhancing an effect of an anticancer agent, or enhancing an effect of radiotherapy.

[15] A method for treating cancer, enhancing an effect of an anticancer agent, or enhancing an effect of radiotherapy by administering an effective amount of the compound or a salt thereof according to [1] or [9] to a subject.

A "subject" is a human or another animal in need of treatment or enhancement of an effect thereof, and in one aspect, a human in need of treatment or enhancement of an effect thereof.

Unless otherwise described, in a case where symbols in one chemical formula in the present specification are also used in another chemical formula, the same symbols have the same meanings.

Advantageous Effects of Invention

The tetrahydrothienopyrimidinesulfonamide compound or a salt thereof of the present invention (also simply referred to as the "tetrahydrothienopyrimidinesulfonamide compound of the present invention" or the "compound of Formula (I)") has a PARG inhibitory action, and can be used as an active ingredient of a pharmaceutical composition for treating cancer, a pharmaceutical composition for enhancing an effect of an anticancer agent, and/or a pharmaceutical composition for enhancing an effect of radiotherapy. Embodiments of the Invention

In the present specification, the term "which may have a substituent" means which is unsubstituted or which has one or more substituents. In a case of having a substituent, it is possible to have a plurality of substituents as long as chemical structures are allowed. In the case of having the plurality of substituents, individual substituents may be the same or different from each other.

In the present specification, "alkyl" includes linear alkyl and branched alkyl. Examples of $C_{1-4}$ alkyl include methyl, ethyl, n-propyl, isopropyl, n-butyl, sec-butyl, and tert-butyl. In one aspect, an example thereof includes methyl or ethyl, and in another aspect, an example thereof includes methyl.

In the present specification, "cycloalkyl" is a monocyclic or polycyclic cycloalkyl. Examples of $C_{3-6}$ cycloalkyl include cyclopropyl, cyclobutyl, cyclopentyl, cyclohexyl, and bicyclo[1.1.1]pentanyl.

In the present specification, "aryl" is an aromatic hydrocarbon ring of $C_{6-14}$. In one aspect, an example thereof includes phenyl or naphthyl, and in another aspect, an example thereof includes phenyl.

In the present specification, a "non-aromatic heterocyclic group" is a monovalent group of a monocyclic non-aromatic heterocycle or a bicyclic non-aromatic heterocycle with a 5- to 10-membered ring containing one, two, three, or four identical or different hetero atoms selected from the group consisting of nitrogen, oxygen, and sulfur. Some of bonds that constitute the non-aromatic heterocycle may be unsaturated bonds. A part of the bicyclic non-aromatic heterocyclic group may have aromaticity. Examples thereof include aspects in a state where the monocyclic non-aromatic heterocycle with a 5- and 6-membered ring is fused with a benzene ring, a pyrrole ring, a furan ring, a thiophene ring, a pyrazole ring, an imidazole ring, an oxazole ring, a thiazole ring, or a pyridine ring, and a state where at least a partially unsaturated hydrocarbon ring is fused with a pyrrole ring, a furan ring, a thiophene ring, a pyrazole ring, an imidazole ring, an oxazole ring, a thiazole ring, or a pyridine ring. Examples of the non-aromatic heterocyclic group include aziridinyl, azetidinyl, pyrrolidinyl, piperidinyl, dihydropyridyl, oxetanyl, tetrahydrofuryl, dihydrofuryl, tetrahydropyranyl, dihydropyranyl, tetrahydrothienyl, tetrahydrothiopyranyl, dihydrothiopyranyl, piperazinyl, dihydropyrazinyl, morpholinyl, thiomorpholinyl, dihydroindolyl, dihydroisoindolyl, dihydrobenzofuryl, dihydroisobenzofuryl, tetrahydrobenzoxazolyl, tetrahydrobenzothiazolyl, tetrahydrobenzimidazolyl, dihydrofuropyridyl, dihydropyrazolomorpholinyl, pyridinodioxanyl, dihydroazabenzofuran, dihydroazaisobenzofuran, and dihydroazaindole.

In the present specification, "cyclic amino" is a non-aromatic heterocyclic group having a bond on a nitrogen atom that forms a ring structure of a non-aromatic heterocycle. Examples of cyclic amino include azetidin-1-yl, pyrrolidin-1-yl, piperidin-1-yl, morpholin-4-yl, thiomorpholin-4-yl, and piperazin-1-yl.

In the present specification, an "aromatic heterocyclic group" is a monovalent group of a monocyclic aromatic heterocycle or a bicyclic aromatic heterocycle with a 5- to 10-membered ring containing one, two, three, or four identical or different hetero atoms selected from the group consisting of nitrogen, oxygen, and sulfur. Examples of the aromatic heterocyclic group include pyrrolyl, furyl, thienyl, pyrazolyl, imidazolyl, oxazolyl, thiazolyl, pyridyl, pyridazinyl, pyrimidinyl, pyrazil, indolyl, isoindolyl, benzofuryl, benzothienyl, indazolyl, benzoimidazolyl, benzoxazolyl, benzothiazolyl, quinolyl, isoquinolyl, cinnolinyl, quinazolinyl, quinoxalyl, pyrolopyridyl, and imidazolopyridyl.

In the present specification, "alkylene" includes linear alkylene and branched alkylene. Examples of $C_{1-4}$ alkylene include methylene, ethylene, trimethylene, and methylmethylene, an example of one aspect includes methylene or ethylene, and an example of another aspect includes methylene.

In the present specification, "alkenylene" includes linear alkenylene and branched alkenylene. Examples of $C_{2-4}$ alkenylene include ethen-1,2-diyl, propen-1,3-diyl, and butan-2-en-1,4-diyl.

In the present specification, "alkynylene" includes linear alkynylene and branched alkynylene. Examples of $C_{2-4}$ alkynylene include ethyn-1,2-diyl, propyn-1,3-diyl, butan-2-yne-1,4-diyl.

In the present specification, "amino" includes $-NH_2$ as well as a group in which one or two hydrogens of $-NH_2$ are substituted with $C_{1-4}$ alkyl or $C_{3-6}$ cycloalkyl. In addition, the $C_{1-4}$ alkyl with which hydrogen of $-NH_2$ is substituted may be substituted with -OH, -O-($C_{1-4}$ alkyl), $-NH_2$, -NH($C_{1-4}$ alkyl), -N($C_{1-4}$ alkyl)($C_{1-4}$ alkyl).

In the present specification, "halogen" is fluoro, chloro, bromo, and iodo, examples of one aspect includes fluoro and chloro, an example of another aspect includes fluoro, and an example of yet another aspect includes chloro.

In Formula (1), $L^1$ is $C_{1-4}$ alkylene; an example of one aspect includes methylene or ethylene; and an example of another aspect includes methylene.

In Formula (I), $L^2$ is a single bond, $C_{1-4}$ alkylene, $C_{2-4}$ alkenylene, $C_{2-4}$ alkynylene, or $C_{1-4}$ alkylene-O-; an example of one aspect includes a single bond or $C_{1-4}$ alkylene; an example of another aspect includes methylene or ethylene; and an example of yet another aspect includes methylene.

In Formula (I), $R^1$ is an aromatic heterocyclic group which may have a substituent or a non-aromatic heterocyclic group which may have a substituent; an aromatic heterocyclic group which may have a substituent in one aspect; a monocyclic 5-membered aromatic heterocyclic group which may have a substituent in another aspect; a monocyclic 5-membered nitrogen-containing aromatic heterocyclic group which may have a substituent in yet another aspect; a monocyclic 5-membered nitrogen-containing aromatic heterocyclic group which may be substituted with $C_{1-4}$ alkyl in yet another aspect; a monocyclic 5-membered nitrogen-containing aromatic heterocyclic group which may be substituted with methyl in yet another aspect; 1-methylpyrazol-4-yl or 2-methylthiazol-5-yl in yet another aspect; 1-methylpyrazol-4-yl in yet another aspect; and 2-methylthiazol-5-yl in yet another aspect.

Examples of a substituent that can be used in the aromatic heterocyclic group or the non-aromatic heterocyclic group in $R^1$ include $C_{1-4}$ alkyl or halogen, and examples of one aspect include $C_{1-4}$ alkyl, and an example of another aspect includes methyl. The aromatic heterocyclic group or the non-aromatic heterocyclic group in $R^1$ may contain one to three $C_{1-4}$ alkyl or halogen as substituents. In a case where the aromatic heterocyclic group or the non-aromatic heterocyclic group in $R^1$ contains a plurality of $C_{1-4}$ alkyl or halogen as substituents, the individual substituents may be the same or different from each other. The $C_{1-4}$ alkyl as a substituent that can be used in the aromatic heterocyclic group or the non-aromatic heterocyclic group in $R^1$ is further substituted with one or two groups selected from the group consisting of -OH, -O-($C_{1-4}$ alkyl), and halogen.

In Formula (1), $R^2$ is -H, amino, $C_{3-6}$ cycloalkyl which may have a substituent, an aryl which may have a substituent, a non-aromatic heterocyclic group which may have a substituent, or an aromatic heterocyclic group which may have a substituent; an aryl which may have a substituent, a non-aromatic heterocyclic group which may have a substituent, or an aromatic heterocyclic group which may have a substituent in one aspect; a non-aromatic heterocyclic group which may have a substituent or an aromatic heterocyclic group which may have a substituent in another aspect; a non-aromatic heterocyclic group which may have a substituent in yet another aspect; and an aromatic heterocyclic group which may have a substituent in yet another aspect.

Examples of the substituent that can be used in $C_{3-6}$ cycloalkyl, the aryl, the non-aromatic heterocyclic group, and the aromatic heterocyclic group in $R^2$ include groups shown in the following S1 group. In the following description of the S1 group, "$C_{1-4}$ alkyl" may be substituted with one to three groups selected from the group consisting of fluoro, -OH, -O-($C_{1-4}$ alkyl), and amino.

The S1 group is the group consisting of

(1) halogen;

(2) -OH, -O-($C_{1-4}$ alkyl), -O-($C_{3-6}$ cycloalkyl), -O-(C=O)-($C_{1-4}$ alkyl), -O-(C=O)-($C_{3-6}$ cycloalkyl), oxo (=O), -SH, and -S-($C_{1-4}$ alkyl);

(3) amino, cyano, and nitro;

(4) -(C=O)-($C_{1-4}$ alkyl), -(C=O)-OH, -(C=O)-O-($C_{1-4}$ alkyl), -(C=O)-amino, - (C=O)-(non-aromatic heterocycle)-(C=O)-$C_{3-6}$ cycloalkyl;

(5) aryl and -O-aryl which may be substituted with one to three substituents on the aryl ring selected from halogen, -OH, -O-($C_{1-4}$ alkyl), and $C_{1-4}$ alkyl;

(6) $C_{3-6}$ cycloalkyl which may be substituted with one to three substituents selected from halogen, -OH, -O-($C_{1-4}$ alkyl), and $C_{1-4}$ alkyl;

(7) an aromatic heterocyclic group which may be substituted with one to three substituents selected from halogen, -OH, -O-($C_{1-4}$ alkyl), and $C_{1-4}$ alkyl;

(8) a non-aromatic heterocyclic group which may be substituted with one to three substituents selected from halogen, -OH, -O-($C_{1-4}$ alkyl), and $C_{1-4}$ alkyl;

(9) $C_{3-6}$ cycloalkyl; and

(10) $C_{1-4}$ alkyl and -O-($C_{1-4}$ alkyl), each of which may be substituted with one to three groups selected from the group consisting of the groups described in (1) to (9) above.

The S1 group in another aspect is the group consisting of

(1a) fluoro and chloro;
(2a) -OH, -O-($C_{1-4}$ alkyl), -O-($C_{3-6}$ cycloalkyl), and oxo (=O);
(3a) amino and cyano;
(4a) -(C=O)-($C_{1-4}$ alkyl);
(6a) $C_{3-6}$ cycloalkyl which may be substituted with one to three substituents selected from halogen, -OH, -O-($C_{1-4}$ alkyl), and $C_{1-4}$ alkyl;
(8a) a non-aromatic heterocyclic group which may be substituted with one to three substituents selected from halogen, -OH, -O-($C_{1-4}$ alkyl), and $C_{1-4}$ alkyl; and
(9a) $C_{1-4}$ alkyl and -O-($C_{1-4}$ alkyl), each of which may be substituted with one to three groups selected from the group consisting of the groups described in (1a) to (4a), (6a), (8a), and (9a) above.

The S1 group in yet another aspect is the group consisting of

(2b) -OH and -O-($C_{1-4}$ alkyl); and
(9b) $C_{1-4}$ alkyl which may be substituted with one to three groups selected from the group consisting of the groups described in (2b) above.

An example of the S1 group in yet another aspect includes $C_{1-4}$ alkyl which may be substituted with one group selected from the group consisting of -OH and -O-($C_{1-4}$ alkyl).

In Formula (I), $R^3$ is $C_{3-6}$ cycloalkyl which may be substituted with one or more groups selected from the group consisting of $C_{1-4}$ alkyl and an cyano, or $C_{1-4}$ alkyl; and $C_{3-6}$ cycloalkyl which may be substituted with $C_{1-4}$ alkyl, or $C_{1-4}$ alkyl in one aspect; $C_{3-6}$ cycloalkyl which may be substituted with $C_{1-4}$ alkyl in another aspect; $C_{3-6}$ cycloalkyl which may be substituted with methyl in yet another aspect; cyclopropyl which may be substituted with methyl in yet another aspect; and 1-methylcyclopropyl in yet another aspect.

Another aspect of the present invention is shown below.

(1) A compound or a salt thereof in which $R^3$ is 1-methylcyclopropyl.
(2) A compound or a salt thereof in which $L^1$ is methylene.
(3) A compound or a salt thereof in which $L^2$ is methylene.
(4) A compound in which $R^1$ is 1-methylpyrazol-4-yl or 2-methylthiazol-5-yl or the compound in which $R^1$ is 1-methylpyrazol-4-yl, or a salt thereof.
(5) A compound or a salt thereof in which $R^2$ is a non-aromatic heterocyclic group which may have a substituent or an aromatic heterocyclic group which may have a substituent.
(6) A compound or a salt thereof containing two or more of the groups described in (1) to (5) above in combination.
Specific examples of the combination described in (6) include the following aspects.
(7) A compound or a salt thereof in which $L^1$ is methylene, and $R^1$ is 1-methylpyrazol-4-yl or 2-methylthiazol-5-yl.
(8) A compound or a salt thereof in which $R^3$ is 1-methylcyclopropyl, $L^1$ is methylene, and $R^1$ is 1-methylpyrazol-4-yl or 2-methylthiazol-5-yl.
(9) A compound or a salt thereof in which $L^1$ is methylene, $R^1$ is 1-methylpyrazol-4-yl or 2-methylthiazol-5-yl, and $L^2$ is methylene.
(10) A compound or a salt thereof in which $R^3$ is 1-methylcyclopropyl, $L^1$ is methylene, $R^1$ is 1-methylpyrazol-4-yl or 2-methylthiazol-5-yl, and $L^2$ is methylene.
(11) A compound or a salt thereof in which $L^1$ is methylene, $R^1$ is 1-methylpyrazol-4-yl or 2-methylthiazol-5-yl, $L^2$ is methylene, and $R^2$ is a non-aromatic heterocyclic group which may have a substituent or an aromatic heterocyclic group which may have a substituent.
(12) A compound or a salt thereof in which $R^3$ is 1-methylcyclopropyl, $L^1$ is methylene, $R^1$ is 1-methylpyrazol-4-yl or 2-methylthiazol-5-yl, $L^2$ is methylene, and $R^2$ is a non-aromatic heterocyclic group which may have a substituent or an aromatic heterocyclic group which may have a substituent.
(13) A compound or a salt thereof in which $L^1$ is methylene, and $R^1$ is 1-methylpyrazol-4-yl.
(14) A compound or a salt thereof in which $R^3$ is 1-methylcyclopropyl, $L^1$ is methylene, and $R^1$ is 1-methylpyrazol-4-yl
(15) A compound or a salt thereof in which $L^1$ is methylene, $R^1$ is 1-methylpyrazol-4-yl, and $L^2$ is methylene.
(16) A compound or a salt thereof in which $R^3$ is 1-methylcyclopropyl, $L^1$ is methylene, $R^1$ is 1-methylpyrazol-4-yl, and $L^2$ is methylene.
(17) A compound or a salt thereof in which $L^1$ is methylene, $R^1$ is 1-methylpyrazol-4-yl, $L^2$ is methylene, and $R^2$ is a non-aromatic heterocyclic group which may have a substituent or an aromatic heterocyclic group which may have a substituent.

(18) A compound or a salt thereof in which $R^3$ is 1-methylcyclopropyl, $L^1$ is methylene, $R^1$ is 1-methylpyrazol-4-yl, $L^2$ is methylene, and $R^2$ is a non-aromatic heterocyclic group which may have a substituent or an aromatic heterocyclic group which may have a substituent.

Specific examples of the compound included in the present invention or specific examples of the compound of Formula (I) each include the following compounds or salts thereof. In the present specification, compound names named by ChemDraw that is a software of a molecule structural formula editor (manufactured by Cambridge Soft) may be used.

1-((2,4-Dimethylthiazol-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 25),
1-((1,3-Dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 28),
1,3-Bis((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 29),
1-((1H-Indazol-6-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 36),
1-((Imidazo[1,5-a]pyridin-6-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 40),
1-((2-(Methoxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 63),
1-((2-(hydroxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 64),
1-((5,7-dihydrofuro[3,4-b]pyridin-3-yl)methyl)-3-((1-methyl-1H-pyrazol-4-yl)methyl)-N-(1-methylcyclopropyl)-2,4-dioxo-1,2,3,4-tetrahydrothieno [2,3-d] pyrimidine-6-sulfonamide (Example 108),
1-((6-fluoro-1,3-dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 110),
1-((6-methoxy-1,3-dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 111),
1-(3-methoxy-4-fluorobenzyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (Example 121),
3-((1-methyl-1H-pyrazol-4-yl)methyl)-N-(1-methylcyclopropyl)-2,4-dioxo-1-((2-oxoindolin-5-yl)methyl)-1,2,3,4-tetrahydrothieno[2,3-d]pyrimidine-6-sulfonamide (Example 130).

The compound of Formula (1) may exist in forms of tautomers and geometric isomers depending on the kind of substituents. In the present specification, a compound of Formula (I) may be described as only one form of an isomer, but the present invention also includes other isomers and isolated forms of the isomers or a mixture thereof.

In addition, the compound of Formula (I) may have asymmetric carbon atoms or axial asymmetry, and correspondingly, may exist in the form of optical isomers. The present invention includes both an isolated form of these optical isomers of the compound of Formula (I) and a mixture thereof.

Furthermore, a pharmaceutically acceptable prodrug of the compound represented by Formula (I) is also included in the present invention. The pharmaceutically acceptable prodrug refers to a compound having a group that can be converted into an amino group, a hydroxyl group, a carboxyl group, or the like, by solvolysis or under a physiological condition. Examples of the group for forming a prodrug include groups as described in Prog. Med., 5, 2157-2161 (1985) or "Pharmaceutical Research and Development" (Hirokawa Publishing Company, 1990), vol. 7, Drug Design, 163-198.

In addition, the salt of the compound of Formula (I) is a pharmaceutically acceptable salt of the compound of Formula (I) and may form an acid addition salt or a salt with a base, depending on the kind of substituents. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or salts with organic bases such as methylamine, ethylamine, ethanolamine, lysine, ornithine, and the like, salts with various amino acids and amino acid derivatives such as acetylleucine and the like, ammonium salts, and others.

Furthermore, the present invention also includes various hydrates or solvates, and any of crystalline polymorphs of the compound of Formula (I) and a salt thereof. In addition, the present invention also includes compounds labeled with various radioactive or non-radioactive isotopes.

(Production Method)

The compound of Formula (I) and a salt thereof can be produced by applying various known synthetic methods with utilizing the characteristics based on basic skeletons thereof or the kind of substituents. In this case, depending on the kind of functional groups, it may be effective, from the viewpoint of production techniques, to substitute the functional group with an appropriate protective group (a group that is can be easily converted into the functional group) at the stage of raw material to intermediate. Examples of the protective group include protective groups described in "Greene's Protective Groups in Organic Synthesis (4th edition, 2006)", written by P. G. M. Wuts and T. W. Greene, which may be appropriately selected and used depending on reaction conditions. In these methods, a desired compound can be obtained by introducing the protective group to carry out the reaction, and then, as desired, removing the protective group.
In addition, the prodrug of the compound of Formula (1) can be produced by introducing a specific group at the stage of raw material to intermediate, in the same manner as for the above-described protective groups, or by further carrying out the reaction using the obtained compound of Formula (I). The reaction can be carried out by applying a method known to those skilled in the art, such as common esterification, amidation, dehydration.
Hereinbelow, a representative production method for the compound of Formula (I) will be described. Each production method may also be carried out with reference to References appended in the explanation. The production methods of the present invention are not limited to the examples as shown below.

(First Production Method)

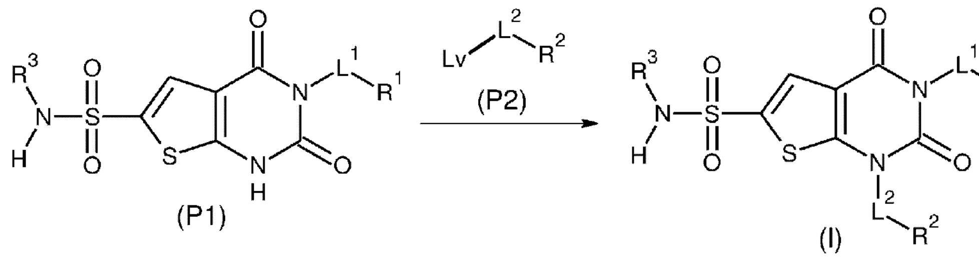

The present production method is a method for producing the compound (I) of the present invention by causing a compound (P2) having a leaving group to react with a dihydrothienopyrimidine compound (P1).
Examples of a leaving group Lv in the compound (P2) include halogen, methanesulfonyloxy, and p-toluenesulfonyloxy.
In this reaction, the compound (P1) and the compound (P2) are used in an equivalent amount or in an excess amount of either component, and usually stirred for 0.1 hours to 5 days in a solvent inert to the reaction or in the absence of a solvent, under cooling or heating to reflux, preferably at 0°C to 120°C. In order to allow the reaction to proceed smoothly, it may be advantageous to carry out the reaction in the presence of an organic base such as triethylamine, diisopropylethylamine, and N-methylmorpholine, or an inorganic base such as potassium carbonate, cesium carbonate, sodium carbonate, and potassium hydroxide.
As a solvent that can be used in this reaction is not particularly limited, but examples thereof include halogenated hydrocarbons such as dichloromethane, 1,2-dichloroethane, and chloroform; aromatic hydrocarbons such as toluene and xylene; ethers such as tetrahydrofuran, 1,4-dioxane, 1,2-dimethoxyethane; alcohols such as methanol, ethanol, and isopropanol; N,N-dimethylformamide; dimethyl sulfoxide; ethyl acetate; acetonitrile; water; and mixtures thereof.
In the present production method, in a case where the leaving group Lv in the compound (P2) is a hydroxyl group, the compound (I) of the present invention can also be produced by utilizing Mitsunobu reaction.
In a case of utilizing Mitsunobu reaction, reaction conditions of Mitsunobu reaction well known to those skilled in the art can be applied, and specifically, reaction conditions used in Preparation Examples and Examples described later, or reaction conditions similar thereto can be applied, for example.
In either method, in a case where another site in a molecule, for example, a hydrogen atom on sulfonamide at 6-position of the dihydrothienopyrimidine compound (P1), complicates the reaction, the site may be protected, the reaction may be then carried out with either the compound (P2) or a compound in which any functional group of $R^2$ in compound (P2) is protected, and thereafter, the protected site of the compound (P1) and/or the compound (P2) may be deprotected, and as a result, the compound of the present invention may be led.
As the protecting group, a group usually used by those skilled in the art can be applied, and for example, the groups described in the above described "Greene's Protective Groups in Organic Synthesis (4th edition, 2006)" can be

used.

(Second Production Method)

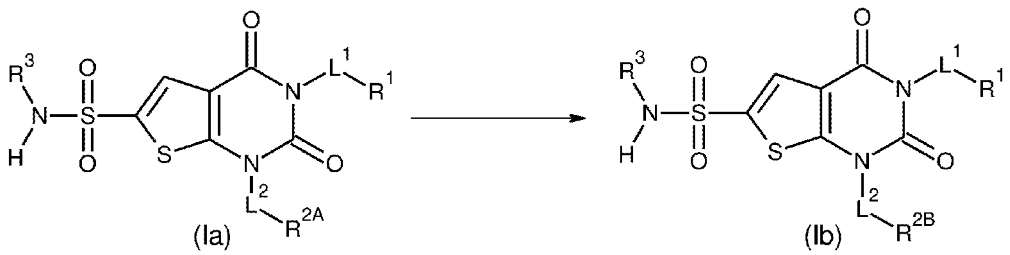

The present production method is, for example, a compound (Ia) of the present invention produced by the first production method (in Formula, $R^{2A}$ represents any one of groups of $R^2$) or the protected compound (Ia) of the present invention (in Formula $R^{2A}$ represents a group in which any functional group of $R^2$ is protected) is carried out functional group conversion or deprotected to produce a compound (Ib) of the present invention (in Formula, $R^{2B}$ is any one of groups of $R^2$, which is different from $R^{2A}$).

The reaction applicable to the present production method is not particularly limited, and another compound of the present invention can be produced by using a reaction that can be used by those skilled in the art to carry out the functional group conversion or deprotection. Examples thereof include alkylation, dealkylation, acylation, deacylation, alkoxycarbonylation, and dealkoxycarbonylation, and reaction conditions well known to those skilled in the art can be applied to each of those examples.

In a case where the hydrogen atom on the sulfonamide at the 6-position of the dihydrothienopyrimidine compound (P1) complicates the reaction, the site may be protected or the protected dihydrothienopyrimidine compound (P1) may be used for the functional group conversion or deprotection, and the compound of the present invention may be then led. In one aspect, a compound in which the hydrogen atom on the sulfonamide at the 6-position of the dihydrothienopyrimidine compound (P1) is substituted with a tert-butyloxycarbonyl group and protected can be used.

The compound represented by Formula (P1) shown above can be produced by the method shown in Examples or Preparation Examples described later, or a method similar thereto.

In a case of introducing $-L^1-R^1$ in Formula (I), with reference to a method described in Preparation Example 1 described later, an amine compound to be condensed with methyl aminothiophenecarboxylate is appropriately selected, and the obtained condensate may be used to carry out structural conversion according to methods of Preparation Example 2, Preparation Example 3, and Example 1. For example, $R^1-L^1-NH_2$ is used as an amine compound to be condensed with methyl aminothiophenecarboxylate to produce a condensate according to the method in Preparation Example 1 described later, the obtained condensate is used to carry out the structural conversion according to Preparation Example 2, Preparation Example 3, and Example 1 described later, and as a result, the compound of Formula (P1) having $-L^1-R^1$ desired can be produced.

In a case of introducing $-R^3$ in Formula (I), with reference to the method described in Example 1 described later, an amine compound to be added to a sulfonyl chloride compound may be appropriately selected. For example, as the amine compound to be added to the sulfonyl chloride compound, $R^3-NH_2$ or a compound in which one hydrogen atom of $R^3-NH_2$ is substituted with a group used as an amine protective group is used to be added to the sulfonyl chloride compound according to the method of Example 1 described later, and as a result, the compound of Formula (P1) having $-R^3$ desired can be produced.

The compound of Formula (I) is isolated and purified as a free compound and a salt thereof, a hydrate, a solvate, or a crystal polymorphic substance. The salt of the compound of Formula (I) can also be produced through a conventional salt forming reaction.

Isolation and purification are carried out by ordinary chemical operations such as extraction, fractionated crystallization, and various fractional chromatography being applied.

Various isomers can be produced through the selection of an appropriate raw material compound, or can be isolated by the utilization of a difference in physicochemical properties between the isomers. For example, optical isomers can be obtained by methods of general optical resolution of racemates (for example, fractionated crystallization for leading diastereomer salts with optically active bases or acids, chromatography using a chiral column, and the like). In addition, the isomers can also be produced from a suitable optically active raw material compound.

The pharmacological activities of the compound of the formula (I) were confirmed by the following tests.

PARG Activity Assay

In order to evaluate the PARG inhibitory activity of the present compound, the inhibitory activity of each test compound against human recombinant PARG was measured. The specific procedure is described below.

(Preparation of Compound Solution)

Test compound was dissolved in dimethyl sulfoxide and prepared a 10 mM stock solution. The stock solution was serially diluted with dimethyl sulfoxide to the desired concentrations from 10 mM to 0.17 μM.

(Assay Method and Measurement Method)

PARG Activity Assay was performed in Streptavidin-coated 96-well plates. Terminal -biotinylated poly (ADP-ribose) polymer was diluted to a final concentration of 25 nM with 0.2% bovine serum albumin (BSA) containing phosphate-buffered saline s olution (PBS) and used for the substrate solution. The substrate solution was added to the 96-well plate at the volume of 200 micro liter per well and incubated for 3 hours at room temperature. After the incubation, the substrate solution was discar ded and assay plate was washed 4 times with 220 micro liter of PBS containing 0.1% Tween20 (PBST). Recombinant human PARG protein was diluted with 0.2% BSA containing PBS to a final concentration of 0.0185 nM. The diluted enzyme s olution was pre-incubated with compounds for 1 hour at room temperature in a 96 -well plate and transferred 100 micro liter per well to the poly (ADP-ribose) substr ate-coated assay plate. The plate was incubated for 1 hour at 25°C and then washe d 4 times with 220 micro liters of PBST. For the detection of non-digested poly (ADP-ribose) polymer on the plate, mouse anti-poly (ADP-ribose) antibody diluted with 0.2% BSA containing PBS was added 100 micro liters per well and incubated 1 hour at room temperature. Plate was washed 4 times with TBST and 100 micr o liter of HRP-labeled anti-mouse Ig antibody was added to the wells. Plate was i ncubated for 30 minutes at room temperature and then washed 4 times with PBST. For the detection 100 micro liters of TMB substrate solution was added to the w ells. After sufficient color development, reaction was stopped by addition of 50 mi cro liter of 0.2 M Sulfonic acid. The 450 nm absorbance (Abs 450) of each well was read with a micro plate reader. The assay was performed by duplicated and a verage of 2 independent assays were used for the determination of PARG inhibitor y activity.

(Negative Control)

Wells treated with dimethyl sulfoxide instead of test compound solution were desig nated as a Negative Control of assay

(Positive Control)

Wells treated with 0.2% BSA containing PBS instead of recombinant human PARG protein and with dimethyl sulfoxide instead of test compound solution were desig nated as a Positive Control of assay.

(Determination of PARG Inhibitory Activity)

The PARG Inhibitory Activity (%) was determined by the following formulation.

$$\text{PARG Inhibitory Activity (\%)} = [(\text{AbsX} - \text{AbsA})/(\text{AbsB} - \text{AbsA})] \times 100$$

(wherein,

AbsA is absorbance (Abs 450) of Negative Control wells
AbsB is absorbance (Abs 450) of Positive Control wells
AbsX is absorbance (Abs 450) of wells treated with test compound.)

The results of some test compounds of Formula (I) are shown in Table 1 as $IC_{50}$ (μM) values for which 50% inhibition concentration was calculated based on PARG inhibition rate (%). In Table, Ex indicates an Example number shown below.

[Table 1]

| Ex | $IC_{50}$ ($\mu$M) |
|---|---|
| 1 | 22. 95 |
| 2 | 0. 065 |
| 3 | 0. 029 |
| 4 | 0.011 |
| 5 | 0. 012 |
| 6 | 0. 014 |
| 7 | 0.21 |
| 8 | 0. 63 |
| 9 | 1.07 |
| 10 | 0. 016 |
| 11 | 0. 16 |
| 12 | 0. 0099 |
| 13 | 0. 041 |
| 14 | 0. 32 |
| 15 | 0.6 |
| 16 | 0. 97 |
| 17 | 0.47 |
| 18 | 0. 58 |
| 19 | 0. 13 |
| 20 | 0. 11 |
| 21 | 75. 52 |
| 22 | 93. 13 |
| 23 | 96. 97 |
| 24 | 2. 22 |
| 25 | 0. 013 |
| 26 | 0.37 |
| 27 | 0. 25 |
| 28 | 0.01 |
| 29 | 0. 041 |
| 30 | 0. 037 |
| 31 | 0. 029 |
| 32 | 6. 99 |
| 33 | 0. 035 |
| 34 | 0.3 |
| 35 | 0. 035 |
| 36 | 0. 0053 |
| 37 | 0. 009 |

(continued)

| Ex | $IC_{50}$ ($\mu$M) |
|---|---|
| 38 | 0. 0041 |
| 39 | 0. 021 |
| 40 | 0. 0041 |
| 41 | 0. 097 |
| 42 | 0. 04 |
| 43 | 0. 029 |
| 44 | 0. 048 |
| 45 | 0. 013 |
| 46 | 0. 0092 |
| 47 | 0. 13 |
| 48 | 0. 12 |
| 49 | 0. 095 |
| 50 | 0. 04 |
| 51 | 0. 033 |
| 52 | 0.1 |
| 53 | 0. 052 |
| 54 | 0. 049 |
| 55 | 0. 041 |
| 56 | 0. 036 |
| 57 | 0. 02 |
| 58 | 0. 26 |
| 59 | 0. 022 |
| 60 | 0. 024 |
| 61 | 0. 011 |
| 62 | 0. 029 |
| 63 | 0. 02 |
| 64 | 0. 015 |
| 65 | 0. 068 |
| 66 | 0. 046 |
| 67 | 0.1 |
| 68 | 0. 063 |
| 69 | 0. 28 |
| 70 | 0. 06 |
| 71 | 0. 12 |
| 72 | 0. 19 |
| 73 | 0. 017 |
| 74 | 0.21 |
| 75 | 0. 13 |

(continued)

| Ex | $IC_{50}$ ($\mu$M) |
|---|---|
| 76 | 0. 057 |
| 77 | 0. 07 |
| 78 | 0. 111 |
| 79 | 0. 07 |
| 80 | 0. 42 |
| 81 | 0. 2 |
| 82 | 0. 097 |
| 83 | 0. 11 |
| 84 | 0.2 |
| 85 | 0. 11 |
| 86 | 0. 16 |
| 87 | 0. 22 |
| 88 | 0. 023 |
| 89 | 0. 013 |
| 90 | 0. 017 |
| 91 | 0. 27 |
| 92 | 0. 23 |
| 93 | 0. 18 |
| 94 | 0. 27 |
| 95 | 0. 32 |
| 96 | 0. 13 |
| 97 | 0. 47 |
| 98 | 5. 94 |
| 99 | 0. 5 |
| 100 | 0. 042 |
| 101 | 0. 064 |
| 102 | 0. 075 |
| 103 | 0. 11 |
| 104b | 0. 12 |
| 105 | 0. 05 |
| 106 | 0. 99 |
| 107 | 0. 07 |
| 108 | 0. 004 |
| 109 | 0. 045 |
| 110 | 0. 017 |
| 111 | 0. 12 |
| 112 | 0. 14 |
| 113 | 0. 094 |

(continued)

| Ex | $IC_{50}$ ($\mu$M) |
|---|---|
| 114 | 0. 034 |
| 115 | 0. 068 |
| 116 | 0. 015 |
| 117 | 0. 041 |
| 118 | 0. 12 |
| 119 | 0. 24 |
| 120 | 0. 12 |
| 121 | 0. 02 |
| 122 | 0. 017 |
| 123 | 0. 029 |
| 124 | 0. 073 |
| 125 | 0. 02 |
| 126 | 0. 045 |
| 127 | 0.32 |
| 128 | 0. 13 |
| 129 | 0. 046 |
| 130 | 0. 003 |
| 131 | 0. 0063 |
| 132 | 0. 66 |
| 133 | 1. 18 |
| 134 | 6. 04 |
| 135 | 0. 037 |
| 136 | 0.86 |

2. Synthetic Lethality Assay of Cancer Cell

In order to evaluate the cancer cell growth inhibitory effect of the present compound, the inhibitory effect of the compound on the growth of a human ovarian cancer cell line, Kuramochi cells, was examined in the presence of methyl methanesulfonate (MMS) which is used as a DNA methylating agent.

(Preparation of Test Compound Solution)

Test compound was dissolved in dimethyl sulfoxide and prepared a 10 mM compound solution. The compound solution was then further serially diluted with dimethyl sulfoxide to prepare a concentration of 400 times test compound solution from 6 mM to 1 $\mu$M. Immediately before addition to the cells, a concentration of 400 times test compound solution was diluted 2-fold with RPMI 1640 cell culture medium (complete medium) containing 10% fetal bovine serum to prepare a 200-fold concentration of a solution. The final dimethyl sulfoxide concentration during a cell assay was 0.25%, and it has been confirmed that there was no effect on cell proliferation or survival.

(Assay Method and Measurement Method)

Kuramochi cells were purchased from the JCRB cell bank and cultured at 37°C in the presence of 5% of $CO_2$ using the complete medium described above. The medium was replaced with a fresh complete medium twice a week during the culture. In a case where the cell density reached 70% to 90% confluency, the cells were washed with PBS,

treated with 0.05% trypsin-EDTA to recover the adherent cells, diluted and seeded for passage.

Synthetic lethality assay was carried out by using the 96-well plate for cell culture. After the Kuramochi cells were washed with PBS, the cells were recovered with 0.05% trypsin-EDTA and dispersed to be a single cell with a density of 50,000 cells/mL by using a complete medium to prepare a cell suspension. 200 micro liters of the cell suspension was added to each well and incubated for 5 hours at 37°C in the presence of 5% $CO_2$. After the incubation, 1 μL of a concentration of 200 times test compound solution was added to each well, stirred with a plate mixer, and cultured at 37°C for 1 hour in the presence of 5% $CO_2$. MMS was diluted in a complete medium to 0.37 mg/mL to prepare a solution with a concentration of 200 times the using concentration, and 1 μL of the solution per well was added 1 hour after the addition of test compound. The plate was stirred with a plate mixer to achieve the solutions uniformly mixed and incubated at 37°C for 96 hours in the presence of 5% $CO_2$.

Cell viability after 96 hours was measured by using CellTiter-Glo (registered trademark) Luminescent Cell Viability Assay (manufactured by Promega Corporation) according to the recommended usage.

The luminescence intensity was measured by using a luminescence detection plate reader (Envision, manufactured by PerkinElmer Inc.). The cell viability of each well was calculated as a relative value (%) of the luminescence intensity of each well to which each test compound with each concentration was added in a case where the luminescence intensity of a well to which dimethyl sulfoxide was added instead of the test compound was defined as 100%. The 50% inhibitory concentration ($IC_{50}$) was calculated from the growth inhibition rate of test compound at 9 different concentrations by fitting a dose-response curve with a 4-parameter method using GraphPad Prism software. The assay was conducted in triplicate in each condition. The average of two independent assays was used as the cell-growth inhibition of the test compound.

$IC_{50}$ (μM) values of some test compounds of Formula (I) are shown in Table 2. In Table, Ex indicates an Example number shown below.

[Table 2]

| Ex | $IC_{50}$ (μM) |
|---|---|
| 25 | 4. 45 |
| 28 | 0. 78 |
| 29 | 8. 76 |
| 36 | 1.98 |
| 40 | 1.64 |
| 63 | 4. 54 |
| 64 | 3.49 |
| 108 | 3. 71 |
| 110 | 7. 65 |
| 111 | 20. 15 |
| 121 | 8. 74 |
| 130 | 11. 68 |

As a result of the above test, the PARG inhibitory action was confirmed in some compounds of Formula (I). Therefore, the compound of Formula (I) can be used for treating cancer, enhancing an effect of an anticancer agent, and/or enhancing an effect of radiotherapy.

A pharmaceutical composition containing one or two or more kinds of the compound of Formula (I) or a salt thereof as an active ingredient can be prepared in accordance with a generally used method, using a pharmaceutically acceptable excipient, that is, an excipient, for example, a pharmaceutical excipient or a pharmaceutical carrier, that is usually used in the art. That is, the pharmaceutical composition of the present invention may contain the compound of Formula (I) or a salt thereof, and the pharmaceutically acceptable excipient.

The administration can be carried out through any form of oral administration via tablets, pills, capsules, granules, powders, liquid preparations, or the like, or parenteral administration via injections such as intraarticular, intravenous, intramuscular, or others, suppositories, eye drops, eye ointments, percutaneous liquid preparations, ointments, percutaneous patches, transmucosal liquid preparations, transmucosal patches, inhalations, and the like.

Regarding a solid composition for oral administration, tablets, powders, granules, or the like are used. In such

a solid composition, one or two or more of active ingredients are mixed with at least one inactive excipient. According to a conventional method, the composition may contain inactive additives such as lubricants, disintegrators, stabilizers, and solubilizers. Tablets or pills may be coated with sugar coating, or with a film of gastric or enteric substance, as necessary.

A liquid composition for oral administration includes pharmaceutically acceptable emulsions, solutions, suspensions, syrups, elixirs, or the like, and contains a generally used inert diluent, such as purified water or ethanol. In addition to the inert diluent, the liquid composition may contain adjuvants such as solubilizing agents, moistening agents, and suspensions, and sweeteners, flavors, aromatics, and antiseptics.

Injections for parenteral administration include sterile, aqueous or non-aqueous solutions, suspensions, or emulsions. As the aqueous solvent, for example, distilled water for injection or physiological saline is included. Examples of the non-aqueous solvent include alcohols such as ethanol. Such a composition may further contain tonicity agents, antiseptics, moistening agents, emulsifying agents, dispersing agents, stabilizers, or solubilizers. These are sterilized, for example, by filtration through a bacteria-retaining filter, blending with bactericides, or irradiation. In addition, these can also be used such that sterile solid compositions are produced, and dissolved or suspended in sterile water or a sterile solvent for injection prior to the use.

In a case of usual oral administration, the daily dose is 0.001 to 100 mg/kg, preferably 0.1 to 30 mg/kg, and more preferably 0.1 to 10 mg/kg, per body weight. The above dose is administered in a single portion or divided into 2 to 4 portions.

In a case of intravenous administration, the appropriate daily dose is 0.0001 to 10 mg/kg, per body weight. The above dose is administered in a single portion or divided into 2 to 4 portions.

In a case of being transmucosally administered, the appropriate daily dose is 0.001 to 100 mg/kg, per body weight. The above dose is administered in a single portion or divided into 2 to 4 portions.

The dose is appropriately determined according to each case, taking into consideration, for example, symptoms, age, and gender.

Depending on the kinds of administration routes, dosage forms, administration sites, excipients, and additives, the pharmaceutical composition of the present invention contains one or more compounds of Formula (I) or salts thereof as active ingredients in an amount of 0.01% to 100% by mass, preferably 0.01% to 50% by mass.

The compound of Formula (I) can be used in combination with various therapeutic agents or prophylactic agents for diseases for which the compound of Formula (I) described above is considered to be effective. Regarding the combination use, coadministration, separate or consecutive administration, or administration at desired time intervals may be employed. Preparations for coadministration may be either combination agents, or preparations that are formulated separately.

[Examples]

Hereinafter, a method for producing the compound of Formula (I) will be described based on Examples. The present invention is not limited to the compounds described in Examples below. In addition, a method for producing a raw material compound is shown as Preparation Example.

The method for producing the compound of Formula (I) is not limited to the production methods of Examples shown below, and the compound of Formula (I) can be produced by a combination of these production methods or a method that is obvious to those skilled in the art.

The concentration mol/L is represented as M.

Examples

Preparation Example 1

2-Amino-N-((2-methylthiazol-5-yl)methyl)thiophene-3-carboxamide

To an ice cold solution of methyl 2-aminothiophene-3-carboxylate (5.0 g, 31.8 mmol) and (2-methylthiazol-5-yl)methanamine (5.3 g, 41.4 mmol) in 1,4-dioxane (100 mL), 2M solution of Trimethylaluminum in Toluene (80 mL) was added in drop wise manner over a period of 30-45 minutes under inert atmosphere. The resultant reaction mixture was stirred at 100° C for 16h. The reaction mixture was diluted by cold saturated ammonium chloride solution (100 mL) and compound was extracted with ethyl acetate. The organic layer was dried over anhydrous sodium sulphate, and concentrated under reduced pressure. The crude thus obtained was triturated with diethyl ether, (collected by filtration and dried to get 2-amino-N-((2-methylthiazol-5-yl)methyl)thiophene-3-carboxamide (4.90 g, 60%).

Preparation Example 2

3-((2-Methylthiazol-5-yl)methyl)thieno[2,3-d]pyrimidine-2,4(1H,3H)-dione

To an ice cooled solution of 2-amino-N-((2-methylthiazol-5-yl)methyl)thiophene-3-carboxamide (4.9 g, 19.3 mmol) in THF (100 mL) was added 1,1'-carbonyldiimidazole (15.7 g, 96.7 mmol) in portion wise manner, and the resulting reaction mass was stirred at 80° C for next 16h. The solvent was evaporated under reduced pressure, and the residue which was triturated with acetonitrile, collected by filtration to get 3-((2-methylthiazol-5-yl)methyl)thieno[2,3-d]pyrimidine-2,4(1H,3H)-dione (4.3 g, 79%).

Preparation Example 3

3-((2-Methylthiazol-5-yl)methyl)-2,4-dioxo-1,2,3,4-tetrahydrothieno[2,3-d]pyrimidine-6-sulfonyl chloride

To an ice cooled chlorosulfonic acid (7.17 mL, 107.73 mmol) was added 3-((2-methylthiazol-5-yl)methyl)thieno[2,3-d]pyrimidine-2,4(1H,3H)-dione (4.3 g, 15.39 mmol) in portion wise manner at 0°C. The resulting reaction mass was heated to 100°C and stirred for 2h. The reaction mixture was slowly poured into ice cold water (200 mL). The precipitated solid was filtered and washed with cold water, dried under reduced pressure to get 3-((2-methylthiazol-5-yl)methyl)-2,4-dioxo-1,2,3,4-tetrahydrothieno[2,3-d]pyrimidine-6-sulfonyl chloride (4.30 g crude). The compound obtained was taken to next reaction without any further purification.

Example 1

N-(1-Methylcyclopropyl)-3-((2-methylthiazol-5-yl)methyl)-2,4-dioxo-1,2,3,4-tetrahydrothieno[2,3-d]pyrimidine-6-sulfonamide

To a solution of 3-((2-methylthiazol-5-yl)methyl)-2,4-dioxo-1,2,3,4-tetrahydrothieno[2,3-d]pyrimidine-6-sulfonyl chloride (4.3 g, 11.38 mmol) in a mixture of tetrahydrofuran and dichloromethane (100 mL, 1:1 ratio) was added diisopropylethylamine (18.2 mL, 102.5 mmol) and 1-methylcyclopropan-1-annine hydrochloride (2.46 g, 22.76 mmol), and the mixture was stirred at room temperature for 16h. The solvent was evaporated under reduced pressure, to get the residue which was diluted with water (80 mL) and t extracted with dichloromethane. The organic layer was dried over anhydrous sodium sulfonate, and evaporated under reduced pressure to get the residue, which was triturated with diethyl ether and collected by filtration to get N-(1-methylcyclopropyl)-3-((2-methylthiazol-5-yl)methyl)-2,4-dioxo-1,2,3,4-tetrahydrothieno[2,3-d]pyrimidine-6-sulfonamide (5.50 g, 62% over 2 steps).

Example 2

1-(Cyclopropylmethyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide

To a solution of N-(1-methylcyclopropyl)-3-((2-methylthiazol-5-yl)methyl)-2,4-dioxo-1,2,3,4-tetrahydrothieno[2,3-d]pyrimidine-6-sulfonamide (50 mg, 0.121 mmol) and (bromomethyl)cyclopropane (24 mg, 0.182 mmol) in N,N-dimethylformamide (2 mL) was added $K_2CO_3$ (25 mg, 0.182 mmol), and the mixture was stirred at 90°C for 3h. To the reaction mixture was added ice-cold water and the solution was extracted with dichloromethane. The combined organic layer was dried over anhydrous sodium sulfate and evaporated under reduced pressure to get 1-(cyclopropylmethyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide (28 mg, 50%).

Preparation Example 4

tert-Butyl (1-methylcyclopropyl) ((3-((2-methylthiazol-5-yl)methyl)-2,4-dioxo-1,2,3,4-tetrahydrothieno[2,3-d]pyrimidin-6-yl)sulfonyl)carbamate

To an ice cold solution of N-(1-methylcyclopropyl)-3-((2-methylthiazol-5-yl)methyl)-2,4-dioxo-1,2,3,4-tetrahydrothieno[2,3-d]pyrimidine-6-sulfonamide (5.50 g, 13.30 mmol) in pyridine (55 mL) was added 4-dimethylaminopyridine (0.163 g, 1.33 mmol ) and di-tert-butyl dicarbonate (14.5 g, 16.7 mmol), and the reaction mixture was stirred at 95°C for 3h. The solvent was evaporated under reduced pressure and the residue was diluted with water and extracted with dichloromethane. The organic layer was washed with 1M hydrochloric acid and then brine dried over anhydrous sodium

sulfate and evaporated under reduced pressure. Obtained solid was triturated with combination of ethyl acetate and diethyl ether (1:8 ratio) to get tert-butyl (1-methylcyclopropyl)((3-((2-methylthiazol-5-yl)methyl)-2,4-dioxo-1,2,3,4-tetrahydrothieno[2,3-d]pyrimidin-6-yl)sulfonyl)carbamate (6.0 g, 87%).

Preparation Example 5

tert-Butyl ((1-((2,4-dimethylthiazol-5-yl)methyl)-3-((2-methylthiazol-5-yl)methyl)-2,4-dioxo-1,2,3,4-tetrahydrothieno[2,3-d]pyrimidin-6-yl)sulfonyl)(1-methylcyclopropyl)carbamate

To an ice cold stirred solution of tert-butyl (1-methylcyclopropyl)((3-((2-methylthiazol-5-yl)methyl)-2,4-dioxo-1,2,3,4-tetrahydrothieno[2,3-d]pyrimidin-6-yl)sulfonyl)carbamate (180 mg, 0.351 mmol) and 5-(chloromethyl)-2,4-dimethylthiazole 9 (114 mg, 0.702 mmol) in N,N-dimethylformamide (2 mL) was added potassium carbonate (146 mg, 1.05 mmol) and sodium iodide (105 mg, 0.702 mmol), and the reaction mass was stirred at 90°C for 3h. The reaction mixture was diluted with ice-cold water and extracted with dichloromethane, and the organic layer was dried over anhydrous sodium sulfate to get tert-butyl ((1-((2,4-dimethylthiazol-5-yl)methyl)-3-((2-methylthiazol-5-yl)methyl)-2,4-dioxo-1,2,3,4-tetrahydrothieno[2,3-d]pyrimidin-6-yl)sulfonyl)(1-methylcyclopropyl)carbamate (175 mg, crude).

Example 6

1-((2,4-Dimethylthiazol-5-yl)methyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide

To an ice-cold solution of tert-butyl ((1-((2,4-dimethylthiazol-5-yl)methyl)-3-((2-methylthiazol-5-yl)methyl)-2,4-dioxo-1,2,3,4-tetrahydrothieno[2,3-d]pyrimidin-6-yl)sulfonyl)(1-methylcyclopropyl)carbamate (170 mg, 0.267 mmol) in dichloromethane (10 mL) was added trifluoroacetic acid (2.5 mL), and the reaction mass was stirred at room temperature for 3h. The solvent was evaporated under reduced pressure and the residue was re-dissolved in dichloromethane and washed with sat. sodium bicarbonate solution. The organic layer was dried over anhydrous sodium sulfate and evaporated under reduced pressure. The residue was further purified over silica gel column chromatography (2% MeOH - dichloromethane) to get 1-((2,4-dimethylthiazol-5-yl)methyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide as off white solid (30 mg, 21%).

Preparation Example 6

2-Amino-N-((1-methyl-1H-pyrazol-4-yl)methyl)thiophene-3-carboxamide

To an ice cold solution of methyl 2-aminothiophene-3-carboxylate (35.0 g, 223 mmol) and (1-methyl-1H-pyrazol-4-yl)methylamine (37.10 g, 334 mmol) in 1,4-Dioxane (1.0 L), 2M solution of trimethylaluminum in Toluene (557 mL) was added in drop wise manner, and the reaction mixture was stirred at 100° C for 16h. The reaction mixture was diluted by cold saturated ammonium chloride solution (600 mL) and extracted with ethyl acetate. The organic layer was washed with brin, dried over anhydrous sodium sulphate, and concentrated under reduced pressure. The residue was triturated with diethyl ether, collected by filtration and dried to get 2-amino-N-((1-methyl-1H-pyrazol-4-yl)methyl)thiophene-3-carboxamide (43.0 g, 82%).

Preparation Example 7

3-((1-Methyl-1H-pyrazol-4-yl)methyl)thieno[2,3-d]pyrimidine-2,4(1H,3H)-dione

To a solution of 2-amino-N-((1-methyl-1H-pyrazol-4-yl)methyl)thiophene-3-carboxamide (43.0 g, 181.97 mmol) in tetrahydrofuran (1.5 L) was added 1,1'-carbonyl diimidazole (88.54 g, 545.9 mmol) and the resulting reaction mass was heated at 80° C for 16h. The solvent was evaporated under reduced pressure and the residue was triturated with acetonitrile and collected by filtration to get 3-((1-methyl-1H-pyrazol-4-yl)methyl)thieno[2,3-d]pyrimidine-2,4(1H,3H)-dione (37.90 g, 79%).

Preparation Example 8

3-((1-Methyl-1H-pyrazol-4-yl)methyl)-N-(1-methylcyclopropyl)-2,4-dioxo-1,2,3,4-tetrahydrothieno[2,3-d]pyrimidine-6-sulfonamide

To chlorosulfonic acid (96.7 mL, 1444.91 mmol) was added 3-((1-methyl-1H-pyrazol-4-yl)methyl)thieno[2,3-d]pyrimidine-2,4(1H,3H)-dione (37.90 g, 144.49 mmol) in portion wise manner at 0°C. The resulting mixture was heated to 100°C and stirred for 2h. The reaction mixture was cooled to room temperature and it was slowly poured into ice cold water (1 L). The precipitated solid was filtered, washed with cold water (2 x 200 mL), and dried to get 3-((1-methyl-1H-pyrazol-4-yl)methyl)-2,4-dioxo-1,2,3,4-tetrahydrothieno [2,3-d] pyrimidine-6-sulfonyl chloride.

To an ice cold solution of the sulfonyl chloride in 1:1 mixture of tetrahydrofuran and dichloromethane (1 L) was added N,N-diisopropylethylamine (195.45 mL, 1122.5 mmol) and 1-methylcyclopropan-1-amine hydrochloride (26.8 g, 249.4 mmol) under inert atmosphere. The reaction mass was stirred at room temperature for 16h.The solvent was evaporated under reduced pressure, and the residue was diluted with water and was extracted with dichloromethane. The organic layer was dried over anhydrous sodium sulfonate, and concentrated. The residue was triturated with diethyl ether, collected by filtration and dried to get 3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (35.50 g, 62%).

Preparation Example 9

tert-Butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To an ice cold solution of 3-((1-methyl-1H-pyrazol-4-yl) methyl)-N-(1-methylcyclopropyl)-2,4-dioxo-1,2,3,4-tetrahydrothieno[2,3-d] pyrimidine-6-sulfonamide (35.50 g, 89.80 mmol) in pyridine (350 mL) was added 4-dimethylaminopyridine (1.10 g, 8.978 mmol) and di-tert-butyl dicarbonate (98 g, 449 mmol). The reaction mixture was stirred at 95°C for 3h, and the solvent was evaporated under reduced pressure. The residue was diluted with water and extracted with dichloromethane. The organic layer was washed with 1M hydrochloric acid and brine, dried over anhydrous sodium sulfate and evaporated under reduced pressure. The residue was triturated with 1:8 mixture of ethyl acetate and diethyl ether and solid was collected by filtration to get tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (32.0 g, 72%).

Preparation Example 10

tert-Butyl ((1-((2,4-dimethylthiazol-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a stirred solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (6 g, 12.1 mmol) and 5-(chloromethyl)-2,4-dimethylthiazole (2.15 g, 13.3 mmol) in N,N-dimethylformamide (60 mL) was added potassium carbonate (3.35, 24.2 mmol) and sodium iodide (3.63 g, 24.2 mmol), and the reaction mass was stirred at 90°C for 3h. The reaction mixture was diluted with ice-cold water and was extracted with dichloromethane, and the organic layer was dried over anhydrous sodium sulfate and then evaporated under reduced pressure. The residue was purified over silica gel column chromatography (5% methanol - dichloromethane) to get tert-butyl ((1-((2,4-dimethylthiazol-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (4.70 g, 63%) as off white solid.

Example 25

1-((2,4-Dimethylthiazol-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice-cold solution of tert-butyl ((1-((2,4-dimethylthiazol-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (5.82 g, 9.38 mmol) in dichloromethane (100 mL) was added trifluoroacetic acid (25 mL), and the resulting reaction mass was stirred at room temperature for 3h. The solvent was evaporated under reduced pressure, and the residue was re-dissolved in dichloromethane and washed with saturated sodium bicarbonate solution. The organic layer was dried over anhydrous sodium sulfate, and evaporated under reduced pressure. The residue was purified over silica gel column chromatography (2%

methanol - dichloromethane) to get 1-((2,4-dimethylthiazol-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (3.37 g, 69%) as off white solid.

Preparation Example 11

(5-Bromo-2-(chloromethyl)phenyl)methanol

To an ice-cold stirred solution of 5-bromoisobenzofuran-1,3-dione (10 g, 44.1 mmol) in tetrahydrofuran (100 mL), added lithium aluminum hydride (35.2 mL, 2.5M, 88.20 mmol) in drop wise manner, and the mixture was stirred at room temperature for 16h. The reaction mixture was cooled to 0°C and quenched by adding ethyl acetate and water, and the separated organic layer was dried over anhydrous sodium sulfate and evaporated under reduced pressure. The residue was purified over silica column chromatography (40% ethyl acetate - hexane). The obtained solid (5.40 g) was mixed with conc. hydrochloric acid (30.0 mL) and stirred at 70°C for 1h. The reaction mixture was diluted with cold water, and extracted with ethyl acetate, and the organic layer was dried over anhydrous sodium sulfate, and evaporated under reduced pressure to get (5-bromo-2-(chloromethyl)phenyl)methanol (4.0 g, 73%) as pale yellow liquid.

Preparation Example 12

5-Bromo-1,3-dihydroisobenzofuran

To an ice cold stirred solution of (5-bromo-2-(chloromethyl)phenyl)methanol (4.0 g, 17.0 mmol) in tetrahydrofuran (40.0 mL), added NaH (1.36 g, 34 mmol) and the mixture was stirred at 45°C for 2h. The reaction mixture was quenched with cold water and compound was extracted with ethyl acetate. The combined organic layer was dried over anhydrous sodium sulfate, and evaporated under reduced pressure. The residue was purified over silica gel column chromatography (20-25% ethyl acetate - hexane) to get 5-bromo-1,3-dihydroisobenzofuran (2.30 g, 68%) as colorless liquid.

Preparation Example 13

1,3-Dihydroisobenzofuran-5-carbaldehyde

To a stirred solution of 5-bromo-1,3-dihydroisobenzofuran (1.0 g, 5.02 mmol) in tetrahydrofuran (20.0 mL) added n-butyl lithium (2.41 mL, 6.03 mmol) in drop wise manner at -78° C and the mixture was stirred at the same temperature for 45 minutes. N,N-dimethylformamide (1.17 mL, 15.10 mmol) was added slowly to the mixture over a period of 15 min, and the mixture was stirred at room temperature for 4h. The reaction mixture was quenched with ammonium chloride aqueous solution and extracted with ethyl acetate, and organic layer was dried over anhydrous sodium sulfate, and then evaporated under reduced pressure. The residue was purified over silica gel column chromatography (5-10% ethyl acetate - hexane) to get 1,3-dihydroisobenzofuran-5-carbaldehyde (500 mg, 67%) as pale yellow liquid.

Preparation Example 14

(1,3-Dihydroisobenzofuran-5-yl)methanol

To an ice-cold stirred solution of 1,3-dihydroisobenzofuran-5-carbaldehyde (400 mg, 2.70 mmol) in isopropanol (4 mL) and chloroform (30 mL) added silica-gel (1.24 g) and sodium borohydride (261 mg, 7.29 mmol), and the mixture was stirred at same temperature for next 2h. The reaction mixture was quenched with water and extracted with ethyl acetate, and the organic layer was dried over anhydrous sodium sulfate, and evaporated under reduced pressure. The residue was purified over silica gel column chromatography (15-20% ethyl acetate - hexane) to get (1,3-dihydroisobenzofuran-5-yl)methanol (310 mg, 76%) as off white solid.

Preparation Example 15

5-(Bromomethyl)-1,3-dihydroisobenzofuran

To an ice cold stirred solution of (1,3-dihydroisobenzofuran-5-yl)methanol (300 mg, 2.0 mmol) in tetrahydrofuran (10 mL)were added triphenyl phosphine (1.57 g, 5.99 mmol) and N-bromosuccinimide (1.42 g, 7.99 mmol), and the mixture was stirred at room temperature for next 1h. The solvent was evaporated under reduced pressure and the

residue was purified over silica gel column chromatography (20% ethyl acetate - hexane) to get 5-(bromomethyl)-1,3-dihydroisobenzofuran (180 mg, 42%) as off white solid.

Preparation Example 16

tert-Butyl ((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a stirred solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (120 mg, 0.24 mmol) and 5-(bromomethyl)-1,3-dihydroisobenzofuran (75 mg, 0.36 mmol) in N,N-dimethylformamide (2 mL) were added potassium carbonate (99 mg, 0.72 mmol) and sodium iodide (17 mg, 0.12 mmol), and the mixture was stirred at 90°C for 3h. The reaction mixture was diluted with cold water and extracted with ethyl acetate. The organic layer was dried over anhydrous sodium sulfate, and evaporated under reduced pressure. The residue was purified over silica gel column chromatography (70% ethyl acetate - hexane) to get tert-butyl ((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (80 mg, 53%) as off white solid.

Example 28

1-((1,3-Dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice cold stirred solution of tert-butyl ((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (70 mg, 11.2 mmol) in dichloromethane (10 mL) was added a solution of trifluoroacetic acid in dichloromethane (about 25%, 3 mL), and the reaction mass was stirred at room temperature for 3h. The solvent was evaporated under reduced pressure, and the residue was dissolved in dichloromethane and washed with saturated sodium bicarbonate aqueous. The organic layer was dried over anhydrous sodium sulfate and evaporated under reduced pressure. The residue was purified over silica gel column chromatography (1% methanol - dichloromethane) to get 1-((1,3-dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (42 mg, 71%) as off white solid.

Preparation Example 17

(1-Methyl-1H-pyrazol-4-yl)methanol

To an ice cold stirred solution of 1-methyl-1H-pyrazole-4-carbaldehyde (18.0 g, 163 mmol) in methanol (300 mL) was added sodium borohydride (12.9 g, 360 mmol) in portion wise manner, and the mixture was stirred at room temperature for next 2h. The solvent was evaporated under reduced pressure, and the residue was diluted with cold water and extracted with ethyl acetate. The organic layer was dried over anhydrous sodium sulfate, and evaporated under reduced pressure. The residue was purified by silica gel column chromatography (70% ethyl acetate - hexane) to get (1-methyl-1H-pyrazol-4-yl)methanol (10.0 g, 55%) as off white solid.

Preparation Example 18

4-(Chloromethyl)-1-methyl-1H-pyrazole hydrochloride

To an ice cold stirred solution of (1-methyl-1H-pyrazol-4-yl)methanol (10.0 g, 89.2 mmol) in dichloromethane (50 mL) was added thionyl chloride (29.10 mL, 401 mmol) in drop wise manner, the mixture was stirred at room temperature for 30 min. The solvent was evaporated under reduced pressure and the residue was triturated with diethyl ether, collected by filtration and dried to get 4-(chloromethyl)-1-methyl-1H-pyrazole hydrochloride (14.0 g, 94%).

Preparation Example 19

tert-Butyl ((1,3-bis((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a stirred solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (7.0 g, 14.1 mmol), 4-(chloromethyl)-1-methyl-1H-pyrazole hydrochloride (7.08 g, 42.4 mmol) in N,N-dimethylformamide (100 mL) was added potassium carbonate (4.88 g, 35.3 mmol) and sodium iodide (5.29 g, 35.3 mmol), and the mixture was stirred at 90°C for 3h. The reaction mixture was diluted with water extracted with ethyl acetate. The organic layer was dried over anhydrous sodium sulfate, and evaporated under reduced pressure. The residue was purified over silica gel column chromatography (50% ethyl acetate - hexane) to get tert-butyl ((1,3-bis((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (5.50 g, 66%) as off white solid.

Example 29

1,3-Bis((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice cold stirred solution of tert-butyl ((1,3-bis((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (5.00 g, 8.48 mmol) in dichloromethane (30 mL) was added a solution of trifluoroacetic acid in dichloromethane (about 25%, 20 mL) in drop wise manner, and the reaction mixture was stirred at room temperature for 3h. The solvent was evaporated under reduced pressure, and the residue was dissolved in dichloromethane and washed with sat. sodium bicarbonate aqueous solution. Organic layer was dried over anhydrous sodium sulfate, and evaporated under reduced pressure, and the residue was purified over silica gel column chromatography (about 3% methanol - dichloromethane) to get 1,3-bis((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (2.80 g, 67%) as off white solid.

Preparation Example 20

1-(2-Methoxyethyl)-1H-pyrazole-4-carbaldehyde

To an ice cold stirred solution of 1H-pyrazole-4-carbaldehyde (1 g, 10.4 mmol) in N,N-dimethylformamide (15 mL) was added cesium carbonate (6.78 g, 20.8 mmol) and 1-bromo-2-methoxyethane (1.45 g, 10.4 mmol), and the reaction mass was stirred at 60°C for 16h. The reaction mixture was quenched with sat. ammonium chloride aqueous solution (75mL) and extracted with ethyl acetate. The organic layer was dried over anhydrous sodium sulfate, and evaporated under reduced. The residue was purified over silica gel column chromatography (20% ethyl acetate - hexane) to get 1-(2-methoxyethyl)-1H-pyrazole-4-carbaldehyde (1.0 g, 62%).

Preparation Example 21

(1-(2-Methoxyethyl)-1H-pyrazol-4-yl)methanol

To an ice cold stirred solution of 1-(2-methoxyethyl)-1H-pyrazole-4-carbaldehyde (1g, 6.4 mmol) in methanol (8.0 mL) and tetrahydrofuran (2 mL) was added sodium borohydride (488 mg, 13.6 mmol) in portion wise manner, and reaction mass was stirred at room temperature for 2h. The solvent was evaporated under reduced pressure, and the residue was purified over silica gel column chromatography (50% ethyl acetate - hexane) to get (1-(2-methoxyethyl)-1H-pyrazol-4-yl)methanol (700 mg, 69%).

Preparation Example 22

tert-Butyl ((1-((1-(2-methoxyethyl)-1H-pyrazol-4-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a stirred solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (78.5 mg, 0.15 mmol) and (1-(2-methoxyethyl)-1H-pyrazol-4-yl)methanol (50 mg, 0.317 mmol) in dichloromethane (5 mL) was added triphenylphosphine (166 mg, 0.63 mmol) and diisopropyl azodicarboxylate (192 mg, 0.95 mmol) at room temperature, and the reaction mixture was stirred at 45°C for 2h. The solvent was evaporated under reduced pressure, and the residue was purified by silica gel column chromatography (5% methanol - dichloromethane) to get tert-butyl ((1-((1-(2-methoxyethyl)-1H-pyrazol-4-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (50 mg, 25%) as off white solid.

Example 57

1-((1-(2-Hydroxyethyl)-1H-pyrazol-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To a stirred solution of tert-butyl ((1-((1-(2-methoxyethyl)-1H-pyrazol-4-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (50 mg, 0.07 mmol) in dichloromethane (5 mL), added boron tribromide (80 mg, 0.31 mmol) at -78°C and slowly raised to room temperature, and the reaction mixture was stirred for 1h. The reaction mixture was quenched with methanol (5 mL), and evaporated under reduced pressure to get the residue which was dissolved in ethyl acetate and washed with saturated sodium bicarbonate aqueous solution (3 x 50 mL). Organic layer was dried over anhydrous sodium sulfate, and evaporated under reduced pressure. The residue was purified over reverse phase prep-HPLC to get 1-((1-(2-hydroxyethyl)-1H-pyrazol-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (4 mg, 15%) as off white solid.

Preparation Example 23

tert-Butyl 4-(6-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)piperidine-1-carboxylate

To a stirred solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (3.0 g, 7.59 mmol), tert-butyl 4-hydroxypiperidine-1-carboxylate (2.29 g, 11.4 mmol) in dichloromethane (30.0 mL) was added triphenylphosphine (3.98 g, 15.2 mmol) and diisopropyl azodicarboxylate (4.60 g, 22.8 mmol) at room temperature and the resulting reaction mixture was stirred at 45°C for 2h. The solvent was evaporated under reduced pressure, and the residue was purified over silica gel column chromatography (5% methanol - dichloromethane to get tert-butyl 4-(6-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)piperidine-1-carboxylate (4g).

Example 91

3-((1-Methyl-1H-pyrazol-4-yl)methyl)-N-(1-methylcyclopropyl)-2,4-dioxo-1-(piperidin-4-yl)-1,2,3,4-tetrahydrothieno[2,3-d]pyrimidine-6-sulfonamide trifluoroacetic acid salt

To an ice cold stirred solution of tert-butyl 4-(6-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)piperidine-1-carboxylate (4 g) in dichloromethane (20 mL) was added a 25% solution of trifluoroacetic acid in dichloromethane (4.50 mL) and the mixture was stirred at room temperature for 2h. The solvent was evaporated under reduced pressure, and the residue was triturated with diethyl ether to get the 3-((1-methyl-1H-pyrazol-4-yl)methyl)-N-(1-methylcyclopropyl)-2,4-dioxo-1-(piperidin-4-yl)-1,2,3,4-tetrahydrothieno[2,3-d]pyrimidine-6-sulfonamide trifluoroacetic acid salt (1.10 g, 39%) as pale yellow color solid. A small portion of solid (about 150 mg) was purified by reverse phase prep-HPLC using ammonium bi-carbonate buffer to get 3-((1-methyl-1H-pyrazol-4-yl)methyl)-N-(1-methylcyclopropyl)-2,4-dioxo-1-(piperidin-4-yl)-1,2,3,4-tetrahydrothieno[2,3-d]pyrimidine-6-sulfonamide trifluoroacetic acid salt as white solid.

Example 92

1-(1-Acetylpiperidin-4-yl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice cold stirred solution of 3-((1-methyl-1H-pyrazol-4-yl)methyl)-N-(1-methylcyclopropyl)-2,4-dioxo-1-(piperidin-4-yl)-1,2,3,4-tetrahydrothieno[2,3-d]pyrimidine-6-sulfonamide trifluoroacetic acid salt (120 mg, 0.25 mmol) in dichloromethane (10 mL) was added pyridine (100 μL, 1.25 mmol) and acetic anhydride (39 mg, 0.37 mmol). The resulting reaction mixture was stirred at room temperature for 2h. The reaction mixture was diluted with saturated sodium bicarbonate aqueous solution and extracted with ethyl acetate. The organic layer was dried over anhydrous sodium sulfate, and evaporated under reduced pressure. The residue was purified over reverse phase prep-HPLC to get 1-(1-acetylpiperidin-4-yl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (32 mg, 25%) as off white solid.

Preparation Example 24

tert-Butyl methyl(3-(4-(3-((1 -methyl-1H-pyrazol-4-yl)methyl)-6-(N-(1-methylcyclopropyl)sulfamoyl)-2,4-dioxo-3,4-dihydrothieno[2,3-d]pyrimidin-1(2H)-yl)piperidin-1-yl)-3-oxopropyl)carbamate

To a stirred solution of 1-(1-acetylpiperidin-4-yl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide trifluoroacetic acid salt (120 mg, 0.25 mmol) and 3-((tert-butoxycarbonyl)(methyl)amino)propanoic acid (77 mg, 0.37 mmol) in dichloromethane (5 mL) was added diisopropylethylamine (274 μL, 1.57 mmol), 1-hydroxybenzotriazole (85 mg, 0.62 mmol) and 1-ethyl-3-(3-dimethylaminopropyl)carbodiimide hydrochloride (120 mg, 0.62 mmol) and the mixture was stirred at room temperature for 1h. The reaction mixture was diluted with saturated ammonium chloride aqueous solution (100 mL) and extracted with ethyl acetate. The organic layer was dried over anhydrous sodium sulfate, and evaporated under reduced pressure. The residue was purified over silica gel column chromatography (5% methanol - dichloromethane) to get tert-butyl methyl(3-(4-(3-((1-methyl-1H-pyrazol-4-yl)methyl)-6-(N-(1-methylcyclopropyl)sulfamoyl)-2,4-dioxo-3,4-dihydrothieno[2,3-d]pyrimidin-1(2H)-yl)piperidin-1-yl)-3-oxopropyl)carbamate (100 mg, 48%) as off white solid.

Example 95

3-((1-Methyl-1H-pyrazol-4-yl)methyl)-1-(1-(3-(methylamino)propanoyl)piperidin-4-yl)-N-(1-methylcyclopropyl)-2,4-dioxo-1,2,3,4-tetrahydrothieno[2,3-d]pyrimidine-6-sulfonamide

To an ice cold stirred solution of tert-butyl methyl(3-(4-(3-((1-methyl-1H-pyrazol-4-yl)methyl)-6-(N-(1-methylcyclopropyl)sulfamoyl)-2,4-dioxo-3,4-dihydrothieno[2,3-d]pyrimidin-1(2H)-yl)piperidin-1-yl)-3-oxopropyl)carbamate (100 mg, 0.15 mmol) in dichloromethane (5 mL), added about 25% solution of trifluoroacetic acid in dichloromethane (5 mL), and the mixture was stirred at room temperature for 3h. The solvent was evaporated under reduced pressure to get the crude which was dissolved in dichloromethane and washed with saturated sodium bicarbonate solution. Organic layer was dried over anhydrous sodium sulfate, and evaporated under reduced pressure. The residue was purified by reverse phase prep-HPLC purification to get 3-((1-methyl-1H-pyrazol-4-yl)methyl)-1-(1-(3-(methylamino)propanoyl)piperidin-4-yl)-N-(1-methylcyclopropyl)-2,4-dioxo-1,2,3,4-tetrahydrothieno[2,3-d]pyrimidine-6-sulfonamide (22 mg, 25%) as off white solid.

Preparation Example 25

Methyl 4-(2,2-dimethoxyethoxy)-3-formylbenzoate

To a stirred solution of methyl 3-formyl-4-hydroxybenzoate (1.00 g, 5.55 mmol) in N,N-dimethylformamide (12 mL) was added potassium carbonate (3.07 g, 22.2 mmol) and 2-bromo-1,1-dimethoxyethane (1.86 g, 11.1 mmol) at room temperature, and the mixture was stirred at 90°C for 24h. The reaction mixture was diluted with cold water and extracted with ethyl acetate. The organic layer was dried over anhydrous sodium sulfate, fand evaporated under reduced pressure. The residue was purified over silica gel column chromatography (25% ethyl acetate - hexane) to get methyl 4-(2,2-dimethoxyethoxy)-3-formylbenzoate (668 mg, 59%).

Preparation Example 26

Methyl 2-formylbenzofuran-5-carboxylate

A mixture of methyl 4-(2,2-dimethoxyethoxy)-3-formylbenzoate (668 mg, 2.49 mmol) and acetic acid (5 mL) was heated up to 120°C and stirred at the same temperature for 16h. The solvent was evaporated under reduced pressure, and the residue was dissolved in ethyl acetate and washed with saturated sodium bicarbonate aqueous solution (3 x 100 mL). Organic layer was dried over anhydrous sodium sulfate, and evaporated under reduced pressure. The residue was purified over silica gel column chromatography (10% ethyl acetate - heptane) to get methyl 2-formylbenzofuran-5-carboxylate (628 mg, 95%).

Preparation Example 27

Methyl 2-(hydroxymethyl)benzofuran-5-carboxylate

To an ice cold solution of methyl 2-formylbenzofuran-5-carboxylate (600 mg, 2.94 mmol) in a mixture of methanol

(8.0 mL) and tetrahydrofuran (2 mL) was added sodium borohydride (210 mg, 5.88 mmol) in portion wise manner. The reaction mass was stirred at room temperature for 2h. The solvent was evaporated under reduced pressure, and the residue was purified over silica gel column chromatography (70% ethyl acetate - hexane) to get methyl 2-(hydroxymethyl)benzofuran-5-carboxylate (220 mg, 36%).

Preparation Example 28

Methyl 2-(((tert-butyldimethylsilyl)oxy)methyl)benzofuran-5-carboxylate

To a solution of methyl 2-(hydroxymethyl)benzofuran-5-carboxylate (160 mg , 0.77 mmol) in dichloromethane (5 mL) was added 1H-imidazole (211 mg, 3.10 mmol) and tert-butyldimethylsilyl chloride (175 mg, 1.16 mmol), and the reaction mixture as stirred at room temperature for 2h. The reaction mixture was diluted with water and extracted with ethyl acetate. The combined organic layer was dried over anhydrous sodium sulfate, and evaporated under reduced pressure to get methyl 2-(((tert-butyldimethylsilyl)oxy)methyl)benzofuran-5-carboxylate (196 mg, 78%).

Preparation Example 29

(2-(((tert-Butyldimethylsilyl)oxy)methyl)benzofuran-5-yl)methanol

To a stirred solution of methyl 2-(((tert-butyldimethylsilyl)oxy)methyl)benzofuran-5-carboxylate (475 mg, 1.48 mmol) in tetrahydrofuran (5 mL) added 1.0M solution of diisobutylaluminum hydride in toluene (7.0 mL, 6.78 mmol) at -78°C and slowly raised the temperature up to room temperature and maintained the same temperature for 2h. The reaction mixture was quenched with ice cold water and extracted with ethyl acetate. The organic layer was dried over anhydrous sodium sulfate, and evaporated under reduced pressure. The residue was purified by silica gel column chromatography (25% ethyl acetate - hexane) to get (2-(((tert-butyldimethylsilyl)oxy)methyl)benzofuran-5-yl)methanol (305 mg, 70%).

Preparation Example 30

tert-Butyl ((1-((2-(((tert-butyldimethylsilyl)oxy)methyl)benzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (80 mg, 0.16 mmol) and (2-(((tert-butyldimethylsilyl)oxy)methyl)benzofuran-5-yl)methanol (95 mg, 0.32 mmol) in dichloromethane (10 mL) was added triphenylphosphine (127 mg, 0.48 mmol) and diisopropyl azodicarboxylate (95 μL, 0.48 mmol) at room temperature and the reaction mass was stirred at 45°C for 2h. The solvent was evaporated under reduced pressure, and the residue was purified by silica gel column chromatography (20% ethyl acetate - hexane) to get tert-butyl ((1-((2-(((tertbutyldimethylsilyl)oxy)methyl)benzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (70 mg, 56%).

Example 64

1-((2-(Hydroxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice cold solution of tert-butyl ((1-((2-(((tertbutyldimethylsilyl)oxy)methyl)benzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (70 mg, 0.09 mmol) in dichloromethane (5 mL) was added 25% solution of trifluoroacetic acid in dichloromethane (5 mL). The reaction mass was stirred at room temperature for 3h. The solvent was evaporated under reduced pressure, and the residue was re-dissolved in dichloromethane and washed with saturated sodium bicarbonate aqueous solution. Organic layer was dried over anhydrous sodium sulfate, and evaporated under reduced pressure. The residue was purified by reverse phase prep-HPLC purification to get 1-((2-(hydroxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (17 mg, 33%) as off white solid.

Preparation Example 31

tert-Butyl (2-((2-fluoro-4-formylphenyl)(methyl)amino)ethyl)(methyl)carbamate

To a solution of 3,4-difluorobenzaldehyde (500 mg, 3.52 mmol) in dimethyl sulfoxide (10 mL) was added tetrabutylammonium bromide (567 mg, 1.76 mmol), potassium carbonate (486 mg, 3.52 mmol) and tert-butyl methyl(2-(methylamino)ethyl)carbamate (795 mg, 4.22 mmol) at room temperature. The reaction mixture was stirred at 90°C for 10h. The reaction mixture was diluted with saturated sodium bicarbonate solution and extracted with ethyl acetate. The organic layer was dried over anhydrous sodium sulfate, and evaporated under reduced pressure. The residue was purified over silica gel column chromatography (15% ethyl acetate - hexane) to get tert-butyl (2-((2-fluoro-4-formylphenyl)(methyl)amino)ethyl)(methyl)carbamate (650 mg, 59%).

Preparation Example 32

tert-Butyl (2-((2-fluoro-4-(hydroxymethyl)phenyl)(methyl)amino)ethyl)(methyl)carbamate

To an ice cold solution of tert-butyl (2-((2-fluoro-4-formylphenyl)(methyl)amino)ethyl)(methyl)carbamate (600 mg, 1.93 mmol) in a mixture of methanol (8 mL) and tetrahydrofuran (2 mL) was added sodium borohydride (261 mg, 7.29 mmol) in portion wise manner, and the reaction mixture was stirred at room temperature for 2h. The reaction mixture was quenched with cold water and extracted with ethyl acetate. The organic layer was dried over anhydrous sodium sulfate, and evaporated under reduced pressure. The residue was purified over silica gel column chromatography (10% ethyl acetate - hexane) to get tert-butyl (2-((2-fluoro-4-(hydroxymethyl)phenyl)(methyl)amino)ethyl)(methyl)carbamate (500 mg, 83%).

Preparation Example 33

tert-Butyl ((1-(4-((2-((tert-butoxycarbonyl)(methyl)amino)ethyl)(methyl)amino)-3-fluorobenzyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (250 mg, 0.50 mmol) and tert-butyl (2-((2-fluoro-4-(hydroxymethyl)phenyl)(methyl)amino)ethyl)(methyl)carbamate (315 mg, 1.01 mmol) in dichloromethane (10.0 mL) was added triphenylphosphine (265 mg, 1.01 mmol) and diisopropyl azodicarboxylate (297 μL, 1.51 mmol) at room temperature. The reaction mass was stirred at 45°C for 2h. The solvent was evaporated under reduced pressure, and the residue was purified by silica gel column chromatography (50% ethyl acetate - hexane) to get tert-butyl ((1-(4-((2-((tert-butoxycarbonyl)(methyl)amino)ethyl)(methyl)amino)-3-fluorobenzyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (650 mg) as yellow solid.

Example 77

1-(3-Fluoro-4-(methyl(2-(methylamino)ethyl)amino)benzyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To an ice cold solution of tert-butyl ((1-(4-((2-((tert-butoxycarbonyl)(methyl)anuno)ethyl)(methyl)amino)-3-fluorobenzyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (300 mg, 0.38 mmol) in dichloromethane (10 mL) was added 25% solution of trifluoroacetic acid in dichloromethane (10 mL), and the reaction mixture was stirred at room temperature for 3h. The solvent was evaporated under reduced pressure, and the residue was re-dissolved in dichloromethane and washed with saturated sodium bicarbonate aqueous solution. Organic layer was dried over anhydrous sodium sulfate, and evaporated under reduced pressure. The residue was purified by reverse phase prep-HPLC purification to get 1-(3-fluoro-4-(methyl(2-(methylamino)ethyl)amino)benzyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (28 mg, 12%) as off white solid.

Example 78

1-(3-Fluoro-4-(methyl(2-(dimethylamino)ethyl)amino)benzyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To a solution of 1-(3-fluoro-4-(methyl(2-(methylamino)ethyl)amino)benzyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (200 mg, 0.30 mmol) in a mixture of methanol (5 mL) and acetic acid (1 mL) was added 37% aq. formaldehyde (106 μL, 1.70 mmol) and stirred for 30 min at room temperature. To the reaction mixture was added sodium cyanoborohydride (55 mg, 0.88 mmol), and was stirred at room temperature for further 16h. The solvent was evaporated under reduced pressure, and the residue was re-dissolved in dichloromethane and washed with saturated sodium bicarbonate solution. Organic layer was dried over anhydrous sodium sulfate, and evaporated under reduced pressure. The residue was purified by reverse phase prep-HPLC purification to get 1-(3-fluoro-4-(methyl(2-(dimethylamino)ethyl)amino)benzyl)-3-((1-methyl-1H-pyrazol-4-yl)methyl)-N-(1-methylcyclopropyl)-2,4-dioxo-1,2,3,4-tetrahydrothieno[2,3-d]pyrinudine-6-sulfonamide (26 mg, 13%).

Preparation Example 34

tert-butyl 4-((6-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)methyl)piperidine-1 -carboxylate

To a solution of tert-butyl ((3-((1-nnethyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (200 mg, 0.40 mmol) and tert-butyl 4-(hydroxymethyl)piperidine-1-carboxylate (217 mg, 1.01 mmol) in dichloromethane (5 mL) was added triphenylphosphine (318 g, 1.2 mmol) and diisopropyl azodicarboxylate (237 μL, 1.2 mmol) at room temperature, and the reaction mass was stirred at room temperature for 2h. The solvent was evaporated under reduced pressure, and the residue was purified by silica gel column chromatography (60% ethyl acetate - hexane) to get tert-butyl 4-((G-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)methyl)piperidine-1-carboxylate (200 mg) as yellow solid.

Example 97

3-((1-Methyl-1H-pyrazol-4-yl)methyl)-N-(1-methylcyclopropyl)-2,4-dioxo-1-(piperidin-4-ylmethyl)-1,2,3,4-tetrahydrothieno[2,3-d]pyrimidine-6-sulfonamide

To an ice-cold solution of tert-butyl 4-((6-(N-(tert-butoxycarbonyl)-N-(1-methylcyclopropyl)sulfamoyl)-3-((1-methyl-1H-pyrazol-4-yl)methyl)-2,4-dioxo-3,4-dihydrothieno[2,3-d]pyrimidin-1(2H)-yl)methyl)piperidine-1-carboxylate (200 mg, 0.29 mmol) in dichloromethane (5 mL) was added 25% solution of trifluoroacetic acid in dichloromethane (5 mL), and the reaction mixture was stirred at room temperature for 3h. The solvent was evaporated under reduced pressure, and the residue was re-dissolved in dichloromethane and washed with sodium bicarbonate aqueous solution. Organic layer was dried over anhydrous sodium sulfate, and evaporated under reduced pressure. The residue was purified by reverse phase prep-HPLC purification to get 3-((1-methyl-1H-pyrazol-4-yl)methyl)-N-(1-methylcyclopropyl)-2,4-dioxo-1-(piperidin-4-ylmethyl)-1,2,3,4-tetrahydrothieno[2,3-d]pyrinudine-6-sulfonamide as off white solid (140, 40%).

To an ice-cold solution of the obtained compound (30 mg, 0.06 mmol) in methanol (2 mL) was added 4M solution of hydrogen chloride in Dioxane (1 mL) and the reaction mixture was stirred at room temperature for 30 min. The reaction mixture was then evaporated under reduced pressure and the residue was triturated with diethyl ether, and collected by filtration to get 3-((1-methyl-1H-pyrazol-4-yl)methyl)-N-(1-methylcyclopropyl)-2,4-dioxo-1-(piperidin-4-ylmethyl)-1 ,2,3,4-tetrahydrothieno[2,3-d]pyrimidine-6-sulfonamide hydrochloride (25 mg, 78%).

Example 98

3-((1-Methyl-1H-pyrazol-4-yl)methyl)-N-(1-methylcyclopropyl)-1-((1-methylpiperidin-4-yl)methyl)-2,4-dioxo-1,2,3,4-tetrahydrothieno[2,3-d]pyrimidine-6-sulfonamide

To a solution of 3-((1-methyl-1H-pyrazol-4-yl)methyl)-N-(1-methylcyclopropyl)-2,4-dioxo-1-(piperidin-4-ylmethyl)-1,2,3,4-tetrahydrothieno[2,3-d]pyrimidine-6-sulfonamide (90 mg, 0.18 mmol) in a mixture of methanol (5 mL) and acetic acid (1 mL) was added 37% aq. formaldehyde (106 μL, 1.26 mmol) and stirred for 30 min at room temperature followed by addition of sodium cyanoborohydride (23 mg, 0.36 mmol) and continued stirring at room temperature for

16h. The solvent was evaporated under reduced pressure, and the residue was re-dissolved in dichloromethane and washed with sodium bicarbonate aqueous solution. Organic layer was dried over anhydrous sodium sulfate, and evaporated under reduced pressure. The residue was purified by reverse phase prep-HPLC purification to get 3-((1-methyl-1H-pyrazol-4-yl)methyl)-N-(1-methylcyclopropyl)-1-((1-methylpiperidin-4-yl)methyl)-2,4-dioxo-1,2,3,4-tetrahydrothieno[2,3-d]pyrimidine-6-sulfonamide (12 mg, 13%) as off white solid.

Preparation Example 35

1-((1-(2-((tert-Butyldinnethylsilyl)oxy)ethyl)piperidin-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To a stirred solution of 3-((1-methyl-1H-pyrazol-4-yl)methyl)-N-(1-methylcyclopropyl)-2,4-dioxo-1-(piperidin-4-ylmethyl)-1,2,3,4-tetrahydrothieno[2,3-d]pyrimidine-6-sulfonamide (120 mg, 0.24 mmol) in a mixture of methanol (5 mL) and acetic acid (1 mL) added 2-((tert-butyldimethylsilyl)oxy)acetaldehyde 48 (84 mg, 0.48 mmol) and stirred for 30 min at room temperature, followed by addition of sodium cyanoborohydride (30 mg, 0.48 mmol) and continued stirring at room temperature for 16h. The solvent was evaporated under reduced pressure, and the residue was re-dissolved in dichloromethane and washed with sodium bicarbonate aqueous solution. Organic layer was dried over anhydrous sodium sulfate, and evaporated under reduced pressure. The residue was purified by silica gel column chromatography (50% ethyl acetate - hexane) to get 1-((1-(2-((tert-butyldimethylsilyl)oxy)ethyl)piperidin-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (100 mg, 44%) as off white solid.

Example 99

1-((1-(2-Hydroxyethyl)piperidin-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

To a stirred solution of 1-((1-(2-((tert-butyldimethylsilyl)oxy)ethyl)piperidin-4-yl)methyl)-3-((1-methyl-1H-pyrazol-4-yl)methyl)-N-(1 -methylcyclopropyl)-2,4-dioxo-1,2,3,4-tetrahydrothieno[2,3-d]pyrimidine-6-sulfonamide (100 mg, 0.15 mmol) in tetrahydrofuran (5 mL) was added 1M solution of tetrabutylammonium fluoride in tetrahydrofuran (760 μL, 0.76 mmol) and stirred at room temperature for 1h. The reaction mixture was diluted with water and extracted with ethyl acetate. The organic layer was dried over anhydrous sodium sulfate, and evaporated under reduced pressure. The residue was purified over reverse phase prep-HPLC to get 1-((1-(2-hydroxyethyl)piperidin-4-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (16 mg, 19%) as off white solid.

Preparation Example 36

N-(1-Cyanocyclopropyl)-3-((1-methyl-1H-pyrazol-4-yl)methyl)-2,4-dioxo-1,2,3,4-tetrahydrothieno[2,3-d]pyrimidine-6-sulfonamide

To an ice-cold solution of 3-((1-methyl-1H-pyrazol-4-yl)methyl)-2,4-dioxo-1,2,3,4-tetrahydrothieno[2,3-d]pyrimidine-6-sulfonyl chloride (9.5 g, 26.33 mmol) in a mixture of tetrahydrofuran and dichloromethane (200 mL, 1:1) was added diisopropylamine (23.45 mL, 131.65 mmol) and 1-aminocyclopropane-1-carbonitrile hydrochloride (6.24 g, 52.66 mmol), and the reaction mixture was stirred at room temperature for 2 h. The reaction solvent was evaporated under reduced pressure, and the residue was diluted with water and extracted with dichloromethane. The organic layer was dried over anhydrous sodium sulfate and concentrated under reduced pressure. The residue was triturated with diethyl ether, solid was collected by filtration to get N-(1-cyanocyclopropyl)-3-((1-methyl-1H-pyrazol-4-yl)methyl)-2,4-dioxo-1,2,3,4-tetrahydrothieno[2,3-d]pyrimidine-6-sulfonamide (5.50 g, 51%).

Preparation Example 37

tert-Butyl (1-cyanocyclopropyl)((3-((1-methyl-1H-pyrazol-4-yl)methyl)-2,4-dioxo-1,2,3,4-tetrahydrothieno[2,3-d]pyrimidin-6-yl)sulfonyl)carbamate

To an ice-cold solution of N-(1-cyanocyclopropyl)-3-((1-methyl-1H-pyrazol-4-yl)methyl)-2,4-dioxo-1,2,3,4-tetrahydrothieno[2,3-d]pyrimidine-6-sulfonamide (5.50 g, 13.53 mmol) in pyridine (50 mL) were added 4-dimethylaminopyridine (0.17 g, 1.35 mmol) and di-tert-butyl dicarbonate (11.8 mL, 54.12 mmol), and the reaction mixture was stirred at

room temperature for 6 h. The reaction solvent was evaporated under reduced pressure, and the residue was diluted with water and extracted with dichloromethane. The organic layer was washed with 1 M hydrochloric acid and saturated saline, dried over anhydrous sodium sulfate, and concentrated under reduced pressure. The residue solid was triturated with diethyl ether to get tert-butyl (1-cyanocyclopropyl)((3-((1-methyl-1H-pyrazol-4-yl)methyl)-2,4-dioxo-1,2,3,4-tetrahydrothieno[2,3-d]pyrimidin-6-yl)sulfonyl)carbamate (4.2 g, 61%).

Preparation Example 38

tert-Butyl (1-cyanocyclopropyl)((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)carbamate

To a solution of tert-butyl (1-cyanocyclopropyl)((3-((1-nnethyl-1H-pyrazol-4-yl)methyl)-2,4-dioxo-1,2,3,4-tetrahydrothieno[2,3-d]pyrimidin-6-yl)sulfonyl)carbamate (200 mg, 0.39 mmol) and 5-(chloromethyl)-1,3-dihydroisobenzofuran (100 mg, 0.59 mmol) in N,N-dimethylformamide (5 mL) were added potassium carbonate (108 mg, 0.78 mmol) and sodium iodide (116 mg, 0.78 mmol), and the reaction mixture was stirred at room temperature for 1 h. The reaction solution was diluted with ice-cold water, and extracted with dichloromethane. The organic layer was dried over anhydrous sodium sulfate and concentrated under reduced pressure. The residue was purified by silica gel column chromatography (3% methanol-dichloromethane) to get tert-butyl (1-cyanocyclopropyl)((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)carbamate (220 mg, 63%) as off white solid.

Example 100

N-(1-Cyanocyclopropyl)-1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidine-6-sulfonamide

To an ice-cold solution of tert-butyl (1-cyanocyclopropyl)((1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidin-6-yl)sulfonyl)carbamate (200 mg, 9.38 mmol) in dichloromethane (10 mL) was added trifluoroacetic acid (2.5 mL), and the reaction mixture was stirred at room temperature for 1 h. The reaction solvent was evaporated under reduced pressure, and the residue was dissolved in dichloromethane (50 mL) and washed with saturated sodium bicarbonate aqueous solution. The organic layer was dried over anhydrous sodium sulfate, and concentrated under reduced pressure. The residue was purified over reverse phase prep-HPLC to get N-(1-cyanocyclopropyl)-1-((1,3-dihydroisobenzofuran-5-yl)methyl)-3-((1-methyl-1H-pyrazol-4-yl)methyl)-2,4-dioxo-1,2,3,4-tetrahydrothieno[2,3-d]pyrimidine-6-sulfonamide (13 mg, 8%) as off white solid.

Preparation Example 39

1-(2-Methoxyethyl)-1H-pyrazole-4-carbaldehyde

To a solution of 1H-pyrazole-4-carbaldehyde (4.0 g, 41.65 mmol) in acetonitrile (50 mL) were added potassium carbonate (17.24 g, 124.96 mmol) and 1-bromo-2-methoxyethane (6.94 g, 49.98 mmol). The reaction mixture was stirred at 80°C for 16 h. The reaction mixture was diluted with water and extracted with ethyl acetate. The organic layer was washed with saturated saline, dried over anhydrous sodium sulfate, and concentrated under reduced pressure. The residue was triturated with pentane, and the mixture was stirred, and the solid was collected by filtration to get 1-(2-methoxyethyl)-1H-pyrazole-4-carbaldehyde (3.9 g, 61%).

Preparation Example 40

(E)-1-(2-Methoxyethyl)-1H-pyrazole-4-carbaldehyde oxime

To a solution of 1-(2-methoxyethyl)-1H-pyrazole-4-carbaldehyde (5.5 g, 35.25 mmol) in methanol (40 mL) was added hydroxylamine hydrochloride (4.90 g, 70.51 mmol), and the reaction mixture was stirred at room temperature for 16 h. The reaction solvent was evaporated under reduced pressure, and the residue was triturated with diethyl ether. The solid was collected by filtration to get (E)-1-(2-methoxyethyl)-1H-pyrazole-4-carbaldehyde oxime (5.5 g, 91%).

Preparation Example 41

(1-(2-Methoxyethyl)-1H-pyrazol-4-yl)methanamine hydrochloride

To a solution of (E)-1-(2-methoxyethyl)-1H-pyrazole-4-carbaldehyde oxime (4.5 g, 26.59 mmol) in a mixture of methanol and 1 M hydrochloric acid (10:0.5; 50 mL) was added 10 % palladium carbon (w/w, 0.45 g). The reaction mixture was stirred for 16 h under hydrogen atmosphere, filtered through celite, and washed with methanol. The filtrate was concentrated under reduced pressure to get (1-(2-methoxyethyl)-1H-pyrazol-4-yl)methanamine hydrochloride (2.6 g, 56%).

Preparation Example 42

2-Amino-N-((1-(2-methoxyethyl)-1H-pyrazol-4-yl)methyl)thiophene-3-carboxamide

A solution of methyl 2-aminothiophene-3-carboxylate (2.46 g, 15.65 mmol) and (1-(2-methoxyethyl)-1H-pyrazol-4-yl)methanamine hydrochloride (1.5g, 7.82 mmol) in 1,4-dioxane (30 mL) was cooled with ice, and then a 2M solution of trimethylaluminum in toluene (19.5 mL, 39.1 mmol) was added dropwise thereto. The reaction mixture was heated to 80°C and stirred for 16 h, diluted with cold saturated ammonium chloride aqueous solution, and extracted with ethyl acetate. The organic layer was washed with saturated saline, dried over anhydrous sodium sulfate, and concentrated under reduced pressure. The residue was triturated with diethyl ether, and the solid was collected by filtration to get 2-amino-N-((1-(2-methoxyethyl)-1H-pyrazol-4-yl)methyl)thiophene-3-carboxamide (1.0 g, 46%).

Preparation Example 43

3-((1-(2-Methoxyethyl)-1H-pyrazol-4-yl)methyl)thieno[2,3-d]pyrimidine-2,4(1H,3H)-dione

To a solution of 2-amino-N-((1-(2-methoxyethyl)-1H-pyrazol-4-yl)methyl)thiophene-3-carboxamide (1.0 g, 3.56 mmol) in tetrahydrofuran (25 mL) was added 1,1'-carbonyldiimidazole (1.74 g, 10.70 mmol) in portion wise manner, and the reaction mixture was stirred at 80°C for 16 h. The reaction solvent was evaporated under reduced pressure, the residue was triturated with diethyl ether, and the solid was collected by filtration to get 3-((1-(2-methoxyethyl)-1H-pyrazol-4-yl)methyl)thieno[2,3-d]pyrimidine-2,4(1H,3H)-dione (700 mg, 64%).

Preparation Example 44

3-((1-(2-Methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide

Chlorosulfonic acid (1.1 mL, 16.0 mmol) was cooled to 0°C, and then 3-((1-(2-methoxyethyl)-1H-pyrazol-4-yl)nnethyl)thieno[2,3-d]pyrimidine-2,4(1H,3H)-dione (700 mg, 2.28 mmol) was added thereto in portion wise manner, and the reaction mixture was heated to 70°C and stirred for 4 h. After the reaction mixture was cooled to room temperature, ice-cold water (100 mL) was slowly added thereto, and the resulting solid was washed with cold water and dried under reduced pressure to get 3-((1-(2-methoxyethyl)-1H-pyrazol-4-yl)methyl)-2,4-dioxo-1 ,2,3,4-tetrahydrothieno[2,3-d]pyrimidine-6-sulfonyl chloride (550 mg, 60%).

A solution of the obtained sulfonyl chloride in a mixture of tetrahydrofuran and dichloromethane (1:1, 10 mL) was cooled with ice, and then diisopropylethylamine (2.13 mL, 12.23 mmol) and 1-methylcyclopropan-1-amine hydrochloride (255 mg, 2.72 mmol) were added thereto, and the reaction mixture was stirred for 16 h. The reaction solvent was evaporated under reduced pressure, the residue was diluted with water and extracted with dichloromethane. The organic layer was dried over anhydrous sodium sulfate and concentrated under reduced pressure, the residue was triturated with diethyl ether, and the solid was collected by filtration to get 3-((1-(2-methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (135 mg, 23%).

Preparation Example 45

tert-Butyl ((3-((1-(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

A solution of 3-((1-(2-methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (135 mg, 0.31 mmol) in pyridine (3.0 mL) was cooled with ice, and then

4-dimethylaminopyridine (4.0 mg, 0.03 mmol) and di-tert-butyl dicarbonate (0.34 mL, 1.55 mmol) were added thereto, and the reaction mixture was heated to 90°C and stirred for 6 h. The reaction solvent was evaporated under reduced pressure, the residue was diluted with water and extracted with dichloromethane. The organic layer was washed with saturated saline, dried over anhydrous sodium sulfate, and concentrated under reduced pressure. The residue was triturated with diethyl ether, and solid was collected by filtration to get tert-butyl ((3-((1-(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (120 mg, 73%).

Preparation Example 46

tert-Butyl ((1-((2,4-dimethylthiazol-5-yl)methyl)-3-((1 -(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl ((3-((1-(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (120 mg, 0.22 mmol) in N,N-dimethylformamide (5 mL) were added potassium carbonate (92 mg, 0.66 mmol) and 5-(chloromethyl)-2,4-dimethylthiazole (43 mg, 0.26 mmol), and the reaction mixture was stirred at room temperature for 1 h. The reaction mixture was diluted with ice-cold water, extracted with dichloromethane, dried over anhydrous sodium sulfate, and concentrated under reduced pressure. The residue was purified by silica gel column chromatography (2% methanol-dichloromethane) to get tert-butyl ((1-((2,4-dimethylthiazol-5-yl)methyl)-3-((1-(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (120 mg, 82%) as off white solid.

Example 104

1-((2,4-Dimethylthiazol-5-yl)methyl)-3-((1-(2-methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (Example 104a) and
1-((2,4-Dimethylthiazol-5-yl)methyl)-3-((1-(2-hydroxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (Example 104b)

A solution of tert-butyl ((1-((2,4-dimethylthiazol-5-yl)methyl)-3-((1-(2-methoxyethyl)-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (120 mg, 0.21 mmol) in dichloromethane (5 mL) was cooled with ice, and then trifluoroacetic acid (1.0 mL) was added thereto. The reaction solvent was evaporated under reduced pressure, and the residue was re-dissolved in dichloromethane (50 mL) and washed with saturated sodium bicarbonate aqueous solution. The organic layer was dried over anhydrous sodium sulfate and concentrated under reduced pressure to get 1-((2,4-dimethylthiazol-5-yl)methyl)-3-((1-(2-methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (Example 104a: 75 mg, 73%).

A solution of 1-((2,4-dimethylthiazol-5-yl)methyl)-3-((1-(2-methoxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (75 mg, 0.13 mmol) in dichloromethane (5 mL) was cooled with ice, and then boron tribromide (25 μL, 0.26 mmol) was added dropwise thereto, and the reaction mixture was stirred at room temperature for 16 h. After the reaction mixture was cooled to 0°C, methanol (0.5 mL) was added thereto, and the reaction solvent was concentrated under reduced pressure. The residue was re-dissolved in dichloromethane (50 mL) and washed with saturated sodium bicarbonate. The organic layer was dried over anhydrous sodium sulfate, and concentrated under reduced pressure. The residue was purified over reverse phase prep-HPLC to get 1-((2,4-dimethylthiazol-5-yl)methyl)-3-((1-(2-hydroxyethyl)-1H-pyrazol-4-yl)methyl)-N-(1-methylcyclopropyl)-2,4-dioxo-1,2,3,4-tetrahydrothieno[2,3-d]pyrimidine-6-sulfonamide (Example 104b: 10 mg, 14%) as off white solid.

Preparation Example 48

2-Amino-N-((6-chloropyridin-3-yl)methyl)thiophene-3-carboxamide

A solution of methyl 2-aminothiophene-3-carboxylate (250 mg, 1.59 mmol) and (6-chloropyridin-3-yl)methanamine (272 mg, 1.91 mmol) in 1,4-dioxane (10 mL) was cooled with ice, and then a 2M solution of trimethylaluminum in toluene (3.98 mL, 7.95 mmol) was added dropwise thereto, and the reaction mixture was heated to 100°C and stirred for 16 h. The reaction mixture was washed with saturated ammonium chloride aqueous solution and extracted with ethyl acetate. The organic layer was washed with saturated saline, dried over anhydrous sodium sulfate, and concentrated

under reduced pressure. The residue was purified by silica gel column chromatography (80% ethyl acetate-hexane) to get 2-amino-N-((6-chloropyridin-3-yl)methyl)thiophene-3-carboxamide (200 mg, 46%) as off white solid.

Preparation Example 49

3-((6-Chloropyridin-3-yl)methyl)thieno[2,3-d]pyrimidine-2,4(1H,3H)-dione

To a solution of 2-amino-N-((6-chloropyridin-3-yl)methyl)thiophene-3-carboxamide (200 mg, 0.75 mmol) in tetrahydrofuran (10 mL) was added 1,1'-carbonyldiimidazole (263 mg, 2.25 mmol) in portion wise manner, and the reaction mixture was stirred at 80°C for 16 h. The reaction solvent was concentrated under reduced pressure, and the residue was triturated with diethyl ether. The solid was collected by filtration to get 3-((6-chloropyridin-3-yl)methyl)thieno[2,3-d]pyrimidine-2,4(1H,3H)-dione (160mg, 73%).

Preparation Example 50

3-((6-Chloropyridin-3-yl)methyl)-N-(1-methylcyclopropyl)-2,4-dioxo-1,2,3,4-tetrahydrothieno[2,3-d]pyrimidine-6-sulfonamide

Chlorosulfonic acid (0.17 mL, 2.43 mmol) was cooled to 0°C, and then 3-((6-chloropyridin-3-yl)methyl)thieno[2,3-d]pyrimidine-2,4(1H,3H)-dione (102 mg, 0.35 mmol) was added thereto in portion wise manner, and the reaction mixture was heated to 70°C and stirred for 4 h. After the reaction solution was cooled to room temperature, to the reaction solution was slowly added ice-cold water (100 mL), and the resulting solid was washed with cold water and dried under reduced pressure. The obtained solid was dissolved in tetrahydrofuran and dichloromethane (1:1, 10 mL) and cooled to 0°C, and then diisopropylamine (0.3 mL, 1.75 mmol) and 1-methylcyclopropan-1-amine hydrochloride (56 mg, 0.52mmol) were added thereto, and the reaction mixture was stirred at room temperature for 16 h. The reaction solvent was evaporated under reduced pressure, and the residue was diluted with water and extracted with dichloromethane. The organic layer was dried over anhydrous sodium sulfate and concentrated under reduced pressure. The residue was triturated with diethyl ether, and solid was collected by filtration to get 3-((6-chloropyridin-3-yl)methyl)-N-(1-methylcyclopropyl)-2,4-dioxo-1,2,3,4-tetrahydrothieno[2,3-d]pyrimidine-6-sulfonamide (70 mg, 47%).

Preparation Example 51

tert-Butyl ((3-((6-chloropyridin-3-yl)methyl)-2,4-dioxo-1,2,3,4-tetrahydrothieno[2,3-d]pyrimidin-6-yl)sulfonyl)(1-methylcyclopropyl)carbamate

A solution of 3-((6-chloropyridin-3-yl)methyl)-N-(1-methylcyclopropyl)-2,4-dioxo-1,2,3,4-tetrahydrothieno[2,3-d]pyrimidine-6-sulfonamide (600 mg, 1.41 mmol) in pyridine (5.0 mL) was cooled with ice, and then 4-dimethylaminopyridine (17 mg, 0.14 mmol) and di-tert-butyl dicarbonate (1.5 mL, 1.41 mmol) were added thereto, and the reaction mixture was heated to 90°C and stirred for 5 h. The reaction solvent was concentrated under reduced pressure, and the residue was diluted with water and extracted with dichloromethane. The organic layer was washed with saturated saline, dried over anhydrous sodium sulfate, and concentrated under reduced pressure. The residue was triturated with diethyl ether, and the solid was collected by filtration to get tert-butyl ((3-((6-chloropyridin-3-yl)methyl)-2,4-dioxo-1,2,3,4-tetrahydrothieno[2,3-d]pyrimidin-6-yl)sulfonyl)(1-methylcyclopropyl)carbamate (450 mg, 60%).

Preparation Example 52

tert-Butyl ((3-((6-chloropyridin-3-yl)methyl)-1-((2,4-dimethylthiazol-5-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl ((3-((6-chloropyridin-3-yl)methyl)-2,4-dioxo-1,2,3,4-tetrahydrothieno[2,3-d]pyrimidin-6-yl)sulfonyl)(1-methylcyclopropyl)carbamate (200 mg, 0.38 mmol) in N,N-dimethylformamide (5 mL) was added a solution of potassium carbonate (63 mg, 0.46 mmol) and 5-(chloromethyl)-2,4-dimethylthiazole (92 mg, 0.57 mmol) in N,N-dimethylformamide (1 mL), and the reaction mixture was heated to 90°C under microwave irradiation and stirred for 1 h. To the reaction mixture was added ice-cold water, and the mixture was extracted with dichloromethane. The organic layer was dried over anhydrous sodium sulfate and concentrated under reduced pressure. The residue was purified by silica gel column chromatography (2% to 3% methanol-dichloromethane) to get tert-butyl ((3-((6-chloropyridin-3-yl)methyl)-1-((2,4-dimethylthiazol-5-yl)methyl)-2,4-dioxo-1,2,3,4-tetrahydrothieno[2,3-d]pyrimidin-6-yl)sulfonyl)(1-methylcyclopropyl)carbamate (120 mg, 48%).

Example 105

3-((6-Chloropyridin-3-yl)methyl)-1-((2,4-dimethylthiazol-5-yl)methyl)-N-(1-methylcyclopropyl)-2,4-dioxo-1,2,3,4-tetrahydrothieno[2,3-d]pyrimidine-6-sulfonamide

A solution of tert-butyl ((3-((6-chloropyridin-3-yl)methyl)-1-((2,4-dimethylthiazol-5-yl)methyl)-2,4-dioxo-1,2,3,4-tetrahydrothieno[2,3-d]pyrimidin-6-yl)sulfonyl)(1-methylcyclopropyl)carbamate (120 mg, 0.21 mmol) in dichloromethane (5 mL) was cooled with ice, and then trifluoroacetic acid (1.0 mL) was added thereto, and the reaction mixture was stirred at room temperature for 1 h. The reaction solvent was concentrated under reduced pressure, and the residue was dissolved in dichloromethane and washed with saturated sodium bicarbonate aqueous solution. The organic layer was dried over anhydrous sodium sulfate and concentrated under reduced pressure. The residue was purified over reverse phase prep-HPLC to get 3-((6-chloropyridin-3-yl)methyl)-1-((2,4-dimethylthiazol-5-yl)methyl)-N-(1-methylcyclopropyl)-2,4-dioxo-1,2,3,4-tetrahydrothieno[2,3-d]pyrimidine-6-sulfonamide (20 mg, 15%).

Preparation Example 53

2-Amino-N-((4,5,6,7-tetrahydrobenzo[d]thiazol-2-yl)methyl)thiophene-3-carboxamide

A solution of methyl 2-aminothiophene-3-carboxylate (1.4 g, 8.91 mmol) and (4,5,6,7-tetrahydrobenzo[d]thiazol-2-yl)methanamine (1.5 g, 8.91 mmol) in 1,4-dioxane (50 mL) was cooled with ice, and then a 2M solution of trimethylaluminum in toluene (22.25 mL, 44.55 mmol) was added dropwise thereto, and the reaction mixture was heated to 100°C and stirred for 16 h. To the reaction mixture was added saturated ammonium chloride aqueous solution, and the mixture was extracted with ethyl acetate. The organic layer was washed with saturated saline, dried over anhydrous sodium sulfate, and concentrated under reduced pressure. The residue was purified by silica gel chromatography (70% ethyl acetate-hexane) to get 2-amino-N-((4,5,6,7-tetrahydrobenzo[d]thiazol-2-yl)methyl)thiophene-3-carboxamide (1.4 g, 53%) as off white solid.

Preparation Example 54

3-((4,5,6,7-tetrahydrobenzo[d]thiazol-2-yl)methyl)thieno[2,3-d]pyrimidine-2,4(1H,3H)-dione

To a solution of 2-amino-N-((4,5,6,7-tetrahydrobenzo[d]thiazol-2-yl)methyl)thiophene-3-carboxamide (1.0 g, 3.41 mmol) in tetrahydrofuran (25 mL) was added 1,1'-carbonyldiimidazole (1.66 g, 10.2 mmol) in portion wise manner, and the reaction mixture was stirred at 80°C for 16 h. The reaction solvent was concentrated under reduced pressure, the residue was triturated with diethyl ether, and the solid was collected by filtration to get 3-((4,5,6,7-tetrahydrobenzo[d]thiazol-2-yl)methyl)thieno[2,3-d]pyrimidine-2,4(1H,3H)-dione (1.0 g, 92%).

Preparation Example 55

N-(1-Methylcyclopropyl)-2,4-dioxo-3-((4,5,6,7-tetrahydrobenzo[d]thiazol-2-yl)methyl)-1,2,3,4-tetrahydrothieno[2,3-d]pyrimidine-6-sulfonamide

Chlorosulfonic acid (1.05 mL, 15.3 mmol) was cooled to 0°C, and then 3-((4,5,6,7-tetrahydrobenzo[d]thiazol-2-yl)methyl)thieno[2,3-d]pyrimidine-2,4(1H,3H)-dione (700 mg, 2.19 mmol) was added thereto in portion wise manner, and the reaction mixture was heated to 70°C and stirred for 4 h. The reaction mixture was cooled to room temperature, to the reaction solution was slowly added ice-cold water (100 mL), and the resulting solid was washed with cold water and dried under reduced pressure. The obtained solid was dissolved in tetrahydrofuran and dichloromethane (1:1, 10 mL) and cooled to 0°C, and then diisopropylethylamine (2.75 mL, 15.3 mmol) and 1-methylcyclopropan-1-amine hydrochloride (472 mg, 4.38 mmol) were added thereto, and the reaction mixture was stirred at room temperature for 16 h. The reaction solvent was concentrated under reduced pressure, and the residue was diluted with water and extracted with dichloromethane. The organic layer was dried over anhydrous sodium sulfate and concentrated under reduced pressure. The residue was triturated with diethyl ether, and the solid was collected by filtration to get N-(1-methylcyclopropyl)-2,4-dioxo-3-((4,5,6,7-tetrahydrobenzo[d]thiazol-2-yl)methyl)-1,2,3,4-tetrahydrothieno[2,3-d]pyrimidine-6-sulfonamide (450 mg, 45%).

Preparation Example 56

tert-Butyl ((2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(1-methylcyclopropyl)carbamate

A solution of N-(1-methylcyclopropyl)-2,4-dioxo-3-((4,5,6,7-tetrahydrobenzo[d]thiazol-2-yl)methyl)-1,2,3,4-tetrahydrothieno[2,3-d]pyrimidine-6-sulfonamide (150 mg, 0.33 mmol) in pyridine (2.0 mL) was cooled with ice, and then 4-dimethylaminopyridine (4 mg, 0.03 mmol) and di-tert-butyl dicarbonate (0.22 mL, 0.99 mmol) were added thereto, and the reaction mixture was heated to 90°C and stirred for 5 h. The reaction solvent was concentrated under reduced pressure, and the residue was diluted with water and extracted with dichloromethane. The organic layer was washed with saturated saline, dried over anhydrous sodium sulfate, and concentrated under reduced pressure. The residue was triturated with diethyl ether, and the solid was collected by filtration to get tert-butyl ((2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(l-methylcyclopropyl)carbamate (70 mg, 38%).

Preparation Example 57

tert-Butyl ((1-((1,3-dihydroisobenzofuran-5-yl)methyl)-2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(1-methylcyclopropyl)carbamate

To a solution of tert-butyl ((2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(1-methylcyclopropyl)carbamate (70 mg, 0.13 mmol) in N,N-dimethylformamide (5 mL) were added potassium carbonate (35 mg, 0.26 mmol), sodium iodide (10 mg, 0.06 mmol), and 5-(chloromethyl)-1,3-dihydroisobenzofuran (32 mg, 0.19 mmol), and the reaction mixture was stirred at room temperature for 3 h. To the reaction mixture was added ice-cold water, and the mixture was extracted with dichloromethane. The organic layer was dried over anhydrous sodium sulfate and concentrated under reduced pressure. The residue was purified by silica gel column chromatography (2% to 3% methanol-dichloromethane) to get tert-butyl ((1-((1,3-dihydroisobenzofuran-5-yl)methyl)-2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(1-methylcyclopropyl)carbamate (40 mg, 46%) as off white solid.

Example 106

1-((1,3-Dihydroisobenzofuran-5-yl)methyl)-N-(1-methylcyclopropyl)-2,4-dioxo-3-((4,5,6,7-tetrahydrobenzo[d]thiazol-2-yl)methyl)-1,2,3,4-tetrahydrothieno[2,3-d]pyrimidine-6-sulfonamide

A solution of tert-butyl ((1-((1,3-dihydroisobenzofuran-5-yl)methyl)-2,4-dioxo-3-((4,5,6,7-tetrahydrobenzo[d]thiazol-2-yl)methyl)-1,2,3,4-tetrahydrothieno[2,3-d]pyrimidin-6-yl)sulfonyl)(1-methylcyclopropyl)carbamate (300 mg, 0.46 mmol) in dichloromethane was cooled with ice, and then trifluoroacetic acid (1.0 mL) was added thereto, and the reaction mixture was stirred at room temperature for 1 h. The reaction solvent was evaporated under reduced pressure, and the residue was re-dissolved in dichloromethane and washed with saturated sodium bicarbonate aqueous solution. The organic layer was dried over anhydrous sodium sulfate, and concentrated under reduced pressure. The residue was purified over reverse phase prep-HPLC to get 1-((1,3-dihydroisobenzofuran-5-yl)methyl)-N-(1-methylcyclopropyl)-2,4-dioxo-3-((4,5,6,7-tetrahydrobenzo[d]thiazol-2-yl)methyl)-1,2,3,4-tetrahydrothieno[2,3-d]pyrimidine-6-sulfonamide (57 mg, 22%).

Preparation Example 58

Ethyl 3-isopropyl-1-methyl-1H-pyrazole-5-carboxylate

To a solution of ethyl 3-isopropyl-1H-pyrazole-5-carboxylate (1.0 g, 5.49 mmol) in N,N-dimethylformamide (10 mL) were added a solution of potassium carbonate (1.52 g, 11.0 mmol), potassium iodide (10 mg, 0.06 mmol), and methyl iodide (0.45 mL, 7.13 mmol) in N,N-dimethylformamide (1 mL), and the reaction mixture was stirred at room temperature for 16 h. To the reaction mixture was added ice-cold water, and the mixture was extracted with dichloromethane. The organic layer was dried over anhydrous sodium sulfate and concentrated under reduced pressure. The residue was purified by silica gel column chromatography (10% ethyl acetate-hexane) to get ethyl 3-isopropyl-1-methyl-1H-pyrazole-5-carboxylate (630 mg, 57%) as off white solid.

Preparation Example 59

(3-Isopropyl-1-methyl-1H-pyrazol-5-yl)methanol

A solution of ethyl 3-isopropyl-1-methyl-1H-pyrazole-5-carboxylate (630 mg, 3.21 mmol) in tetrahydrofuran (10 mL) was cooled with ice, and then a 2M solution of lithium aluminum hydride in tetrahydrofuran (4.8 mL, 9.63 mmol) was slowly added thereto, and the reaction mixture was stirred at room temperature for 2 h. The reaction mixture was cooled to 0°C, and to the reaction mixture were added 15% sodium hydroxide aqueous solution (~5 mL) and ice-cold water (50 mL). The mixture was extracted with dichloromethane. The organic layer was dried over anhydrous sodium sulfate and concentrated under reduced pressure. The residue was purified by silica gel column chromatography (70% ethyl acetate-hexane) to get (3-isopropyl-1-methyl-1H-pyrazol-5-yl)methanol (400 mg, 80%) as off white solid.

Preparation Example 60

tert-Butyl ((1 -((3-isopropyl-1 -methyl-1H-pyrazol-5-yl)methyl)-3-((2-methylthiazol-5-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl (1-methylcyclopropyl)((3-((2-methylthiazol-5-yl)methyl)-2,4-dioxo-1,2,3,4-tetrahydrothieno[2,3-d]pyrimidin-6-yl)sulfonyl)carbamate (200 mg, 0.39 mmol) in tetrahydrofuran (10 mL) was cooled with ice, and then (3-isopropyl-1-methyl-1H-pyrazol-5-yl)methanol (72 mg, 0.46 mmol), diethyl azodicarboxylate (0.11 mL, 0.69 mmol), and triphenylphosphine (180 mg, 0.69 mmol) were added thereto, and the reaction mixture was stirred at room temperature for 16 h. To the reaction mixture was added ice-cold water, and the mixture was extracted with dichloromethane. The organic layer was dried over anhydrous sodium sulfate and concentrated under reduced pressure. The residue was purified by silica gel column chromatography (2% methanol-dichloromethane) to get tert-butyl ((1-((3-isopropyl-1-methyl-1H-pyrazol-5-yl)methyl)-3-((2-methylthiazol-5-yl)methyl)-2,4-dioxo-1,2,3,4-tetrahydrothieno[2,3-d]pyrimidin-6-yl)sulfonyl)(1-methylcyclopropyl)carbamate (150 mg, 59%) as off white solid.

Example 107

1-((3-Isopropyl-1-methyl-1H-pyrazol-5-yl)methyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide

A solution of tert-butyl ((1-((3-isopropyl-1-methyl-1H-pyrazol-5-yl)methyl)-3-((2-methylthiazol-5-yl)methyl)-2,4-dioxo-1,2,3,4-tetrahydrothieno [2,3-d]pyrimidin-6-yl)sulfonyl)(1-methylcyclopropyl)carbamate (150 mg, 0.23 mmol) in dichloromethane (5 mL) was cooled with ice, and then trifluoroacetic acid (1.0 mL) was added thereto, and the reaction mixture was stirred at room temperature for 1 h. The reaction solvent was evaporated under reduced pressure, and the residue was re-dissolved in dichloromethane and washed with saturated sodium bicarbonate aqueous solution. The organic layer was dried over anhydrous sodium sulfate, and concentrated under reduced pressure. The residue was purified over reverse phase prep-HPLC to get 1-((3-isopropyl-1-methyl-1H-pyrazol-5-yl)methyl)-N-(1-methylcyclopropyl)-3-((2-methylthiazol-5-yl)methyl)-2,4-dioxo-1,2,3,4-tetrahydrothieno[2,3-d]pyrimidine-6-sulfonamide (40 mg, 31%).

Preparation Example 61

Iodo(iodomethoxy)methane

A mixture of 1,3,5-trioxane (30 g, 333.0 mmol) and trimethylsilyl iodide was heated at 50°C for 16 h. The reaction mixture was purified by distillation at 120°C to 150°C under reduced pressure to get iodo(iodomethoxy)methane (40.0 g, 40%).

Preparation Example 62

(Oxobis(methylene))bis(tributyltin)

A solution of tributyltin hydride (73.7 g, 252.5 mmol) in tetrahydrofuran (150 mL) was cooled to -78°C, and then a 2M solution of lithium diisopropylamide in tetrahydrofuran (15.0 mL, 15.06 mmol) was added thereto, and the reaction mixture was stirred at -30°C for 30 min. The reaction mixture was cooled again to -78°C, and to the reaction mixture was added dropwise iodo(iodomethoxy)methane (30.0 g, 101.1 mmol). The reaction solution was stirred at the same temperature for 2 h. To the reaction mixture was slowly added saturated ammonium chloride aqueous solution, and the

mixture was extracted with dichloromethane. The organic layer was dried over anhydrous sodium sulfate and concentrated under reduced pressure. The residue was purified by silica gel column chromatography (n-heptane) to get (oxobis(methylene))bis(tributyltin) (3.4 g, 5.5%) as colorless oil.

Preparation Example 63

Methyl 5,7-dihydrofuro[3,4-b]pyridine-3-carboxylate

To a solution of methyl 5,6-dichloronicotinate (1.5 g, 7.28 mmol) and (oxobis(methylene))bis(tributyltin) (4.54 g, 7.28 mmol) in 1,4-dioxane (15 mL) were added XPhos (0.87 g, 1.82 mmol) and tris(dibenzylideneacetone)dipalladium(0) (0.67 g, 0.73 mmol), and the reaction mixture was stirred at 100°C for 16 h. The reaction mixture was diluted with water (100 mL) and extracted with ethyl acetate. The organic layer was washed with saturated saline, dried over anhydrous sodium sulfate, and concentrated under reduced pressure. The residue was purified by silica gel chromatography (30% ethyl acetate-hexane) to get methyl 5,7-dihydrofuro[3,4-b]pyridine-3-carboxylate (0.9 g, 69%) as off white solid.

Preparation Example 64

(5,7-Dihydrofuro[3,4-b]pyridin-3-yl)methanol

A solution of methyl 5,7-dihydrofuro[3,4-b]pyridine-3-carboxylate (0.9 g, 5.02 mmol) in tetrahydrofuran (40 mL) was cooled to -30°C, and then a 1.0 M solution of diisobutylaluminum hydride in tetrahydrofuran (15.0 mL, 15.06 mmol) was added thereto, and the reaction mixture was stirred at room temperature for 1.5 h. The reaction mixture was cooled to 0°C, and then a saturated ammonium chloride aqueous solution was added thereto, and the mixture was extracted with ethyl acetate. The organic layer was washed with saturated saline, dried over anhydrous sodium sulfate, and concentrated under reduced pressure. The residue was purified by silica gel chromatography (5% methanol-dichloromethane) to get (5,7-dihydrofuro[3,4-b]pyridin-3-yl)methanol (0.23 g, 30%).

Preparation Example 65

tert-Butyl ((1 -((5,7-dihydrofuro[3,4-b]pyridin-3-yl)methyl)-3-((1 -methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

A solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (150 mg, 0.30 mmol) in dichloromethane (10 mL) was cooled with ice, and then (5,7-dihydrofuro[3,4-b]pyridin-3-yl)methanol (91 mg, 0.60 mmol), diisopropyl azodicarboxylate (154 μL, 0.75 mmol), and triphenylphosphine (196 mg, 0.75 mmol) were added thereto, and the reaction mixture was stirred at room temperature for 2 h. The reaction mixture was diluted with water and extracted with ethyl acetate. The organic layer was washed with saturated saline, dried over anhydrous sodium sulfate, and concentrated under reduced pressure. The residue was purified by silica gel chromatography (3% methanol-dichloromethane) to get tert-butyl ((1-((5,7-dihydrofuro[3,4-b]pyridin-3-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (130 mg, 68%).

Example 108

1-((5,7-Dihydrofuro[3,4-b]pyridin-3-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide

A solution of tert-butyl ((1-((5,7-dihydrofuro[3,4-b]pyridin-3-yl)methyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (130 mg, 0.21 mmol) in dichloromethane (5 mL) was cooled with ice, and then trifluoroacetic acid (1.0 mL) was added thereto, and the reaction mixture was stirred at room temperature for 1 h. The reaction solvent was concentrated under reduced pressure, and the residue was re-dissolved in dichloromethane (50 mL) and washed with saturated sodium bicarbonate aqueous solution. The organic layer was dried over anhydrous sodium sulfate and concentrated under reduced pressure. The residue was purified over reverse phase prep-HPLC to get 1-((5,7-dihydrofuro[3,4-b]pyridin-3-yl)methyl)-3-((1-methyl-1H-pyrazol-4-yl)methyl)-N-(1 -methylcyclopropyl)-2,4-dioxo-1,2,3,4-tetrahydrothieno[2,3-d]pyrimidine-6-sulfonamide (40 mg, 36%) as off white solid.

Preparation Example 66

Methyl 5,6-dichloropicolinate

A solution of 5,6-dichloropicolinic acid (2.0 g, 10.4 mmol) in dichloromethane (20 mL) was cooled with ice, and then N,N-dimethylformamide (80 μL, 1.04 mmol) and oxalyl chloride (2.68 mL, 31.3 mmol) were added dropwise thereto, and the reaction mixture was stirred at room temperature for 30 min. The reaction mixture was concentrated under reduced pressure, the residue was dissolved in tetrahydrofuran (10.0 mL), cooled to 0°C, and the mixture was triturated with methanol (25 mL) at room temperature for 30 minutes. The reaction mixture was diluted with water and extracted with ethyl acetate. The organic layer was washed with saturated saline, dried over anhydrous sodium sulfate, and concentrated under reduced pressure to get methyl 5,6-dichloropicolinate (2.1 g, 97%).

Preparation Example 67

Methyl 5,7-dihydrofuro[3,4-b]pyridine-2-carboxylate

To a solution of methyl 5,6-dichloropicolinate (1.5 g, 7.28 mmol) and (oxobis(methylene))bis(tributyltin) (4.54 g, 7.28 mmol) in 1,4-dioxane (15 mL) were added XPhos (0.87 g, 1.82 mmol) and tris(dibenzylideneacetone)dipalladium(0) (0.67 g, 0.73 mmol), and the reaction mixture was stirred at 100°C for 16 h. The reaction mixture was diluted with water and extracted with ethyl acetate. The organic layer was washed with saturated saline, dried over anhydrous sodium sulfate, and concentrated under reduced pressure. The residue was purified by silica gel chromatography (30% ethyl acetate-hexane) to get methyl 5,7-dihydrofuro[3,4-b]pyridine-2-carboxylate (0.95 g, 72%) as off white solid.

Preparation Example 68

(5,7-Dihydrofuro[3,4-b]pyridin-2-yl)methanol

A solution of methyl 5,7-dihydrofuro[3,4-b]pyridine-2-carboxylate (0.85 g, 4.74 mmol) in tetrahydrofuran (40 mL) was cooled to -30°C, and then a 1.0 M solution of diisobutylaluminum hydride in tetrahydrofuran (14.0 mL, 14.2 mmol) was added thereto, and the reaction mixture was stirred at room temperature for 1.5 h. The reaction mixture was cooled to 0°C, and then a saturated ammonium chloride aqueous solution was added thereto, and the mixture was extracted with ethyl acetate. The organic layer was washed with saturated saline, dried over anhydrous sodium sulfate, and concentrated under reduced pressure. The residue was purified by silica gel column chromatography (4% methanol-dichloromethane) to get (5,7-dihydrofuro[3,4-b]pyridin-2-yl)methanol (0.4 g, 55%).

Preparation Example 69

Methyl 4,5-dichloro-2-fluorobenzoate

A solution of 4,5-dichloro-2-fluorobenzoic acid (2.0 g, 9.57 mmol) in dichloromethane (20 mL) was cooled with ice, and then a catalytic amount of N,N-dimethylformamide and oxalyl chloride (2.46 mL, 28.7 mmol) were added dropwise thereto, and the reaction solution was stirred at room temperature for 30 min. The reaction mixture was concentrated under reduced pressure, and the residue was dissolved in tetrahydrofuran (10.0 mL). After the mixture was cooled to 0°C, the mixture was triturated with methanol (25 mL) at room temperature for 30 minutes, and the reaction mixture was diluted with water and extracted with ethyl acetate. The organic layer was washed with saturated saline, dried over anhydrous sodium sulfate, and concentrated under reduced pressure to get methyl 4,5-dichloro-2-fluorobenzoate (2.0 g, 94%) as off white solid.

Preparation Example 70

Methyl 6-fluoro-1,3-dihydroisobenzofuran-5-carboxylate

To a solution of methyl 4,5-dichloro-2-fluorobenzoate (500 mg, 2.24 mmol) and (oxobis(methylene))bis(tributyltin) (1.4 g, 2.24 mmol) in 1,4-dioxane (10 mL) were added XPhos (640 mg, 1.35 mmol) and tris(dibenzylideneacetone)dipalladium(0) (205 mg, 0.24 mmol), and the reaction mixture was stirred at 100°C for 16 h. The reaction solution was diluted with water and extracted with ethyl acetate. The organic layer was washed with saturated saline, dried over anhydrous sodium sulfate, and concentrated under reduced pressure. The residue was purified by silica gel column chromatography (12% ethyl acetate-hexane) to get methyl 6-fluoro-1,3-dihydroisobenzofuran-5-carboxylate (210 mg,

47%) as off white solid.

Preparation Example 71

(6-Fluoro-1,3-dihydroisobenzofuran-5-yl)methanol

A solution of methyl 6-fluoro-1,3-dihydroisobenzofuran-5-carboxylate (280 mg, 1.43 mmol) in tetrahydrofuran (10 mL) was cooled to -30°C, and then a 1.0 M solution of diisobutylaluminum hydride in tetrahydrofuran (4.3 mL, 4.3 mmol) was added thereto, and the reaction mixture was stirred at room temperature for 1.5 h. The reaction solution was cooled to 0°C, and then a saturated ammonium chloride aqueous solution was added thereto, and the mixture was extracted with dichloromethane. The organic layer was washed with saturated saline, dried over anhydrous sodium sulfate, and concentrated under reduced pressure. The residue was purified by silica gel column chromatography (4% methanol-dichloromethane) to get (6-fluoro-1,3-dihydroisobenzofuran-5-yl)methanol (100 mg, 41%).

Preparation Example 72

Methyl 4,5-dichloro-2-methoxybenzoate

A solution of 4,5-dichloro-2-methoxybenzoic acid (5.0 g, 22.6 mmol) in N,N-dimethylformamide (50 mL) was cooled with ice, and then potassium carbonate (9.38 g, 67.9 mmol) and methyl iodide (4.82 g, 33.9 mmol) were added thereto, and the reaction mixture was stirred at room temperature for 30 min. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (30% ethyl acetate-hexane) to get methyl 4,5-dichloro-2-methoxybenzoate (5.0 g, 93%) as off white solid.

Preparation Example 73

Methyl 6-methoxy-1,3-dihydroi sobenzofuran-5-carboxylate

To a solution of methyl 4,5-dichloro-2-methoxybenzoate (2.0 g, 8.51 mmol) and (oxobis(methylene))bis(tributyltin) (5.31 g, 8.51 mmol) in 1,4-dioxane (25 mL) were added XPhos (1.01 g, 2.13 mmol) and tris(dibenzylideneacetone)dipalladium(0) (850 mg, 0.85 mmol), and the reaction mixture was stirred at 100°C for 16 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (5% methanol-dichloromethane) to get methyl 6-methoxy-1,3-dihydroisobenzofuran-5-carboxylate (1.0 g, 56%) as off white solid.

Preparation Example 74

(6-Methoxy-1,3-dihydroisobenzofuran-5-yl)methanol

A solution of methyl 6-methoxy-1,3-dihydroisobenzofuran-5-carboxylate (500 mg, 2.40 mmol) in tetrahydrofuran (10 mL) was cooled to -30°C, and then a 1.0 M solution of diisobutylaluminum hydride in tetrahydrofuran (12 mL, 12.0 mmol) was added thereto, and the reaction mixture was stirred at room temperature for 1 h. The reaction mixture was cooled to 0°C, and then a saturated ammonium chloride aqueous solution was added thereto, and the mixture was extracted with dichloromethane. The organic layer was washed with saturated saline, dried over anhydrous sodium sulfate, and concentrated under reduced pressure. The residue was purified by silica gel column chromatography (10% methanol-dichloromethane) to get (6-methoxy-1,3-dihydroisobenzofuran-5-yl)methanol (180 mg, 34%).

Preparation Example 75

1-(1,3-Dihydroisobenzofuran-5-yl)ethan-1-ol

To a mixture of dipropargyl ether (1.0 g, 10.6 mmol) and 3-butyn-2-ol (3.72 g, 53.1 mmol) was added ruthenium chloride hydrate (44 mg, 0.2 mmol), and the reaction mixture was heated at 110°C for 8 h. The reaction mixture was concentrated under reduced pressure, and the residue was purified by silica gel column chromatography (30% ethyl acetate-hexane) to get 1-(1,3-dihydroisobenzofuran-5-yl)ethan-1-ol (400 mg, 23%).

Preparation Example 76

(6-Methyl-1,3-dihydroisobenzofuran-5-yl)methanol

To a mixture of dipropargyl ether (500 mg, 5.31 mmol) and 2-butyn-1-ol (1.86 g, 26.6 mmol) was added ruthenium chloride hydrate (11 mg, 0.05 mmol), and the reaction mixture was heated at 110°C for 8 h. The reaction mixture was concentrated under reduced pressure, and the residue was purified by silica gel column chromatography (25% ethyl acetate-hexane) to get (6-methyl-1,3-dihydroisobenzofuran-5-yl)methanol (120 mg, 14%).

Preparation Example 77

(4-Methyl-1,3-dihydroisobenzofuran-5-yl)methanol and (7-methyl-1,3-dihydroisobenzofuran-5-yl)methanol

To a mixture of 1-(2-propyn-1-yloxy)-2-butyne (1.0 g, 9.25 mmol) and 2-butyn-1-ol (2.59 g, 46.2 mmol) was added Wilkinson's catalyst (172 mg, 0.18 mmol), and the reaction mixture was heated at 110°C for 8 h. The reaction mixture was concentrated under reduced pressure, and the residue was purified by silica gel column chromatography (40% ethyl acetate-hexane) to get a mixture of (4-methyl-1,3-dihydroisobenzofuran-5-yl)methanol and (7-methyl-1,3-dihydroisobenzofuran-5-yl)methanol (350 mg, 23%).

Preparation Example 78

Methyl 3-iodo-4-(2,2,2-trifluoroacetamido)benzoate

A solution of methyl 4-amino-3-iodobenzoate (5.0 g, 18.0 mmol) in tetrahydrofuran (20 mL) was cooled with ice, and then thereto was added trifluoroacetic anhydride (5.02 mL, 36.0 mmol), and the reaction mixture was stirred for 1 h. The reaction solution was diluted with water and extracted with ethyl acetate. The organic layer was dried over anhydrous sodium sulfate and concentrated under reduced pressure to get methyl 3-iodo-4-(2,2,2-trifluoroacetamido)benzoate (6.0 g, 89%).

Preparation Example 79

Methyl 2-(((tetrahydro-2H-pyran-2-yl)oxy)methyl)-1H-indole-5-carboxylate

To a solution of methyl 3-iodo-4-(2,2,2-trifluoroacetamido)benzoate (2.5 g, 6.7 mmol) in N,N-dimethylformamide (20 mL) were added 2-(2-propyn-1-yloxy)tetrahydro-2H-pyran (939 mg, 6.7 mmol), triethylamine (4.84 mL, 33.5 mmol), copper iodide (180 mg, 0.67 mmol), and dichlorobis(triphenylphosphine)palladium (235 mg, 0.34 mmol), and the reaction mixture was heated at 100°C for 5 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (20% ethyl acetate-hexane) to get methyl 2-(((tetrahydro-2H-pyran-2-yl)oxy)methyl)-1H-indole-5-carboxylate (1.2 g, 60%).

Preparation Example 80

1-tert-butyl 5-methyl 2-(((tetrahydro-2H-pyran-2-yl)oxy)methyl)-1H-indole-1,5-dicarboxylate

A solution of methyl 2-(((tetrahydro-2H-pyran-2-yl)oxy)methyl)-1H-indole-5-carboxylate (1.2 g, 4.15 mmol) in dichloromethane (20 mL) was cooled with ice, and then triethylamine (1.2 mL, 8.3 mmol), 4-dimethylaminopyridine (51 mg, 0.42 mmol) and di-tert-butyl dicarbonate (1.21 mL, 4.98 mmol) were added thereto, and the reaction mixture was stirred at room temperature for 6 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (10% ethyl acetate-hexane) to get 1-tert-butyl 5-methyl 2-(((tetrahydro-2H-pyran-2-yl)oxy)methyl)-1H-indole-1,5-dicarboxylate (1.5 g, 92%) as brown solid.

Preparation Example 81

tert-Butyl 5-(hydroxymethyl)-2-(((tetrahydro-2H-pyran-2-yl)oxy)methyl)-1H-indole-1-carboxylate

A solution of 1-tert-butyl 5-methyl 2-(((tetrahydro-2H-pyran-2-yl)oxy)methyl)-1H-indole-1,5-dicarboxylate (1.5

g, 3.85 mmol) in tetrahydrofuran (30 mL) was cooled to -78°C, and then a 1.0 M solution of diisobutylaluminum hydride in tetrahydrofuran (16.7 mL, 16.7 mmol) was added thereto, and the reaction mixture was stirred for 2 h. To the reaction mixture was added a saturated ammonium chloride aqueous solution, and the mixture was extracted with ethyl acetate. The organic layer was washed with saturated saline, dried over anhydrous sodium sulfate, and concentrated under reduced pressure. The residue was purified by silica gel column chromatography (10% ethyl acetate-hexane) to get tert-butyl 5-(hydroxymethyl)-2-(((tetrahydro-2H-pyran-2-yl)oxy)methyl)-1H-indole-1-carboxylate (1.3 g, 93%) as brown solid.

Preparation Example 82

7-Bromo-2-methyl-3,4-dihydroisoquinolin-1(2H)-one

7-Bromo-3,4-dihydroisoquinolin-1(2H)-one (1.0 g, 4.42 mmol) in N,N-dimethylformamide (10 mL) was cooled with ice, and then sodium hydride (60% oily, 195 mg, 4.87 mmol) and methyl iodide (303 μL, 4.87 mmol) were added thereto in portion wise manner, and the reaction mixture was stirred at 0°C for 2 h. The reaction mixture was diluted with ice-cold water and extracted with ethyl acetate. The organic layer was washed with saturated saline, dried over anhydrous sodium sulfate, and concentrated under reduced pressure. To the residue was added pentane, and the solid was collected by filtration to get 7-bromo-2-methyl-3,4-dihydroisoquinolin-1(2H)-one (900 mg, 90%) as white solid.

Preparation Example 83

2-Methyl-7-vinyl-3,4-dihydroisoquinolin-1 (2H)-one

To a solution of 7-bromo-2-methyl-3,4-dihydroisoquinolin-1(2H)-one (900 mg, 3.75 mmol) in N,N-dimethylformamide (10 mL) were added tributyl(vinyl)tin (2.3 g, 7.5 mmol) and tetrakis(triphenylphosphine)palladium(0) (439 mg, 0.38 mmol), and the reaction mixture was stirred at 100°C for 6 h. The reaction mixture was filtered through celite and washed with ethyl acetate. The filtrate was washed with saturated saline, dried over anhydrous sodium sulfate, and concentrated under reduced pressure. The residue was purified by silica gel column chromatography (70% ethyl acetate-heptane) to get 2-methyl-7-vinyl-3,4-dihydroisoquinolin-1(2H)-one (650 mg, 92%).

Preparation Example 84

2-Methyl-1 -oxo-1,2,3,4-tetrahydroisoquinoline-7-carbaldehyde

A solution of 2-methyl-7-vinyl-3,4-dihydroisoquinolin-1(2H)-one (650 mg, 3.47 mmol) in a mixture of 1,4-dioxane and water (1:1, 10 mL) was cooled with ice, and then osmium tetroxide (86.8 μL, 0.35 mol) was added thereto, and the reaction mixture was stirred for 1 h. To the reaction mixture was added a solution of sodium periodate (1.63 g, 7.63 mmol) in water (50 mL), and the reaction mixture was stirred at room temperature for 15 min. The reaction mixture was extracted with ethyl acetate. The organic layer was washed with saturated saline, dried over anhydrous sodium sulfate, and concentrated under reduced pressure. The residue was purified by silica gel column chromatography (70% ethyl acetate-heptane) to get 2-methyl-1-oxo-1,2,3,4-tetrahydroisoquinoline-7-carbaldehyde (300 mg, 46%).

Preparation Example 85

7-(Hydroxymethyl)-2-methyl-3,4-dihydroisoquinolin-1 (2H)-one

A solution of 2-methyl-1-oxo-1,2,3,4-tetrahydroisoquinoline-7-carbaldehyde (300 mg, 1.59 mmol) in a mixture of methanol and tetrahydrofuran (5:1, 12 mL) was cooled with ice, and then sodium borohydride (120 mg, 3.17 mmol) was added thereto, and the reaction mixture was stirred for 1 h. The reaction mixture was diluted with ice-cold water and extracted with ethyl acetate. The organic layer was concentrated under reduced pressure. The residue was purified by silica gel column chromatography (70% ethyl acetate-heptane) to get 7-(hydroxymethyl)-2-methyl-3,4-dihydroisoquinolin-1(2H)-one (150 mg, 49%).

Preparation Example 86

Methyl 3 -fluorobicyclo [1.1.1] pentane-1-carboxylate

To an aqueous solution (5.0 mL) of silver nitrate (150 mg, 0.88 mmol) were added 1-chloromethyl-4-fluoro-1,4-diazoniabicyclo[2.2.2]octane bis(tetrafluoroborate) (2.08 g, 5.88 mmol) and 3-(methoxycarbonyl)bicyclo[1.1.1]pentane-1-carboxylic acid (500mg, 2.94mmol), and the reaction mixture was heated at 60°C for 16 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (30% ethyl acetate-hexane) to get methyl 3-fluorobicyclo[1.1.1]pentane-1-carboxylate (110 mg, 26%).

Preparation Example 87

3-Fluorobicyclo[1.1.1]pentane-1-carboxylic acid

To a solution of methyl 3-fluorobicyclo[1.1.1]pentane-1-carboxylate (500 mg, 3.46 mmol) in tetrahydrofuran and water (3:1, 8 mL) was added lithium hydroxide hydrate (291 mg, 6.93 mmol), and the reaction mixture was stirred at room temperature for 4 h. The reaction mixture was diluted with water and extracted with diethyl ether. The aqueous layer was acidified with 2M hydrochloric acid and extracted with ethyl acetate. The combined organic layer was dried over anhydrous sodium sulfate and concentrated under reduced pressure to get 3-fluorobicyclo[1.1.1]pentane-1 -carboxylic acid (300 mg, 66%).

Preparation Example 88

(3-Fluorobicyclo[1.1.1]pentan-1-yl)methanol

A solution of 3-fluorobicyclo[1.1.1]pentane-1-carboxylic acid (300 mg, 2.30 mmol) in tetrahydrofuran (10 mL) was cooled with ice, and then a 1.0 M solution of borane in tetrahydrofuran (4.6 mL, 4.60 mmol) was added thereto, and the reaction mixture was stirred at room temperature for 5 h. The reaction solution was diluted with water and extracted with ethyl acetate. The organic layer was washed with saturated saline, dried over anhydrous sodium sulfate, and concentrated under reduced pressure to get (3-fluorobicyclo[1.1.1]pentan-1-yl)methanol (150 mg, 56%).

Preparation Example 89

Ethyl 2-(oxetan-3-ylidene)acetate

A solution of oxetan-3-one (2.0 g, 27.8 mmol) in dichloromethane (10.0 mL) was cooled with ice, and then (carboethoxymethylene)triphenylphosphorane (29 g, 83.3 mmol) was added thereto, and the reaction mixture was stirred at room temperature for 2 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (10% ethyl acetate-hexane) to get ethyl 2-(oxetan-3-ylidene)acetate (3.5 g, 88%).

Preparation Example 90

Ethyl 2-(3-methyloxetan-3-yl)acetate

To a solution of ethyl 2-(oxetan-3-ylidene)acetate (3.0 g, 21.1 mmol) in tetrahydrofuran (30 mL) were added copper iodide (410 mg, 2.10 mmol) and chlorotrimethylsilane (3.44 g, 31.7 mmol), and the reaction mixture was stirred at room temperature for 30 min. The reaction mixture was cooled to -15°C, and then a 3.0 M solution of methylmagnesium chloride in tetrahydrofuran (28.13 mL, 84.4 mmol) was added thereto, and the reaction mixture was stirred at room temperature for 1 h. To the reaction mixture was added saturated ammonium chloride aqueous solution, and the mixture was extracted with ethyl acetate. The organic layer was washed with saturated saline, dried over anhydrous sodium sulfate, and concentrated under reduced pressure. The residue was purified by silica gel column chromatography (70% ethyl acetate-hexane) to get ethyl 2-(3-methyloxetan-3-yl)acetate (1.0 g, 30%).

Preparation Example 91

2-(3-Methyloxetan-3-yl)ethan-1-ol

A solution of ethyl 2-(3-methyloxetan-3-yl)acetate (200 mg, 1.26 mmol) in tetrahydrofuran (10 mL) was cooled with ice, and then a 1.0 M solution of lithium aluminum hydride in tetrahydrofuran (1.26 mL, 1.26 mmol) was added thereto, and the reaction mixture was stirred at room temperature for 2 h. To the reaction mixture was added saturated ammonium chloride aqueous solution, and the mixture was extracted with ethyl acetate. The organic layer was washed

with saturated saline, dried over anhydrous sodium sulfate, and concentrated under reduced pressure to get 2-(3-methyloxetan-3-yl)ethan-1-ol (80 mg, 54%).

Preparation Example 92

3-(2-((tert-Butyldimethyl silyl)oxy)ethoxy)-4-fluorobenzaldehyde

A solution of 4-fluoro-3-hydroxybenzaldehyde (300 mg, 2.14 mmol) and tert-butyldimethylsilanol (600 mg, 4.54 mmol) in dichloromethane (10.0 mL) was cooled with ice, and then triphenylphosphine (842 mg, 3.21 mmol) and diisopropyl azodicarboxylate (649 mg, 3.21 mmol) were added thereto, and the reaction mixture was stirred at room temperature for 1 h. The reaction mixture was diluted with water and extracted with dichloromethane. The organic layer was washed with saturated saline, dried over anhydrous sodium sulfate, and concentrated under reduced pressure. The residue was purified by silica gel column chromatography (80% ethyl acetate-hexane) to get 3-(2-((tert-butyldimethylsilyl)oxy)ethoxy)-4-fluorobenzaldehyde (600mg, 95%) as off white solid.

Preparation Example 93

(3-(2-((tert-Butyldimethylsilyl)oxy)ethoxy)-4-fluorophenyl)methanol

A solution of 3-(2-((tert-butyldimethylsilyl)oxy)ethoxy)-4-fluorobenzaldehyde (500 mg, 1.67 mmol) in a mixture of methanol and tetrahydrofuran (3:2, 10.0 mL) was cooled with ice, and then sodium borohydride (127 mg, 3.35 mmol) was added thereto in portion wise manner, and the reaction mixture was stirred at room temperature for 1 h. The reaction mixture was diluted with ice-cold water and extracted with dichloromethane. The organic layer was washed with saturated saline, dried over anhydrous sodium sulfate, and concentrated under reduced pressure. The residue was purified by silica gel column chromatography (80% ethyl acetate-hexane) to get (3-(2-((tertbutyldimethylsilyl)oxy)ethoxy)-4-fluorophenyl)methanol (170mg, 34%) as off white solid.

Preparation Example 94

3,5-Difluoro-4-methoxybenzaldehyde

To a solution of 3,5-difluoro-4-hydroxybenzaldehyde (500 mg, 3.16 mmol) in N,N-dimethylformamide (10.0 mL) were added potassium carbonate (656 mg, 4.74 mmol) and methyl iodide (0.3 mL, 4.74 mmol), and the reaction mixture was stirred at room temperature for 2 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (35% ethyl acetate-hexane) to get 3,5-difluoro-4-methoxybenzaldehyde (300 mg, 55%) as off white solid.

Preparation Example 95

(3,5-Difluoro-4-methoxyphenyl)methanol

A solution of 3,5-difluoro-4-methoxybenzaldehyde (100 mg, 0.58 mmol) in methanol (5.00 mL) was cooled with ice, and then sodium borohydride (42 mg, 1.16 mmol) was slowly added thereto, and the reaction mixture was stirred at room temperature for 2 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (80% ethyl acetate-hexane) to get (3,5-difluoro-4-methoxyphenyl)methanol (80 mg, 79%) as off white solid.

Preparation Example 96

Methyl 2,4-difluoro-5-(methylamino)benzoate

To a solution of methyl 5-amino-2,4-difluorobenzoate (1.0 g, 5.34 mmol) in chloroform (10.0 mL) were added methylboronic acid (480 mg, 8.02 mmol), pyridine (1.08 mL, 13.4 mmol), and copper acetate (1.16 g, 6.41 mmol), and the reaction mixture was stirred at room temperature for 16 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (6% ethyl

acetate-hexane) to get methyl 2,4-difluoro-5-(methylamino)benzoate (150 mg, 14%).

Preparation Example 97

(2,4-Difluoro-5-(methylamino)phenyl)methanol

A solution of methyl 2,4-difluoro-5-(methylamino)benzoate (150 mg, 0.77 mmol) in methanol (5.0 mL) was cooled with ice, and then sodium borohydride (56 mg, 1.49 mmol) was slowly added thereto, and the mixture was stirred at room temperature for 2 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (3% methanol-dichloromethane) to get (2,4-difluoro-5-(methylamino)phenyl)methanol (75 mg, 58%) as off white solid.

Preparation Example 98

Methyl 3,5-difluoro-4-(methylamino)benzoate

A solution of methyl 3,4,5-trifluorobenzoate (1.0 g, 5.26 mmol) in N,N-dimethylformamide (10.0 mL) was cooled with ice, and then cesium carbonate (4.28 g, 13.1 mmol) and methylamine hydrochloride (710 mg, 10.5 mmol) were added thereto, and the mixture was stirred at room temperature for 2 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (40% ethyl acetate-hexane) to get methyl 3,5-difluoro-4-(methylamino)benzoate (650 mg, 61%) as off white solid.

Preparation Example 99

(3,5-Difluoro-4-(methylamino)phenyl)methanol

A solution of methyl 3,5-difluoro-4-(methylamino)benzoate (120 mg, 0.59 mmol) in methanol (5.0 mL) was cooled with ice, and then sodium borohydride (45 mg, 1.19 mmol) was slowly added thereto, and the reaction mixture was stirred at room temperature for 1 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (70% ethyl acetate-hexane) to get (3,5-difluoro-4-(methylamino)phenyl)methanol (80 mg, 77%) as off white solid.

Preparation Example 100

Methyl 2-fluoro-5-formylbenzoate

A solution of 3-bromo-4-fluorobenzaldehyde (5.0 g, 24.6 mmol) in a mixture of methanol (5.0 mL) and N,N-dimethylformamide (20 mL) was cooled with ice, and then triethylamine (12.1 mL, 86.2 mmol), 1,1'-bis(diphenylphosphino)ferrocene (1.36 g, 2.46 mmol), and palladium acetate (276 mg, 1.23 mmol) were added thereto, and the reaction mixture was heated at 80°C under carbon monoxide atmosphere (80 Psi) for 16 h. The reaction mixture was diluted with ice-cold water and extracted with ethyl acetate. The organic layer was washed with saturated saline, dried over anhydrous sodium sulfate, and concentrated under reduced pressure. The residue was triturated with diethyl ether, and solid was collected by filtration to get methyl 2-fluoro-5-formylbenzoate (2.7 g, 60%) as off white solid.

Preparation Example 101

Methyl 2-fluoro-5-(hydroxymethyl)benzoate

A solution of methyl 2-fluoro-5-formylbenzoate (100 mg, 0.55 mmol) in methanol (5.0 mL) was cooled with ice, and then sodium borohydride (42 mg, 1.09 mmol) was added thereto, and the reaction mixture was stirred at 0°C for 1 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (60% ethyl acetate-hexane) to get methyl 2-fluoro-5-(hydroxymethyl)benzoate (60 mg, 77%) as off white solid.

Preparation Example 102

tert-Butyl 4-(cyclopropanecarbonyl)piperazine-1-carboxylate

A solution of tert-butyl piperazine-1-carboxylate (1.5 g, 8.05 mmol) in dichloromethane (10.0 mL) was cooled with ice, and then triethylamine (1.7 mL, 12.1 mmol) and cyclopropanecarbonyl chloride (1.01 g, 9.66 mmol) were added thereto, and the reaction mixture was stirred at room temperature for 1 h. The reaction mixture was diluted with water and extracted with ethyl acetate. The organic layer was washed with saturated saline, dried over anhydrous sodium sulfate, and concentrated under reduced pressure to get tert-butyl 4-(cyclopropanecarbonyl)piperazine-1-carboxylate (1.4 g, 68%).

Preparation Example 103

1-(Cyclopropanecarbonyl)piperazine

To a solution of tert-butyl 4-(cyclopropanecarbonyl)piperazine-1-carboxylate (1.0 g, 3.93 mmol) in dichloromethane (5 mL) was added a 4M solution of hydrogen chloride in dioxane (5.0 mL), and the reaction mixture was stirred at room temperature for 30 min. To the reaction mixture was added a saturated sodium bicarbonate aqueous solution (100 mL), and the mixture was extracted with ethyl acetate. The organic layer was washed with saturated saline, dried over anhydrous sodium sulfate, and concentrated under reduced pressure to get 1-(cyclopropanecarbonyl)piperazine (500 mg, 83%).

Preparation Example 104

3-(4-(Cyclopropanecarbonyl)piperazine-1-carbonyl)-4-fluorobenzaldehyde

A solution of 2-fluoro-5-formylbenzoic acid (1.5 g, 8.92 mmol) in dichloromethane (10 mL) was cooled with ice, and then diisopropylethylamine (4.67 mL, 26.8 mmol), 1-ethyl-3-(3-dimethylaminopropyl)carbodiimide hydrochloride (4.26 g, 22.32 mmol), 1-hydroxybenzotriazole (3.6 g, 22.32 mmol), and 1-(cyclopropanecarbonyl)piperazine (1.7 g, 8.92 mmol) were added thereto, and the reaction mixture was stirred at room temperature for 12 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (90% ethyl acetate-hexane) to get 3-(4-(cyclopropanecarbonyl)piperazine-1-carbonyl)-4-fluorobenzaldehyde (1.2g, 44%).

Preparation Example 105

(4-(Cyclopropanecarbonyl)piperazin-1-yl)(2-fluoro-5-(hydroxymethyl)phenyl)methanone

A solution of 3-(4-(cyclopropanecarbonyl)piperazine-1-carbonyl)-4-fluorobenzaldehyde (500 mg, 1.64 mmol) in tetrahydrofuran (10 mL) was cooled with ice, and then sodium borohydride (88 mg, 2.46 mmol) was added thereto, and the reaction mixture was stirred at room temperature for 2 h. The reaction solution was diluted with ice-cold water and extracted with ethyl acetate. The organic layer was washed with saturated saline, dried over anhydrous sodium sulfate, and concentrated under reduced pressure. The residue was purified by silica gel column chromatography (20% ethyl acetate-hexane) to get (4-(cyclopropanecarbonyl)piperazin-1-yl)(2-fluoro-5-(hydroxymethyl)phenyl)methanone (350 mg, 69%).

Preparation Example 106

6-(4-Fluorophenoxy)nicotinaldehyde

A solution of 6-chloronicotinaldehyde (2.0 g, 14.18 mmol) in N,N-dimethylformamide (20 mL) was cooled with ice, and then 4-fluorophenol (1.58 g, 14.18 mmol), cesium carbonate (13.8, 42.4 mmol), and copper iodide (1.9 g, 14.18 mmol) were added thereto, and the mixture was heated at 100°C for 1 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (40% ethyl acetate-hexane) to get 6-(4-fluorophenoxy)nicotinaldehyde (2.6 g, 84%).

Preparation Example 107

(6-(4-Fluorophenoxy)pyridin-3-yl)methanol

A solution of 6-(4-fluorophenoxy)nicotinaldehyde (230 mg, 1.06 mmol) in methanol (5 mL) was cooled with ice, and then sodium borohydride (80 mg, 2.22 mmol) was added thereto, and the reaction mixture was stirred at room temperature for 2 h. The reaction mixture was diluted with ice-cold water and extracted with ethyl acetate. The organic layer was washed with saturated saline, dried over anhydrous sodium sulfate, and concentrated under reduced pressure. The residue was purified by silica gel column chromatography (6% methanol-dichloromethane) to get (6-(4-fluorophenoxy)pyridin-3-yl)methanol (130 mg, 56%).

Preparation Example 108

5-Vinylindolin-2-one

To a solution of 5-bromoindolin-2-one (2.0 g, 9.43 mmol) and tributyl(vinyl)tin (5.98 g, 18.86 mmol) in N,N-dimethylformamide (15 mL) was added tetrakis(triphenylphosphine)palladium(0) (2.17 g, 1.86 mmol), and the reaction mixture was heated at 90°C for 16 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (45% ethyl acetate-hexane) to get 5-vinylindolin-2-one (0.9 g, 69%) as off white solid.

Preparation Example 109

5-(Hydroxymethyl)indolin-2-one

A solution of 5-vinylindolin-2-one (250 mg, 1.57 mmol) in dichloromethane (5 mL) was cooled to -30°C, and then ozone gas was injected thereto for 5 min, and the mixture was stirred at room temperature for 30 min. To the reaction mixture were added methanol (3.0 mL), and then sodium borohydride (119 mg, 3.17 mmol), and the reaction mixture was stirred at room temperature for 1 h. To the reaction mixture was added water, and the mixture was extracted with ethyl acetate. The organic layer was dried over anhydrous sodium sulfate and concentrated under reduced pressure. The residue was purified by silica gel column chromatography (70% ethyl acetate-hexane) to get 5-(hydroxymethyl)indolin-2-one (160 mg, 62%) as off white solid.

Preparation Example 110

5-(Chloromethyl)indolin-2-one

A solution of 5-(hydroxymethyl)indolin-2-one (160 mg, 0.98 mmol) in chloroform (5 mL) was cooled with ice, and then thionyl chloride (0.11 mL, 1.47 mmol) was added thereto, and the reaction mixture was stirred at room temperature for 1 h. The reaction mixture was concentrated under reduced pressure. The residue was triturated with pentane to get 5-(chloromethyl)indolin-2-one (160 mg).

Preparation Example 111

tert-Butyl ((3-((1-methyl-1H-pyrazol-4-yl)methyl)-2,4-dioxo-1 -((2-oxoindolin-5-yl)methyl)-1,2,3,4-tetrahydrothieno[2,3-d]pyrimidin-6-yl)sulfonyl)(1-methylcyclopropyl)carbamate

To a solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (100 mg, 0.20 mmol) and 5-(chloromethyl)indolin-2-one (73 mg, 0.40 mmol) in N,N-dimethylformamide (5 mL) were added potassium carbonate (42 mg, 0.30 mmol) and sodium iodide (6 mg, 0.04 mmol), and the reaction mixture was stirred at 80°C for 1 h. To the reaction mixture was added ice-cold water, and the mixture was extracted with dichloromethane. The organic layer was dried over anhydrous sodium sulfate and concentrated under reduced pressure. The residue was purified by silica gel column chromatography (5% methanol-dichloromethane) to get tert-butyl ((3-((1-methyl-1H-pyrazol-4-yl)methyl)-2,4-dioxo-1 -((2-oxoindolin-5-yl)methyl)-1,2,3,4-tetrahydrothieno[2,3-d]pyrimidin-6-yl)sulfonyl)(1-methylcyclopropyl)carbamate (110 mg, 85%) as off white solid.

Example 130

3-((1-Methyl-1H-pyrazol-4-yl)methyl)-N-(1-methylcyclopropyl)-2,4-dioxo-1-((2-oxoindolin-5-yl)methyl)-1,2,3,4-tetrahydrothieno[2,3-d]pyrimidine-6-sulfonamide

A solution of tert-butyl ((3-((1-methyl-1H-pyrazol-4-yl)methyl)-2,4-dioxo-1-((2-oxoindolin-5-yl)methyl)-1,2,3,4-tetrahydrothieno[2,3-d]pyrimidin-6-yl)sulfonyl)(1-methylcyclopropyl)carbamate (110 mg, 0.17 mmol) in dichloromethane (5 mL) was cooled with ice, and then trifluoroacetic acid (1.0 mL) was added thereto, and the reaction mixture was stirred at room temperature for 4 h. The reaction solvent was evaporated under reduced pressure, and the residue was re-dissolved in dichloromethane (25 mL) and washed with a saturated sodium bicarbonate aqueous solution. The organic layer was dried over anhydrous sodium sulfate, and concentrated under reduced pressure. The residue was purified over reverse phase prep-HPLC to get 3-((1-methyl-1H-pyrazol-4-yl)methyl)-N-(1-methylcyclopropyl)-2,4-dioxo-1-((2-oxoindolin-5-yl)methyl)-1,2,3,4-tetrahydrothieno[2,3-d]pyrimidine-6-sulfonamide (28 mg, 30%) as off white solid.

Preparation Example 112

6-Vinylindolin-2-one

To a solution of 6-bromoindolin-2-one (1.0 g, 4.72 mmol) and tributyl(vinyl)tin (2.99 g, 9.43 mmol) in N,N-dimethylformamide (10 mL) were added lithium chloride (600 mg, 14.1 mmol) and dichlorobis(triphenylphosphine)palladium (66 mg, 0.094 mmol), and the mixture was heated at 90°C for 6 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (45% ethyl acetate-hexane) to get 6-vinylindolin-2-one (0.5 g, 66%).

Preparation Example 113

6-(Hydroxymethyl)indolin-2-one

A solution of 6-vinylindolin-2-one (500 mg, 3.14 mmol) in dichloromethane (10 mL) was cooled to -30°C, and then ozone gas was injected thereto for 5 min, and the mixture was stirred at room temperature for 30 min. To the reaction mixture was added methanol (5.0 mL), and then sodium borohydride (238 mg, 6.34 mmol), and the reaction mixture was stirred at room temperature for 1 h. The reaction mixture was diluted with water and extracted with ethyl acetate. The organic layer was washed with saturated saline, dried over anhydrous sodium sulfate, and concentrated under reduced pressure. The residue was purified by silica gel column chromatography (70% ethyl acetate-hexane) to get 6-(hydroxymethyl)indolin-2-one (250 mg, 48%).

Preparation Example 114

6-(Chloromethyl)indolin-2-one

A solution of 6-(hydroxymethyl)indolin-2-one (250 mg, 1.53 mmol) in chloroform (5 mL) was cooled with ice, and then thionyl chloride (0.17 mL, 2.29 mmol) was added thereto, and the reaction mixture was stirred at room temperature for 1 h. The reaction mixture was concentrated under reduced pressure. The residue was triturated with pentane, and the solid was collected by filtration to get 6-(chloromethyl)indolin-2-one (250 mg).

Preparation Example 115

tert-Butyl ((1-(4-fluorophenyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate

To a solution of tert-butyl ((3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (500 mg, 1.01 mmol) and (4-fluorophenyl)boronic acid (210 mg, 1.5 mmol) in dichloroethane (25 mL) were added pyridine (0.4 mL, 5.05 mmol) and copper(II) acetate (271 mg, 1.5 mmol), and the reaction mixture was stirred at room temperature for 16 h. The reaction mixture was diluted with water and extracted with ethyl acetate. The organic layer was washed with 0.1 M hydrochloric acid and saturated saline, dried over anhydrous sodium sulfate, and concentrated under reduced pressure. The residue was purified by silica gel column chromatography (70% ethyl acetate-hexane) to get tert-butyl ((1-(4-fluorophenyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (400 mg, 67%) as off white solid.

Example 136

1-(4-Fluorophenyl)-3-((1-methyl-1H-pyrazol-4-yl)metbyl)-N-(1-methylcyclopropyl)-2,4-dioxo-1,2,3,4-tetrahydrothieno[2,3-d]pyrimidine-6-sulfonamide

A solution of tert-butyl ((1-(4-fluorophenyl)-3-((1-methyl-1H-pyrazol-4-yl)methyl)-2,4-dioxo-1,2,3,4-tetrahydrothieno[2,3-d]pyrimidin-6-yl)sulfonyl)(1-methylcyclopropyl)carbamate (400 mg, 0.68 mmol) in dichloromethane (5 mL) was cooled with ice, and then trifluoroacetic acid (1.0 mL) was added thereto, and the reaction mixture was stirred at room temperature for 1 h. The reaction solvent was evaporated under reduced pressure, and the residue was re-dissolved in dichloromethane (50 mL) and washed with saturated sodium bicarbonate aqueous solution. The organic layer was dried over anhydrous sodium sulfate and concentrated under reduced pressure. The residue was purified over reverse phase prep-HPLC to get 1-(4-fluorophenyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide (180 mg, 54%) as off white solid.

Compounds shown in Tables 2 to 51 were produced in the same manner as in the production methods of Preparation Examples or Examples shown above. Physicochemical data of each compound are shown in Tables 2 to 21 and Tables 52 to 72.

The following abbreviations are used in Tables.

PEx: Preparation Example number
Ex: Example number
Syn: Example number manufactured by the same method
Chemical Structure: structures of chemicals
Data: Physicochemical data
NMR: δ value (ppm) of the signal in $^1$H-NMR in DMSO -$d_6$
ESI+/ESI-: m/z value in liquid chromatography-mass spectrometry (ionization method: ESI) (represented by $[M+H]^+$/$[M-H]^-$, respectively)

The following abbreviations were used in chemical structural formulae.

Boc: tert-Butyloxycarbonyl
CN: Cyano
TBS: tert-Butyldimethylsilyl

The compound having a chemical structural formula with "HCl" in the vicinity thereof represents that the compound was isolated as hydrochloride.

The compound having a chemical structural formula marked with "*" represents that the compound is an isomer having the configuration of the represented structure.

[Table 3]

| PEx | Chemical Structure | Data |
|---|---|---|
| 1 | 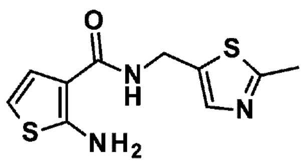 | ESI+: 254.0<br>NMR: δ 8.32 (m, 1H), 7.45 (s, 1H), 7.24 (s, 2H), 7.03 (d, *J* = 5.72 Hz, 1H), 6.25 (d, *J* = 5.76 Hz, 1H), 4.47 (d, *J* = 5.52 Hz, 2H) and 2.57 (s, 3H) |
| 2 | 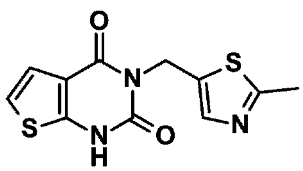 | ESI+: 280.1<br>NMR: δ 12.35 (s, 1H), 7.56 (s, 1H), 7.18 (d, *J* = 5.56 Hz, 1H), 7.13 (d, *J* = 5.56 Hz, 1H), 5.12 (s, 2H) and 2.56 (s, 3H) |

(continued)

| PEx | Chemical Structure | Data |
|---|---|---|
| 3 | 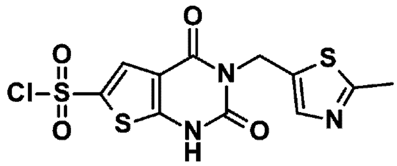 | |
| 4 | 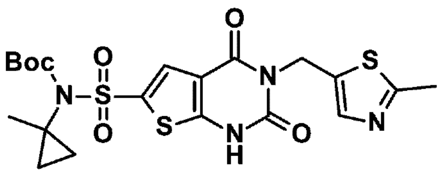 | ESI+: 513.0<br><br>NMR: δ 12.46 (s, 1H), 8.15 (s, 1H), 7.49 (S, 1H), 5.06 (s, 2H), 3.04 (s, 3H), 1.40 (s, 9H), 1.38 (s, 3H) and 0.90 (m, 4H) |
| 5 | 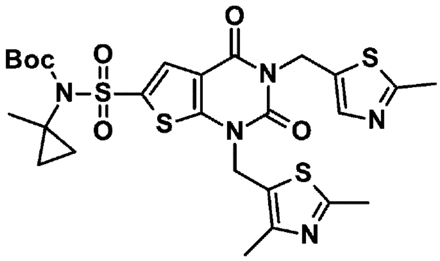 | ESI+: 638.1 |
| 6 | 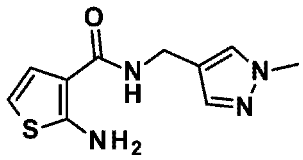 | ESI+: 237.0<br><br>NMR: δ 8.06 (s, 1H), 7.53 (s, 1H), 7.30 (s, 2H), 7.19 (s, 1H), 7.07 (d, *J* = 6.0 Hz, 1H), 6.24 (d, *J* = 5.6 Hz, 1H), 4.18 (d, *J* = 5.6 Hz, 2H) and 3.76 (s, 3H) |

[Table 4]

| PEx | Chemical Structure | Data |
|---|---|---|
| 7 | 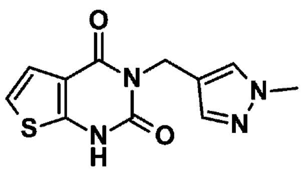 | ESI+: 263.0<br><br>NMR: δ 12.23 (r s, 1H), 7.60 (s, 1H), 7.33 (s, 1H), 7.16 (d, *J* = 5.6 Hz, 1H), 7.10 (t, *J* = 5.52 Hz, 1H), 4.82 (s, 2H), and 3.74 (s, 3H) |
| 8 | 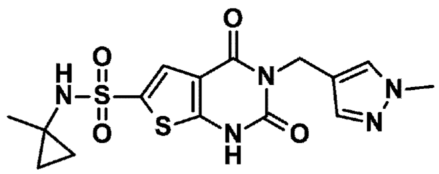 | ESI+: 396.0<br><br>NMR: δ 12.52 (brs, 1H), 8.36 (s, 1H), 7.76 (s, 1H), 7.75 (s, 1H), 7.34 (s, 1H), 4.81 (s, 2H), 3.75 (s, 3H), 1.28 (s, 3H), 0.69 (m, 2H) and 0.45 (m, 2H) |
| 9 | 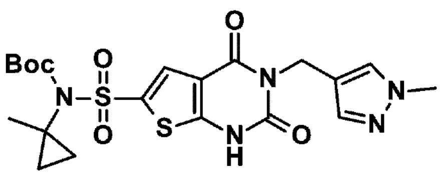 | ESI+: 496.2<br><br>NMR: δ 12.48 (s, 1H), 7.75 (s, 1H), 7.62 (s, 1H), 7.33 (s, 1H), 4.81 (s, 2H), 3.74 (s, 3H), 1.43 (s, 9H), 1.42 (s, 3H) and 0.90 (m, 4H) |

(continued)

| PEx | Chemical Structure | Data |
|---|---|---|
| 10 | 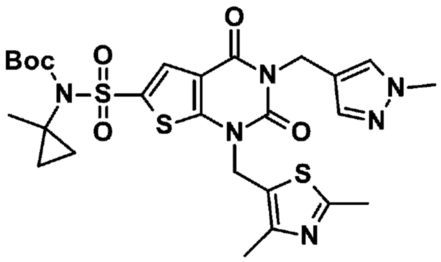 | ESI+: 621.2 |
| 11 | 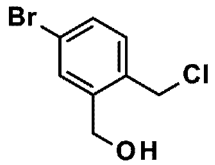 | ESI+: 235.5 |
| 12 | 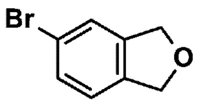 | ESI+: 198.0, 200.0 |

[Table 5]

| PEx | Chemical Structure | Data |
|---|---|---|
| 13 | 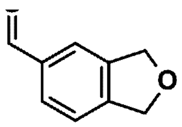 | ESI+: 149.2 |
| 14 | 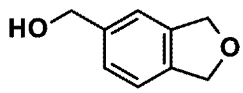 | ESI+: 151.0 |
| 15 | 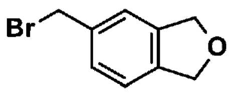 | ESI+: 213.0 |
| 16 | 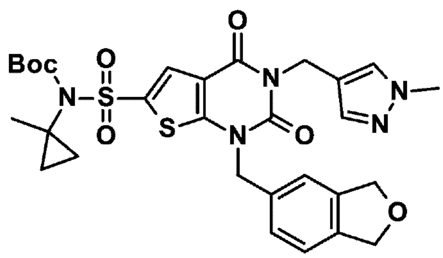 | ESI+: 628.4 |
| 17 | 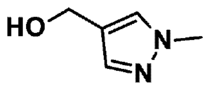 | ESI+: 113.1 |
| 18 | 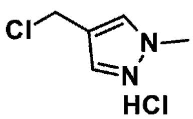 | ESI+: 131.6 |

(continued)

| PEx | Chemical Structure | Data |
|---|---|---|
| 19 | 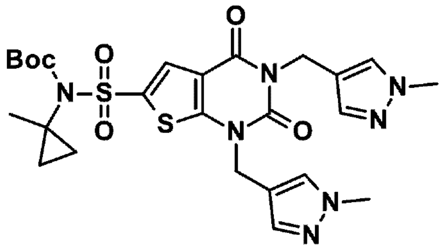 | ESI+: 590.1 |
| 20 | 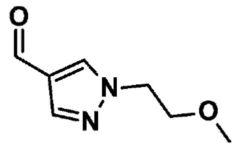 | ESI+: 155.1 |

[Table 6]

| PEx | Chemical Structure | Data |
|---|---|---|
| 21 | 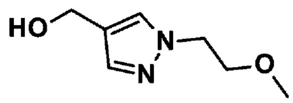 | ESI+: 157.0 |
| 22 | 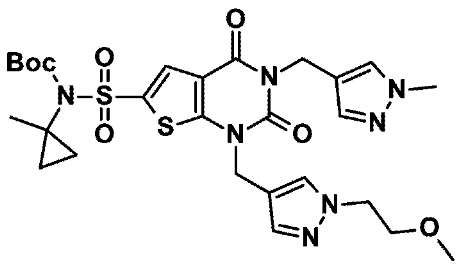 | ESI+: 634.2 |
| 23 | 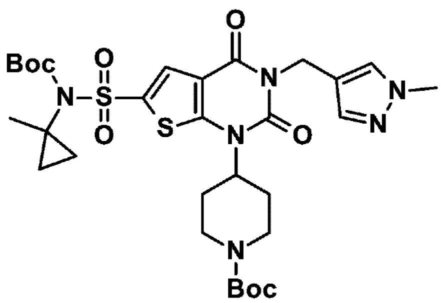 | ESI+: 679.4 |
| 24 | 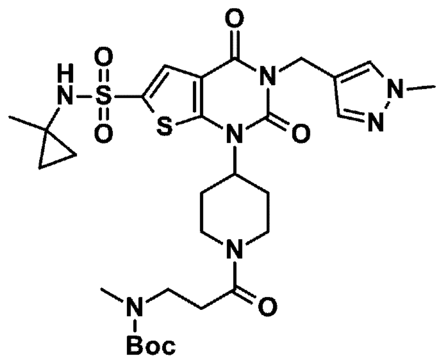 | ESI+: 664.2 |

(continued)

| PEx | Chemical Structure | Data |
|---|---|---|
| 25 | 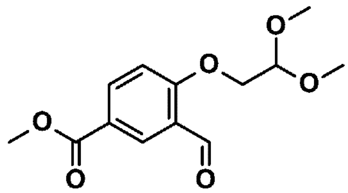 | ESI+: 269.3 |

[Table 7]

| PEx | Chemical Structure | Data |
|---|---|---|
| 26 | 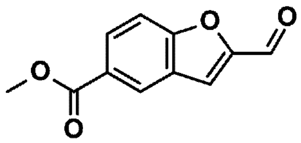 | ESI+: 205.2 |
| 27 | 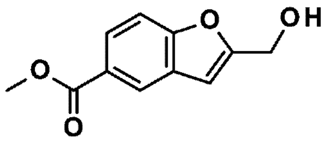 | ESI+: 207.2 |
| 28 | 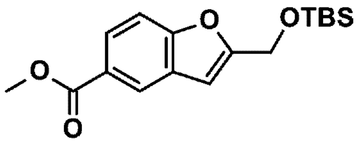 | ESI+: 321.3 |
| 29 | 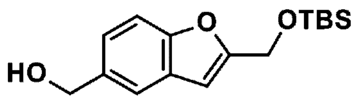 | ESI+: 293.4 |
| 30 | 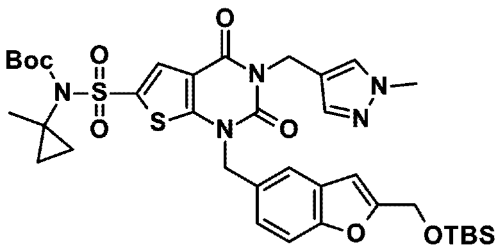 | ESI+: 770.1 |
| 31 | 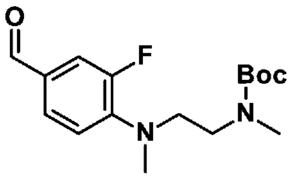 | ESI+: 311.1 |
| 32 | 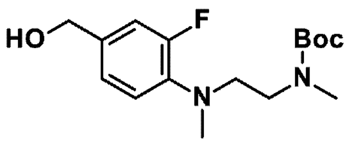 | ESI+: 313.2 |

[Table 8]

| PEx | Chemical Structure | Data |
|---|---|---|
| 33 | 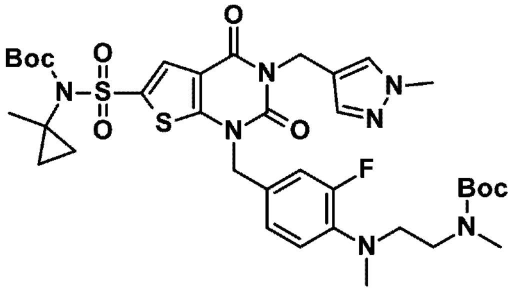 | ESI+: 790.5 |
| 34 | 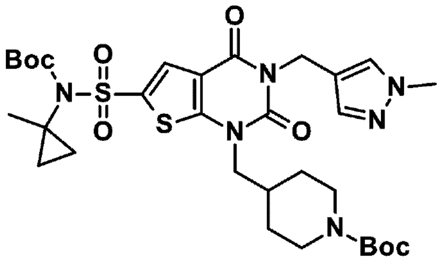 | ESI+: 693.2 |
| 35 | 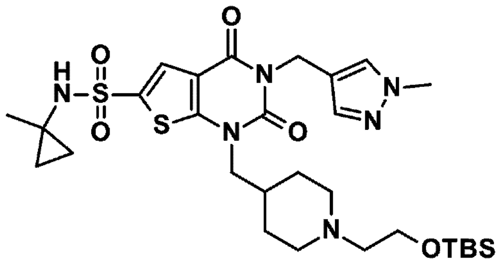 | ESI+: 651.2 |

[Table 9]

| PEx | ChemicalStructure | Data |
|---|---|---|
| 36 | 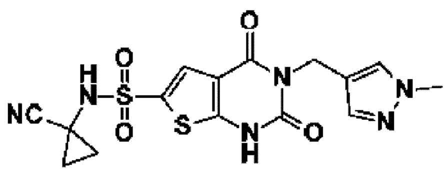 | ESI + :407.2<br><br>NMR:δ12.57(brs,1H),9.48(s, 1H),7.68(s,1H),7.65(s,1H), 7.35(s,1H),4.83(s,2H),3.76 (s,3H),1.49(s,2H),1.34(s,2 H) |
| 37 | 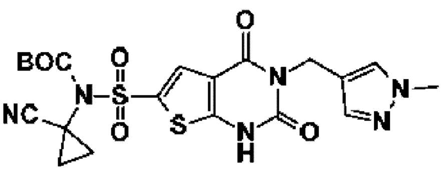 | ESI+:507.0 |
| 38 | 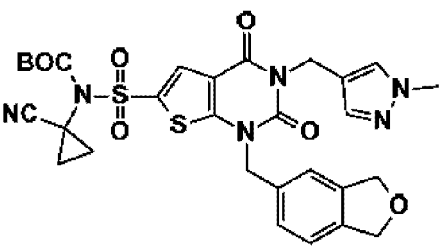 | ESI+:639.10 |

(continued)

| PEx | ChemicalStructure | Data |
|---|---|---|
| 39 | 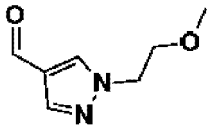 | ESI+:155.0 |
| 40 | 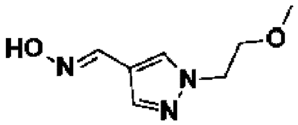 | ESI+: 170.1 |
| 41 | 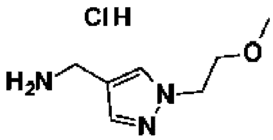 | ESI+: 156.2 |

[Table 10]

| PEx | ChemicalStructure | Data |
|---|---|---|
| 42 | 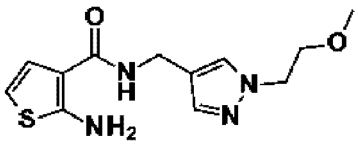 | ESI+:281.1<br>NMR:δ8.04(t,J=5.6Hz,1H),7. 55(s,1H),7.33(s,1H),7.19 (s, 2H),7.06(d,J=6.0Hz,1H),6.2 3(d,J=6.0Hz,1H), 4.22-4.17 (m,4H),3.62(t,J=5.2Hz,2H), 3.20(s,3H) |
| 43 | 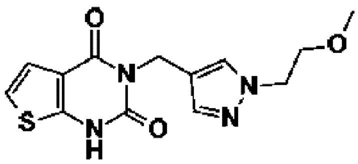 | ESI+:307.2<br>NMR:δ7.58(s,1H),7.33(s,1 H),7.19(s,1H),7.08(d,J=6.0 Hz,1H),6.23(d,1=5.6Hz,1H), 4.20-4.17(m,4H),3.63(t,J= 5.2Hz,2H),3.21(s,3H) |
| 44 | 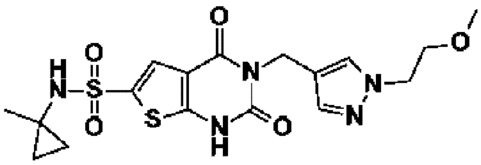 | ESI+:440.2 |
| 45 | 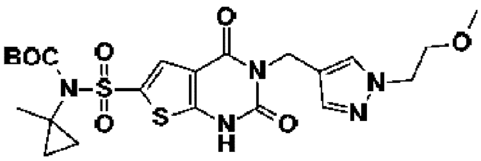 | ESI+: 540.2 |
| 46 | 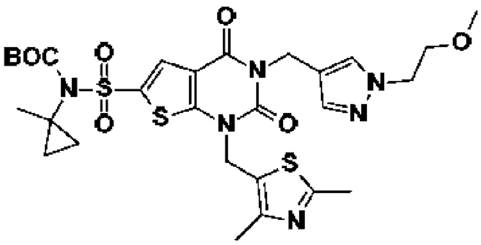 | ESI+: 665.3 |

[Table 11]

| PEx | ChemicalStructure | Data |
|---|---|---|
| 48 | 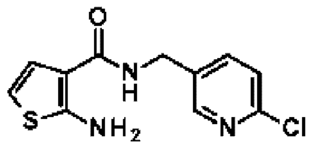 | ESI+:266.0 |
| 49 | 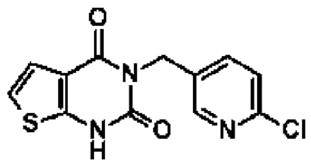 | ESI+:294.1 |
| 50 | 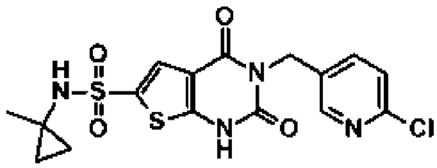 | ESI+:427.1 |
| 51 | 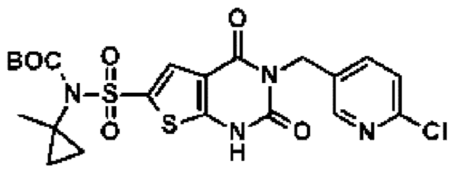 | ESI+:527.1 |
| 52 | 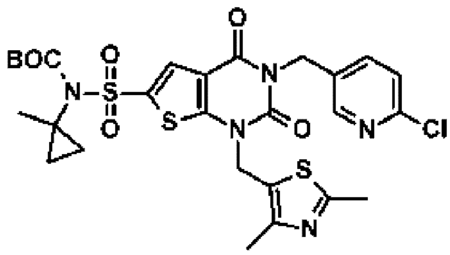 | ESI+:652.2 |

[Table 12]

| PEx | ChemicalStructure | Data |
|---|---|---|
| 53 | 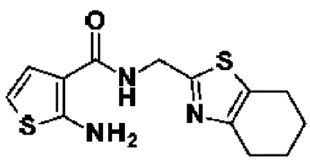 | ESI+: 294.1<br>NMR : 58.50(d,J=5.2Hz,1H), 7.25(s,2H),7.09(d,J=6.0Hz, 1H),6.26(d,J=6.0Hz,1H),4.5 2(d,J=5.6Hz,2H),2.67-2.63 (m, 4H), 1. 75 (m,4H) |
| 54 | 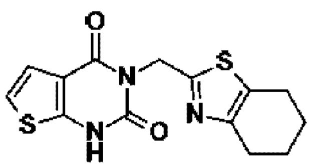 | ESI+: 320.2 |
| 55 | 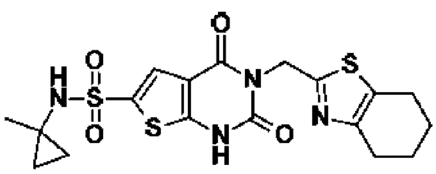 | ESI+:453.0<br>NMR:δ12.61(brs,1H),8.39(s, 1H),7.53(s,1H),5.20(s,2H), 2.67-2.60(m,4H),1.75(m,4 H),1.18(s,3H),0.70-0.67(m, 2H), 0.48-0.45(m,2H) |

(continued)

| PEx | ChemicalStructure | Data |
|---|---|---|
| 56 | 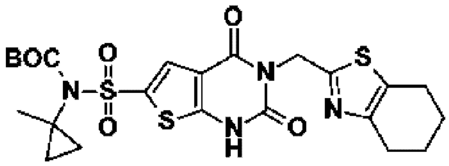 | ESI+: 553.2 |
| 57 | 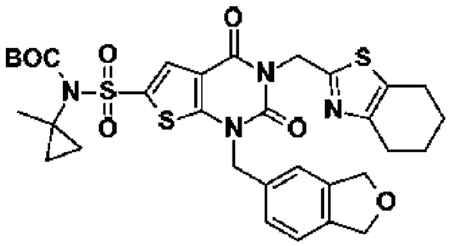 | ESI+:685.3 |

[Table 13]

| PEx | ChemicalStructure | Data |
|---|---|---|
| 58 | 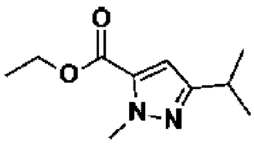 | ESI+:197.1 |
| 59 | 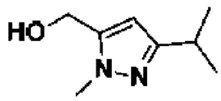 | ESI+: 155.1 |
| 60 | 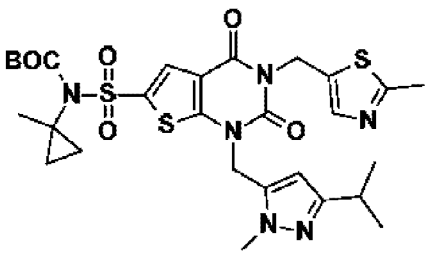 | ESI+:649.2 |
| 61 | 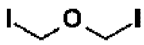 | NMR:δ5.72(s,4H) |
| 62 | 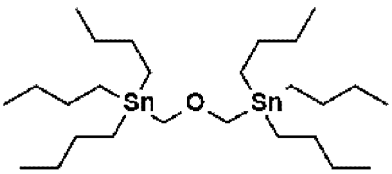 | NMR:δ3.66(s,4H),1.55-1.43 (m, 12H), 1.34-1.26(m, 12H), 0.93-0.85(m,30H) |
| 63 | 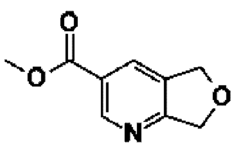 | ESI+: 180.0 |

(continued)

| PEx | ChemicalStructure | Data |
|---|---|---|
| 64 | 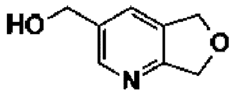 | ESI+: 152.0 |

[Table 14]

| PEx | ChemicalStructure | Data |
|---|---|---|
| 65 | 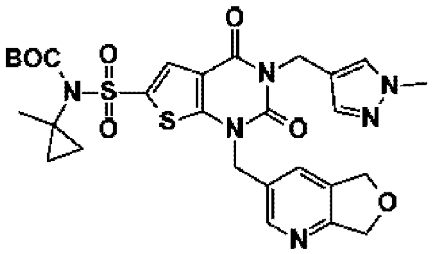 | ESI+: 629.3 |
| 66 | 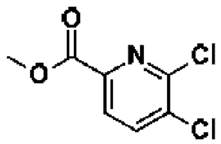 | NMR:δ8.32(d,J=8,0Hz,1H), 8.04(d,J=8.0Hz,1H),3.89(s, 3H) |
| 67 | 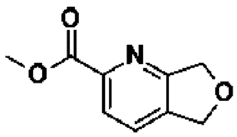 | ESI+: 180.1 |
| 68 | 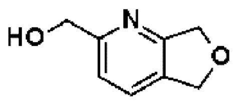 | NMR:δ7.73(d,J=7,6Hz,1H), 7.35(d,J=8.0Hz,1H),5.42(t,J =6.0Hz,1H),5.05(s,2H),4.89 (s,2H),4.56(d,J=6.0Hz,2H) |
| 69 | 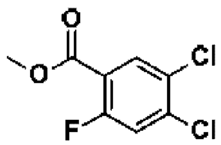 | NMR:δ8.06(d,J=6,8Hz,1H), 7.91(d,J=10.4Hz,1H),3.86 (s,3H) |
| 70 | 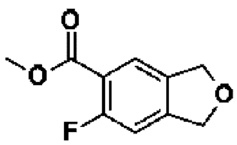 | ESI+:197.0 |

[Table 15]

| PEx | ChemicalStructure | Data |
|---|---|---|
| 71 | 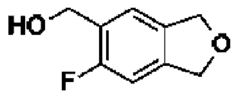 | NMR:57.37(d,3 =7.6Hz, 1H), 7.12(d,J=9.6Hz,1H),5.25(t,J =5.6Hz,1H),4.96(s, 4H),4.53 (d, I = 5.6Hz,2H) |

(continued)

| PEx | ChemicalStructure | Data |
|---|---|---|
| 72 | 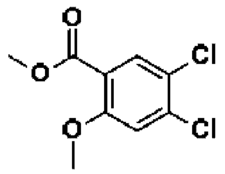 | NMR:δ7.84(s,1H),7.46(s,1 H),3.85(s,3H),3.79(s,3H) |
| 73 | 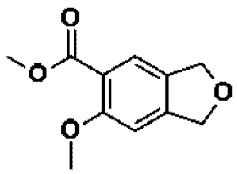 | NMR:δ7.57(s,1H),7.13(s,1 H),5.09-4.94(m,4H),3.80(s, 3H),3.76(s,3H) |
| 74 | 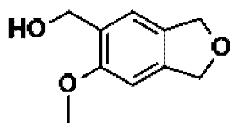 | NMR:δ7.27(s,1H),6.89(s,1 H),5.01-4.94(m,5H),4.47(d, J=6.8Hz,2H),3.75(s,3H). |
| 75 | 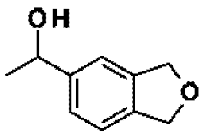 | NMR:δ7.26-7.22(m,3H),5.14 (d,J=4.0Hz,1H),4.96(s,4H), 4.74-4.71(m,1H),1.31 (d,J= 6.4Hz, 3H) |
| 76 | 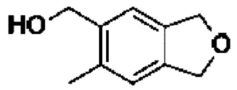 | NMR:δ7.27(s,1H),7.05(s,1 H),5.07(t,J=5.2Hz,1H),4.97-4.9 5(m,4H),4.48(d,3 = 5.2 Hz, 2H),2.23(s,3H) |

[Table 16]

| PEx | ChemicalStructure | Data |
|---|---|---|
| 77 | 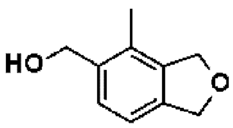 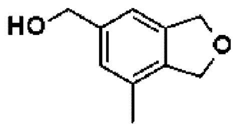 | NMR: 67.26(d, I =7.6Hz, 1H), 7.07(d,J=8.0Hz,1H),7.03(s, 1H), 6.99(s,1H),5.12(t,J=6.0 Hz,1H),5.02(t,J=5.5Hz,1H), 4.99-4.95 (m,8H),4.49-4.45 (m,4H),2.18(s,3H),2.12(s,3 H) |
| 78 | 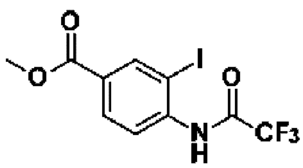 | ESI+: 3 72. 1 |
| 79 | 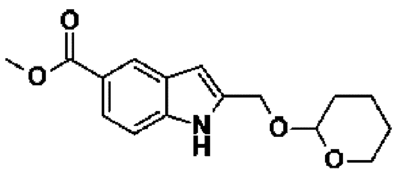 | NMR:δ11.53(s,1H),8.20(s,1 H),7.71(dd,J=8.4&1.6Hz,1 H),7.40 (d,J=8.0Hz,1H),6.53 (s,1H),4.78-4.71(m,2H),4.6 0-4.57(m,1H), 3.80(s,3H),3. 50-3.47(m,1H),1.75-1.62 (m, 2 H), 1. 53 -1. 48 (m, 5 H) |

(continued)

| PEx | ChemicalStructure | Data |
|---|---|---|
| 80 | 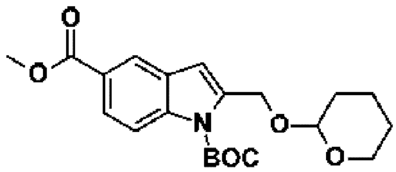 | ESI+:390.2 |
| 81 | 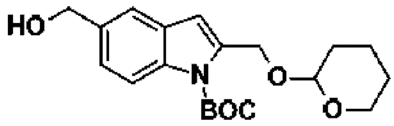 | ESI+:360.2 |
| 82 | 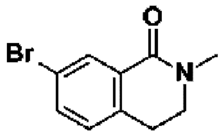 | ESI+:240.2 |

[Table 17]

| PEx | ChemicalStructure | Data |
|---|---|---|
| 83 | 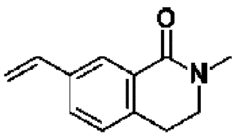 | ESI+: 188.1 |
| 84 | 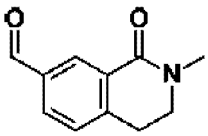 | ESI+: 190.3 |
| 85 | 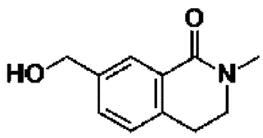 | ESI+:192.1 |
| 86 | 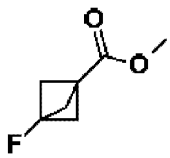 | NMR:δ3.06(s,3H),2.19-2.14 (m,6H) |
| 87 | 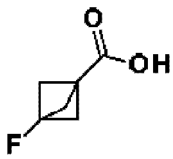 | ESI+: 129.1 |
| 88 | 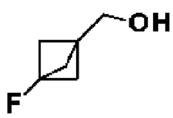 | ESI+:117.1 |

(continued)

| PEx | ChemicalStructure | Data |
|---|---|---|
| 89 | 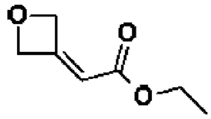 | ESI+: 143.1 |

[Table 18]

| PEx | ChemicalStructure | Data |
|---|---|---|
| 90 | 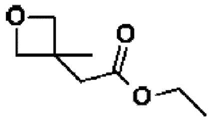 | NMR:δ4.44(d,J=6.0Hz,2H), 4.22(d,J = 6.0Hz,2H),4.07-4. 02(m,2H), 2.66(s,2H),1.32 (s,3H),1.17(t,J=7.2Hz,3H) |
| 91 | 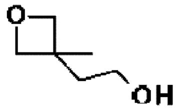 | NMR:δ4.37-4.35(m,3H),4.16 (d,J=5.6Hz,2H),3.47-3.41 (m,2H), 1.76-1.72(m,2H),1.2 4(s,3H) |
| 92 | 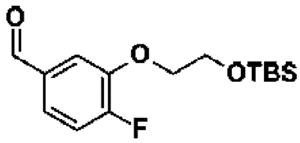 | NMR:δ(s,1H),7.54(d,J=6.8H z,1H),7.4(brs,1H),7.22-7.2 0,1H),4.19(t, J=6.0Hz,2H),4. 03(t,J=4.8Hz,2H),0.90(s,9 H),0.09(s,6H) |
| 93 | 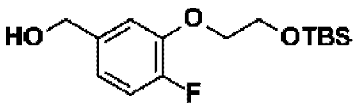 | NMR:δ(m,2H),6.8(t,J=4.4H z,1H),5.2(t,J=6.0Hz,1H),4.4 (t,J=5.6Hz, 2H),4.08(t,J=4.4 Hz,2H),3.9(t,3=4.8Hz,2H),0. 85(s,9H),0.06(s,6H) |
| 94 | 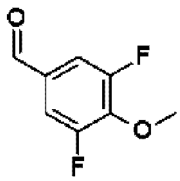 | NMR:δ(s,1H), 7.72-7. 70(m,2 H),4.07(s,3H) |
| 95 | 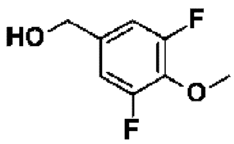 | NMR:δ(m,2H),5.37(t,J=4.8H z,1H),4.43(s,2H),3.88(s,3H) |

[Table 19]

| PEx | ChemicalStructure | Data |
|---|---|---|
| 96 | 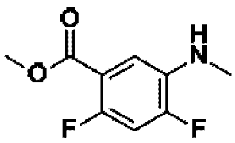 | ESI+:202.2 |
| 97 | 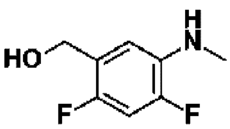 | ESI+:174.1 |

(continued)

| PEx | ChemicalStructure | Data |
|---|---|---|
| 98 | 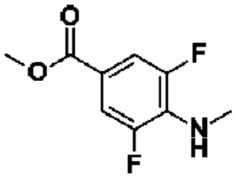 | ESI+:202.0 |
| 99 | 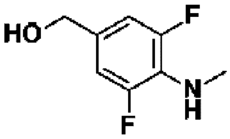 | ESI+: 174.2 |
| 100 | 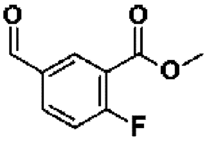 | NMR:δ10.04(s,1H),8.45-8.4 3(m,1H),8.22-8.18(m,1H),7. 62-7.57(m,1H)3.90 (s,3H) |
| 101 | 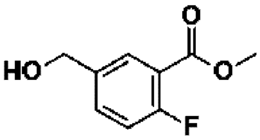 | NMR:δ7.84(d,J=7.2Hz,1H), 7.59-7.56(m,1H),7.32-7.27 (m,1H),5.35(t, J=5.6Hz,1H) 4.52(d,J=6.0Hz,2H),3.85(s, 3H) |
| 102 | 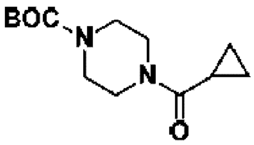 | ESI+:255.1 |

[Table 20]

| PEx | ChemicalStructure | Data |
|---|---|---|
| 103 | 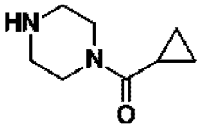 | ESI+: 155.1 |
| 104 | 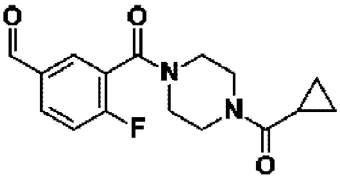 | ESI + :305.0 |
| 105 | 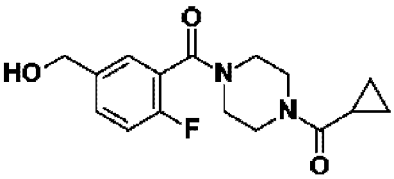 | NMR:δ7.43-7.43(m,3H),5.30 (m,1H),4.50-4.49(m,2H),3.7 6-3.43 (m,6H),3.31-3.21(m, 2H),2.00-1.93(m,1H),0.80-0.73(m,4H) |

(continued)

| PEx | ChemicalStructure | Data |
|---|---|---|
| 106 | 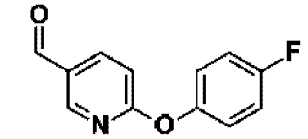 | ESI+:218.0 |
| 107 | 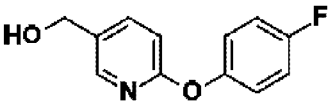 | ESI+:220.1 |
| 108 | 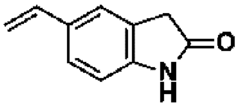 | ESI+: 159.9 |
| 109 | 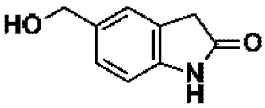 | ESI+: 164.0 |
| 110 | 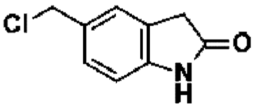 | ESI+: 182.1 |

[Table 21]

| PEx | ChemicalStructure | Data |
|---|---|---|
| 111 | 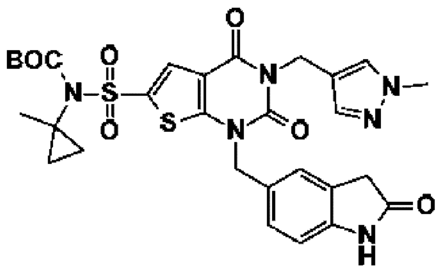 | ESI+: 641.1 |
| 112 | 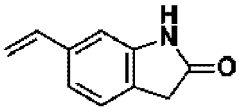 | ESI+: 160.1 |
| 113 | 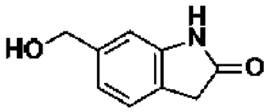 | ESI+: 164.1 |
| 114 | 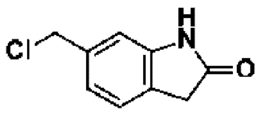 | ESI+: 182.2 |

(continued)

| PEx | ChemicalStructure | Data |
|---|---|---|
| 115 | 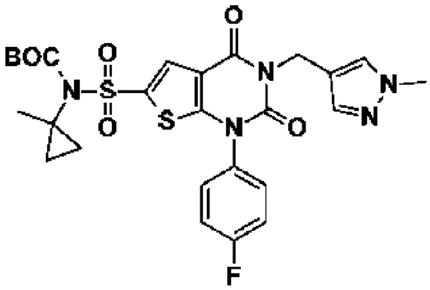 | ESI+: 590.1 |

[Table 22]

| Ex | Syn | Chemical Structure |
|---|---|---|
| 1 | 1 | 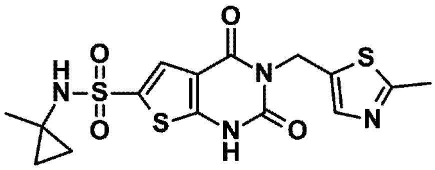 |
| 2 | 2 | 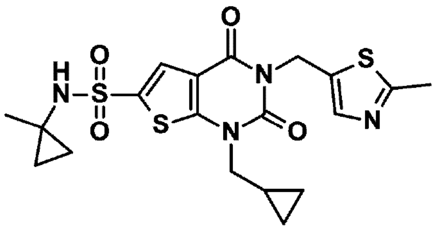 |
| 3 | 2 | 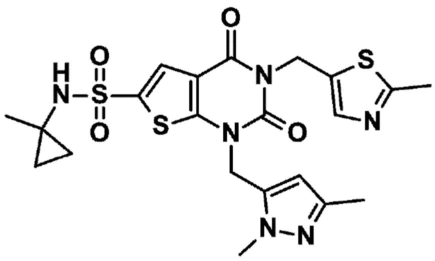 |
| 4 | 2 | 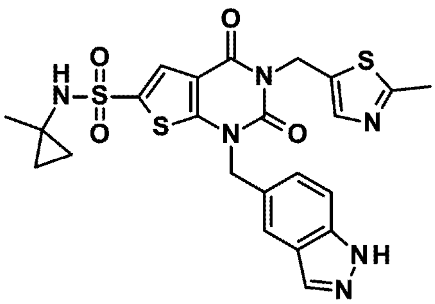 |

(continued)

| Ex | Syn | Chemical Structure |
|---|---|---|
| 5 | 2 | 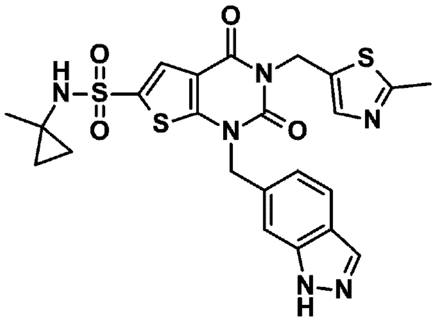 |

[Table 23]

| Ex | Syn | Chemical Structure |
|---|---|---|
| 6 | 6 | 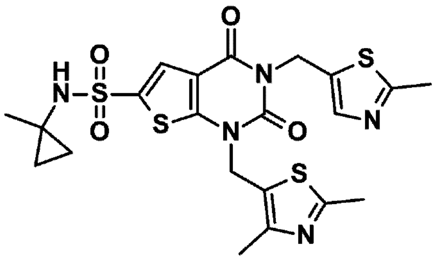 |
| 7 | 6 | 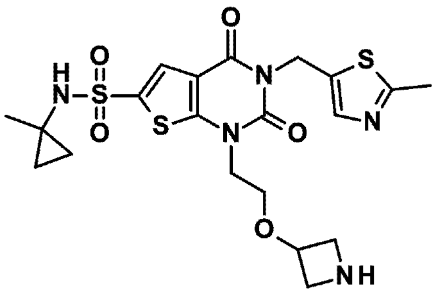 |
| 8 | 6 | 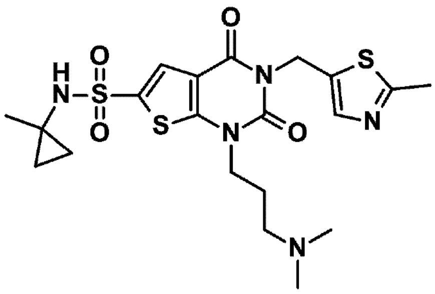 |
| 9 | 6 | 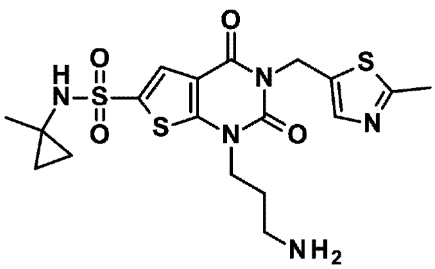 |

(continued)

| Ex | Syn | Chemical Structure |
|---|---|---|
| 10 | 6 | 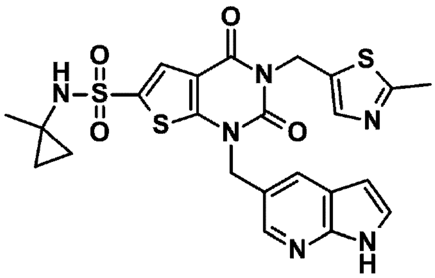 |

[Table 24]

| Ex | Syn | Chemical Structure |
|---|---|---|
| 11 | 6 | 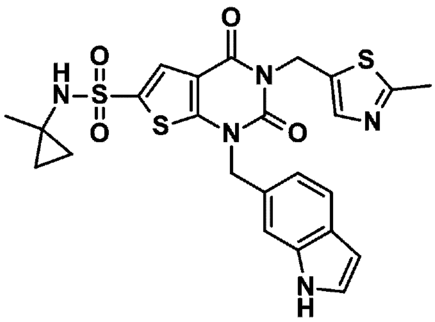 |
| 12 | 6 | 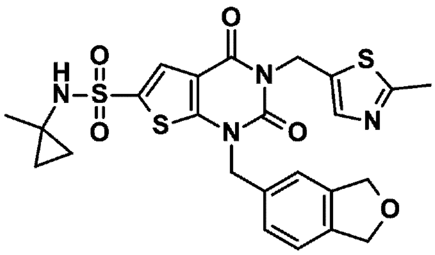 |
| 13 | 6 | 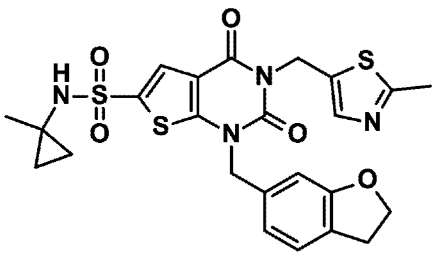 |
| 14 | 6 | 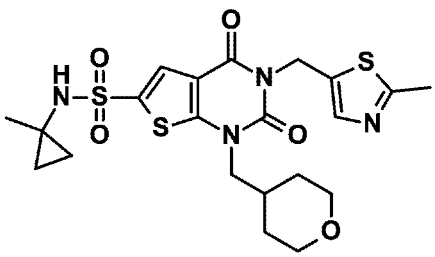 |

(continued)

| Ex | Syn | Chemical Structure |
|---|---|---|
| 15 | 6 | 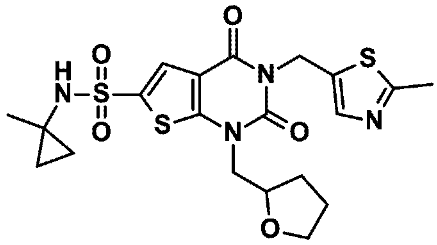 |

[Table 25]

| Ex | Syn | Chemical Structure |
|---|---|---|
| 16 | 6 | 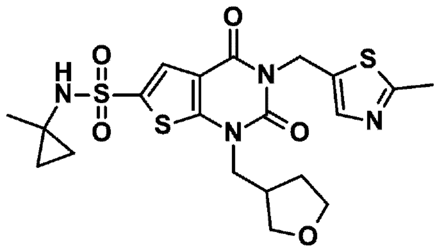 |
| 17 | 6 | 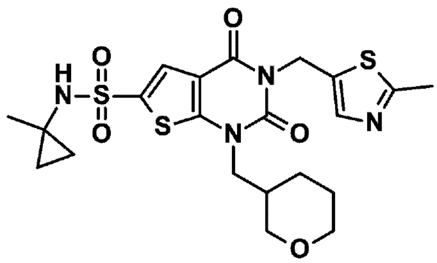 |
| 18 | 6 | 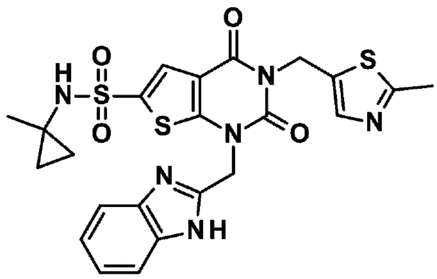 |
| 19 | 6 | 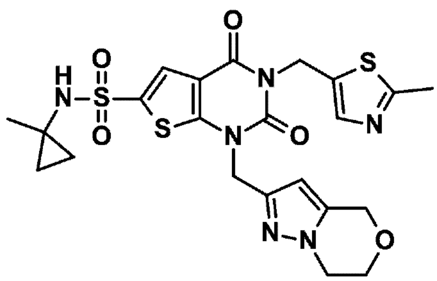 |

(continued)

| Ex | Syn | Chemical Structure |
|---|---|---|
| 20 | 6 | 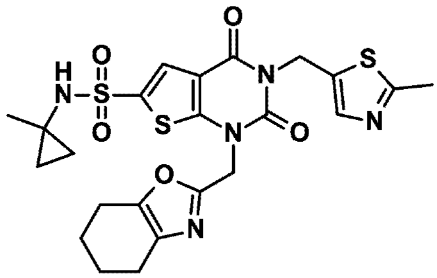 |

[Table 26]

| Ex | Syn | Chemical Structure |
|---|---|---|
| 21 | 2 | 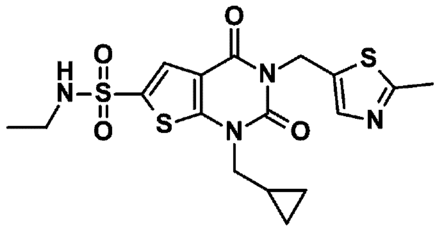 |
| 22 | 2 | 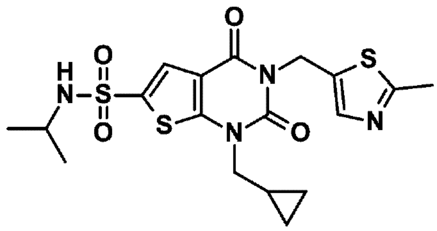 |
| 23 | 2 | 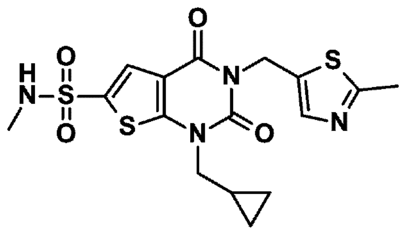 |
| 24 | 6 | 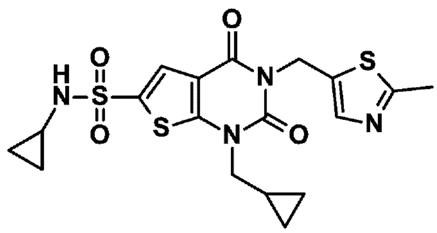 |

(continued)

| Ex | Syn | Chemical Structure |
|---|---|---|
| 25 | 25 | 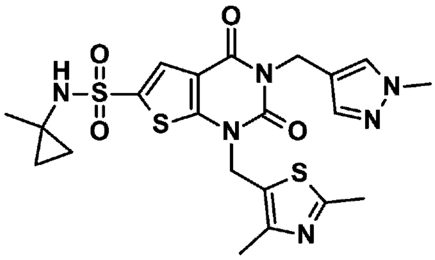 |

[Table 27]

| Ex | Syn | Chemical Structure |
|---|---|---|
| 26 | 25 | 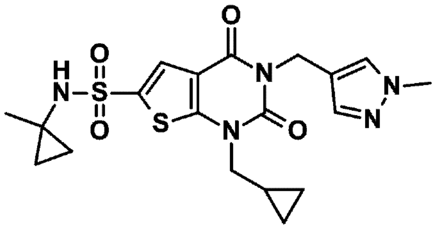 |
| 27 | 25 | 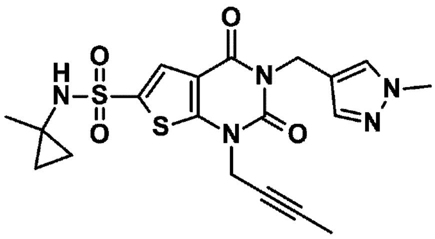 |
| 28 | 28 | 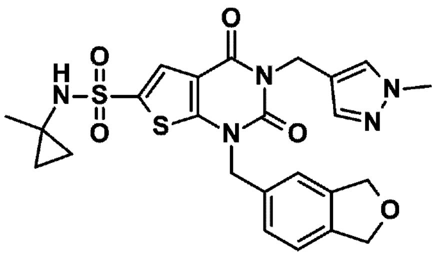 |
| 29 | 29 | 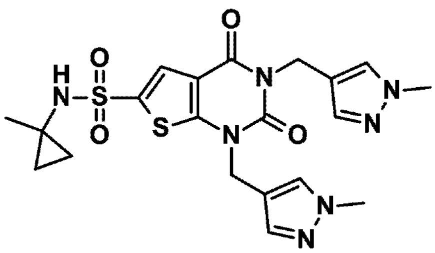 |

(continued)

| Ex | Syn | Chemical Structure |
|---|---|---|
| 30 | 25 | 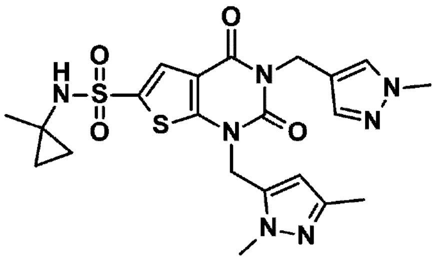 |

[Table 28]

| Ex | Syn | Chemical Structure |
|---|---|---|
| 31 | 25 | 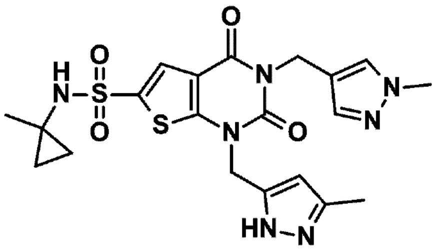 |
| 32 | 25 | 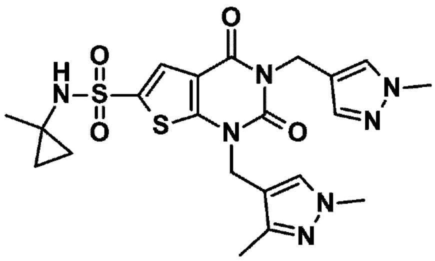 |
| 33 | 25 | 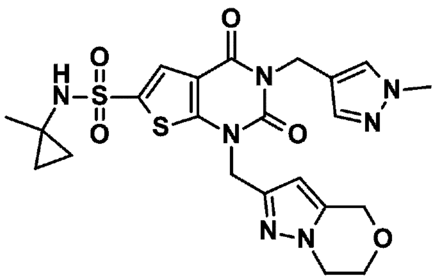 |
| 34 | 25 | 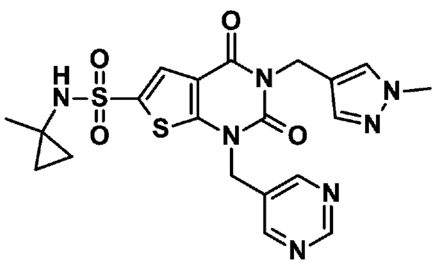 |

(continued)

| Ex | Syn | Chemical Structure |
|---|---|---|
| 35 | 25 | 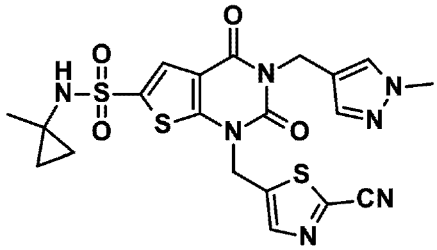 |

[Table 29]

| Ex | Syn | Chemical Structure |
|---|---|---|
| 36 | 25 | 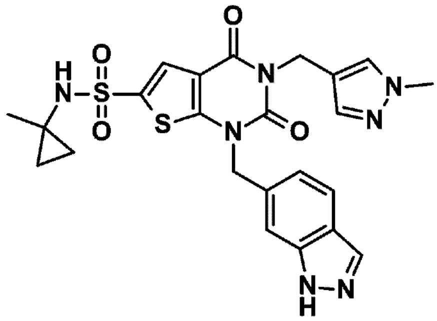 |
| 37 | 25 | 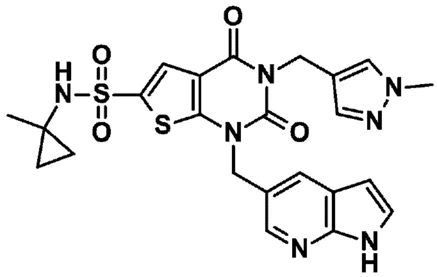 |
| 38 | 25 | 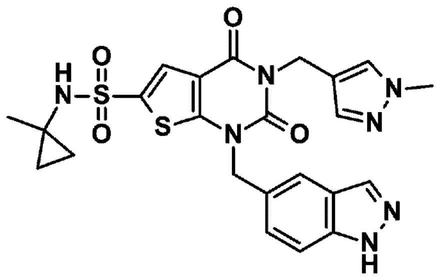 |
| 39 | 25 | 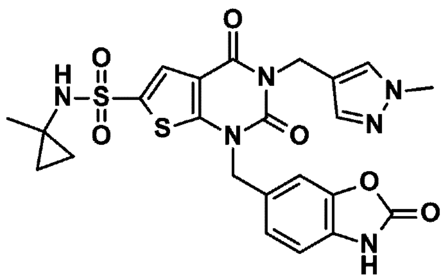 |

(continued)

| Ex | Syn | Chemical Structure |
|---|---|---|
| 40 | 25 | 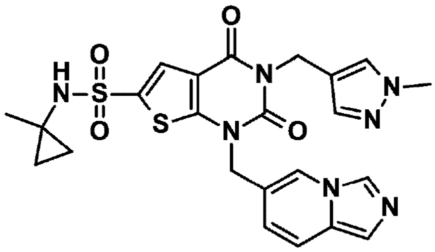 |

[Table 30]

| Ex | Syn | Chemical Structure |
|---|---|---|
| 41 | 25 | 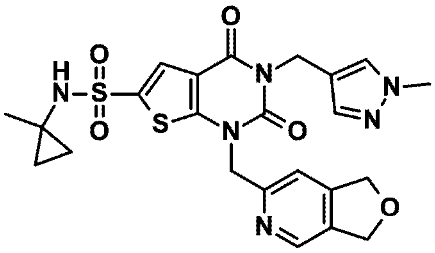 |
| 42 | 25 | 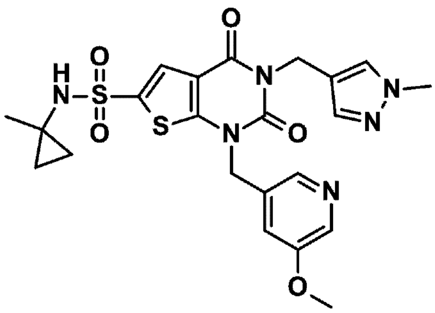 |
| 43 | 25 | 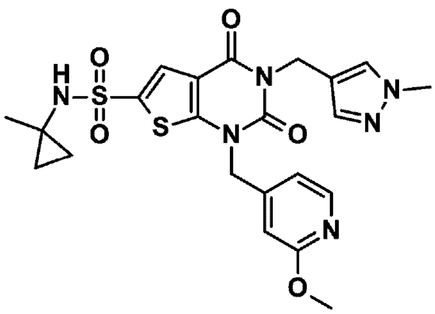 |
| 44 | 25 | 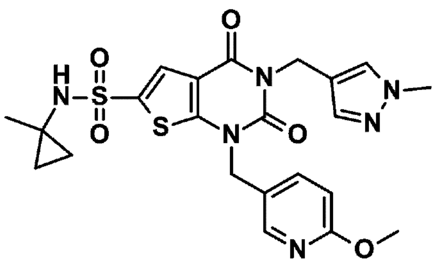 |

(continued)

| Ex | Syn | Chemical Structure |
|---|---|---|
| 45 | 25 | 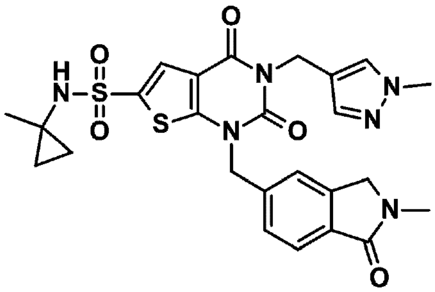 |

[Table 31]

| Ex | Syn | Chemical Structure |
|---|---|---|
| 46 | 25 | 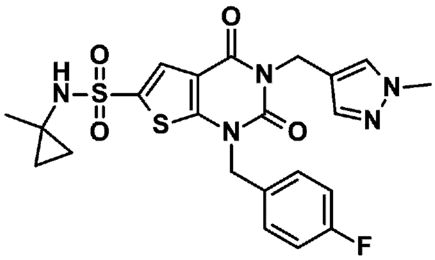 |
| 47 | 25 | 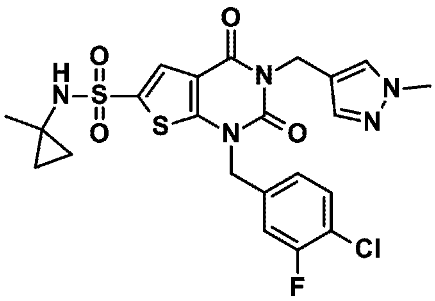 |
| 48 | 25 | 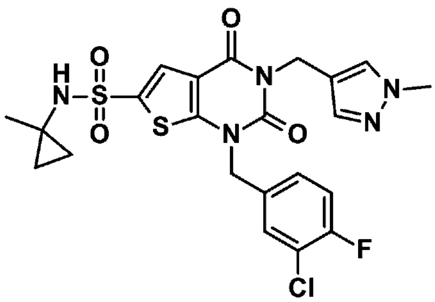 |
| 49 | 25 | 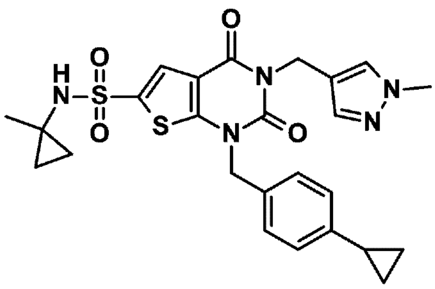 |

(continued)

| Ex | Syn | Chemical Structure |
|---|---|---|
| 50 | 25 | 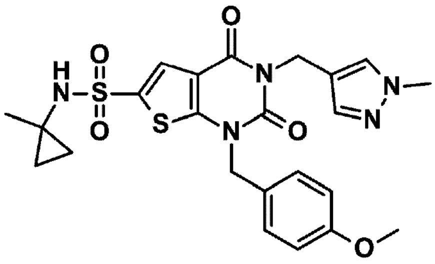 |

[Table 32]

| Ex | Syn | Chemical Structure |
|---|---|---|
| 51 | 25 | 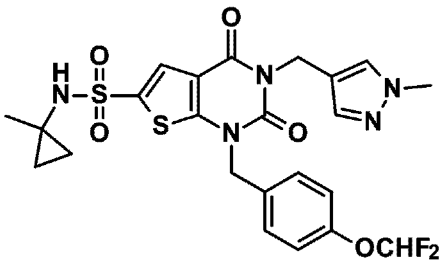 |
| 52 | 25 | 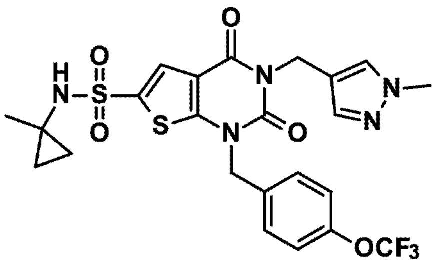 |
| 53 | 25 | 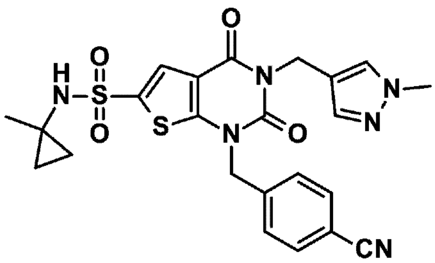 |
| 54 | 25 | 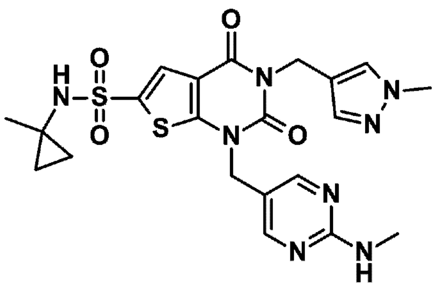 |

(continued)

| Ex | Syn | Chemical Structure |
|---|---|---|
| 55 | 6 | 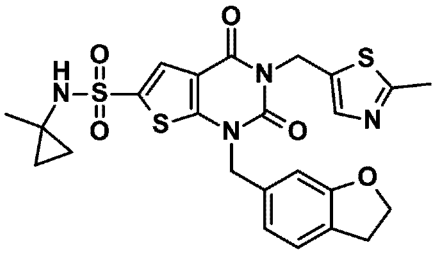 |

[Table 33]

| Ex | Syn | Chemical Structure |
|---|---|---|
| 56 | 25 | 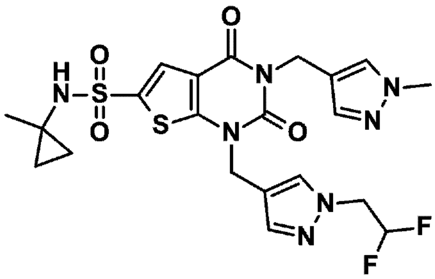 |
| 57 | 57 | 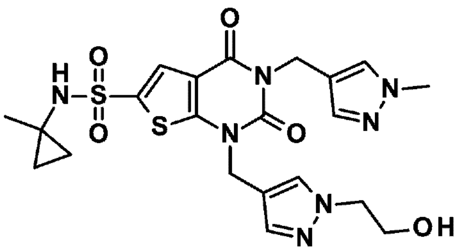 |
| 58 | 25 | 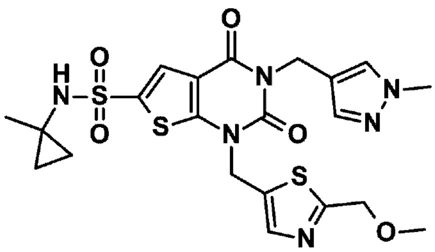 |
| 59 | 25 | 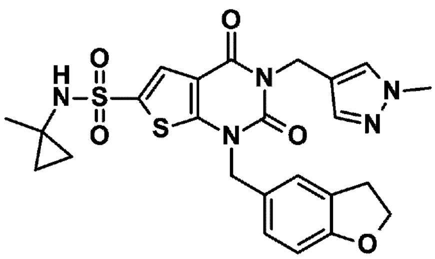 |

(continued)

| Ex | Syn | Chemical Structure |
|---|---|---|
| 60 | 25 | 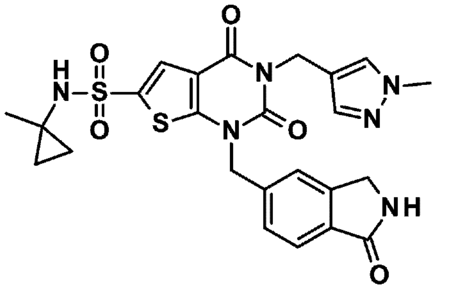 |

[Table 34]

| Ex | Syn | Chemical Structure |
|---|---|---|
| 61 | 25 | 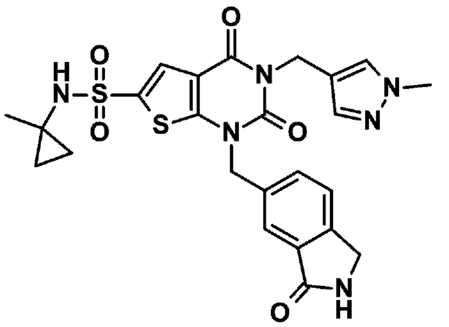 |
| 62 | 25 | 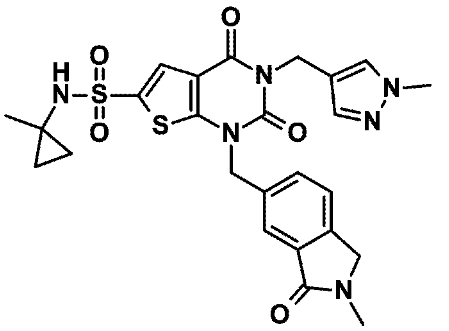 |
| 63 | 25 | 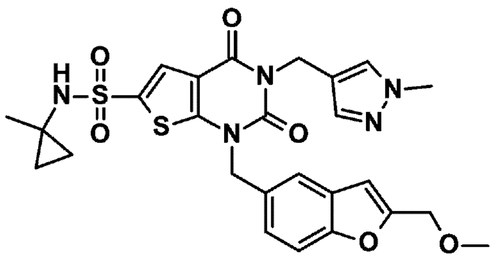 |

(continued)

| Ex | Syn | Chemical Structure |
|---|---|---|
| 64 | 64 | 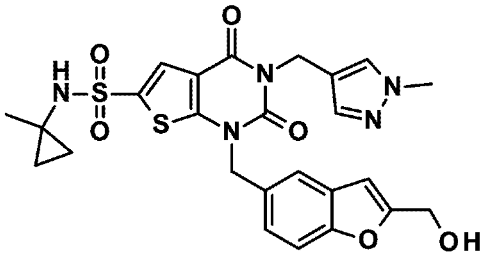 |
| 65 | 25 | 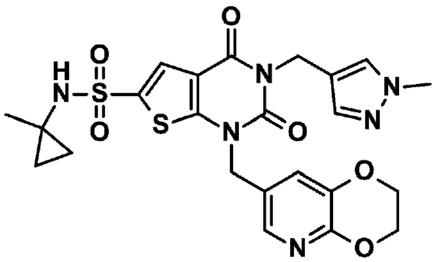 |

[Table 35]

| Ex | Syn | Chemical Structure |
|---|---|---|
| 66 | 25 | 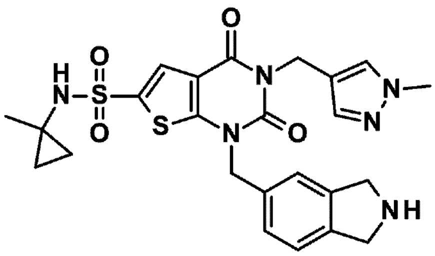 |
| 67 | 78 | 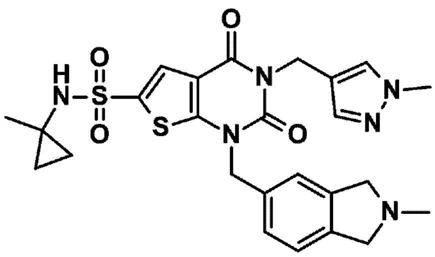 |
| 68 | 25 | 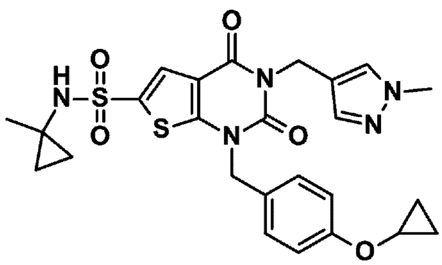 |

(continued)

| Ex | Syn | Chemical Structure |
|---|---|---|
| 69 | 25 | 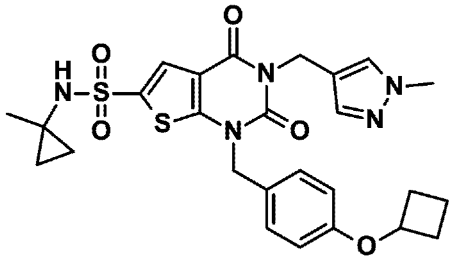 |
| 70 | 25 | 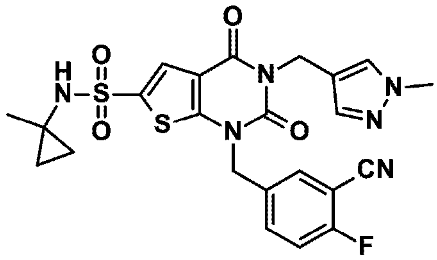 |

[Table 36]

| Ex | Syn | Chemical Structure |
|---|---|---|
| 71 | 25 | 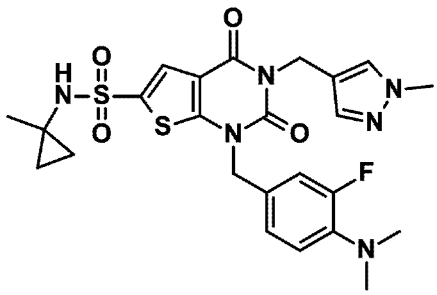 |
| 72 | 25 | 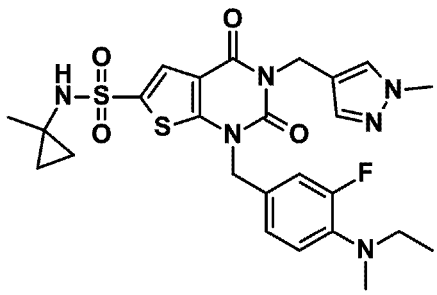 |
| 73 | 25 | 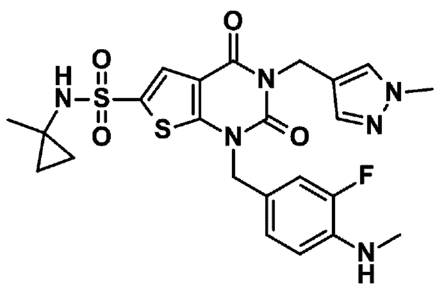 |

(continued)

| Ex | Syn | Chemical Structure |
|---|---|---|
| 74 | 25 | 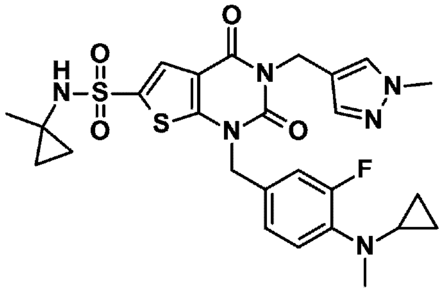 |
| 75 | 25 | 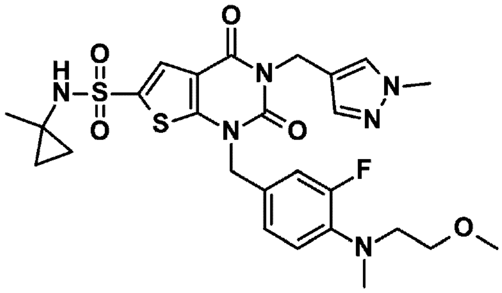 |

[Table 37]

| Ex | Syn | Chemical Structure |
|---|---|---|
| 76 | 57 | 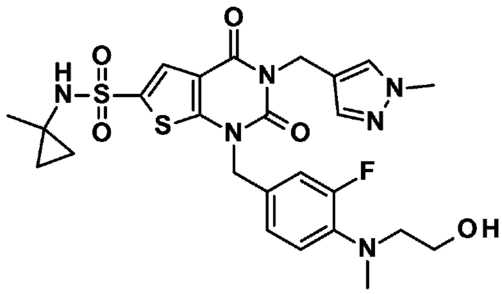 |
| 77 | 77 | 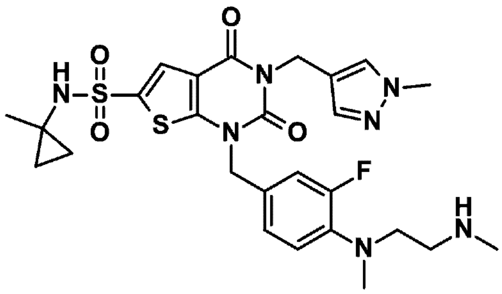 |

(continued)

| Ex | Syn | Chemical Structure |
|---|---|---|
| 78 | 78 | 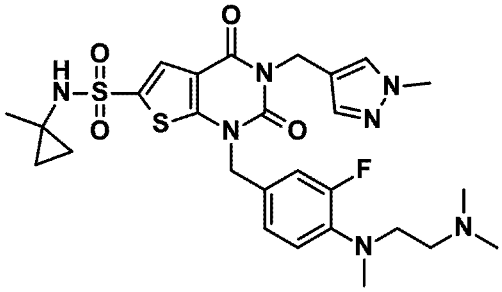 |
| 79 | 25 | 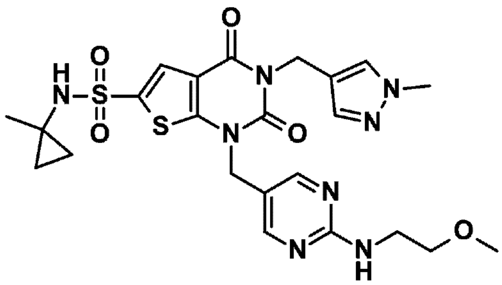 |
| 80 | 57 | 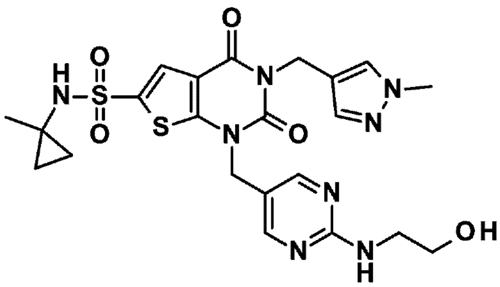 |

[Table 38]

| Ex | Syn | Chemical Structure |
|---|---|---|
| 81 | 25 | 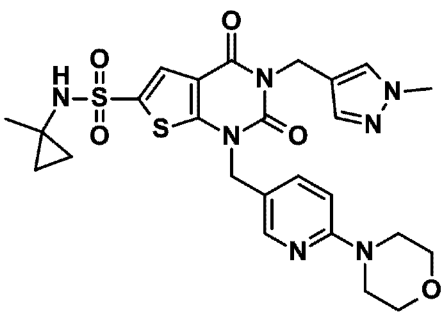 |

(continued)

| Ex | Syn | Chemical Structure |
|---|---|---|
| 82 | 25 | 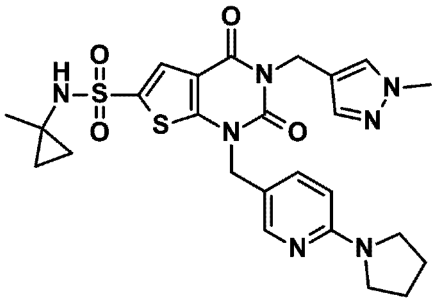 |
| 83 | 25 | 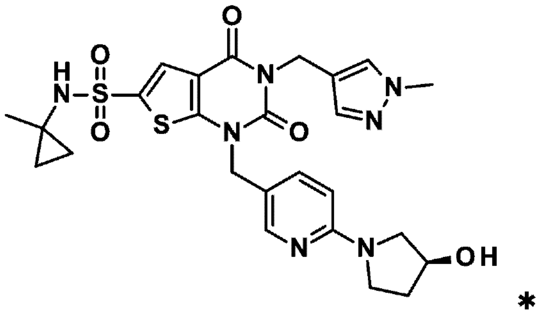 |
| 84 | 25 | 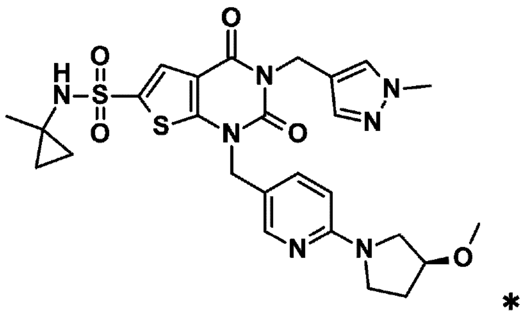 |

[Table 39]

| Ex | Syn | Chemical Structure |
|---|---|---|
| 85 | 25 | 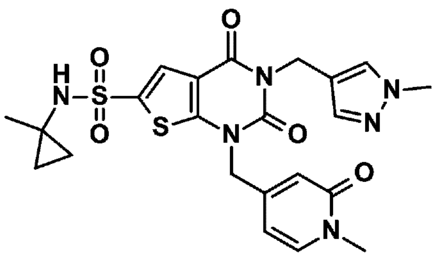 |

(continued)

| Ex | Syn | Chemical Structure |
|---|---|---|
| 86 | 25 | 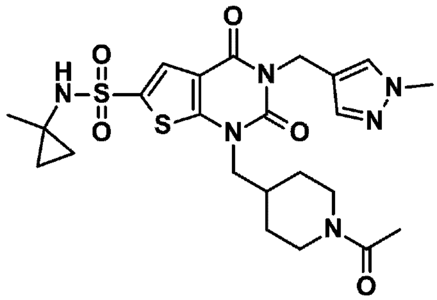 |
| 87 | 25 | 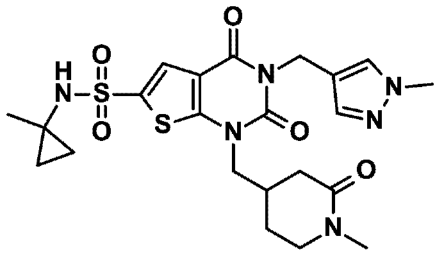 |
| 88 | 25 | 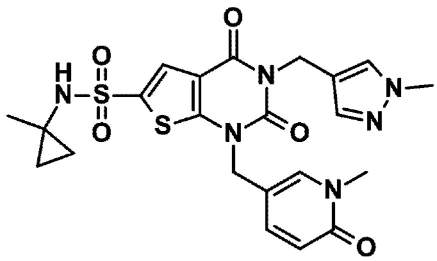 |
| 89 | 25 | 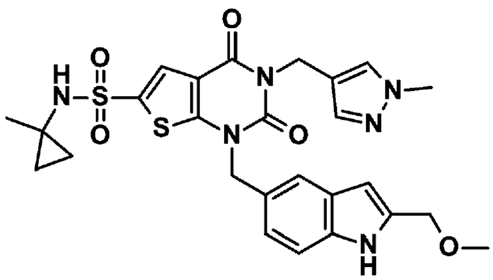 |

[Table 40]

| Ex | Syn | Chemical Structure |
|---|---|---|
| 90 | 57 | 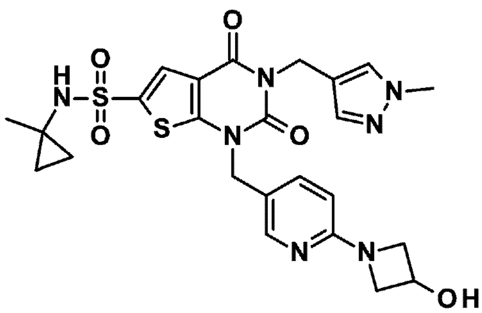 |

(continued)

| Ex | Syn | Chemical Structure |
|---|---|---|
| 91 | 91 | 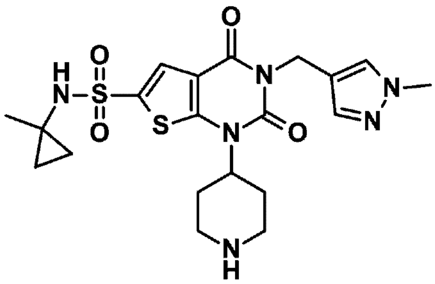 |
| 92 | 92 | 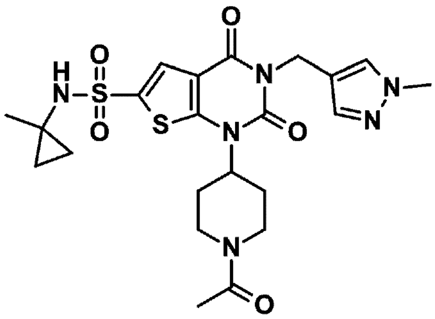 |
| 93 | 92 | 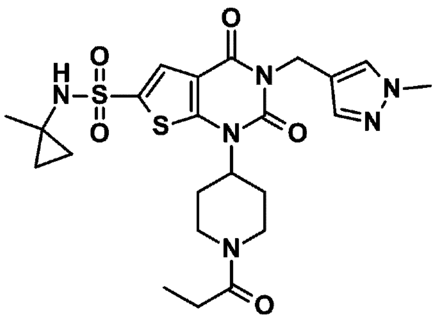 |

[Table 41]

| Ex | Syn | Chemical Structure |
|---|---|---|
| 94 | 92 | 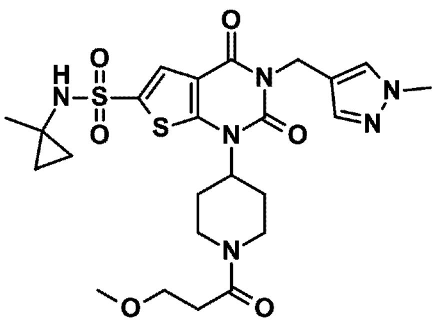 |

(continued)

| Ex | Syn | Chemical Structure |
|---|---|---|
| 95 | 95 | 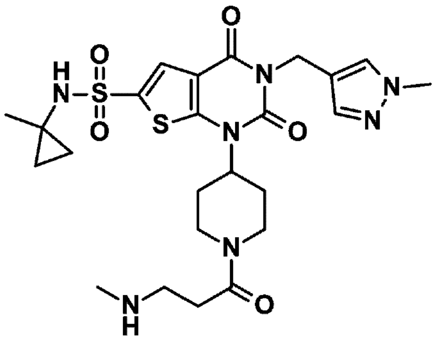 |
| 96 | 95 | 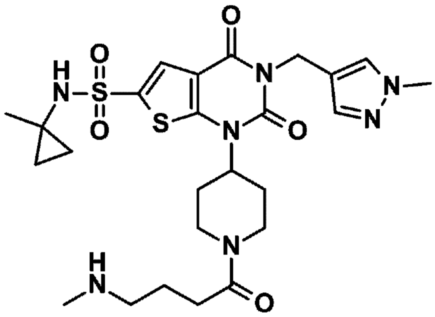 |
| 97 | 97 | 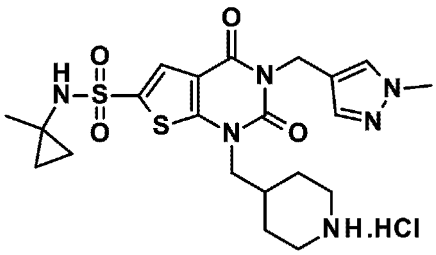 |

[Table 42]

| Ex | Syn | Chemical Structure |
|---|---|---|
| 98 | 98 | 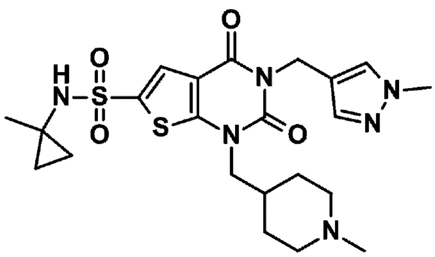 |
| 99 | 99 | 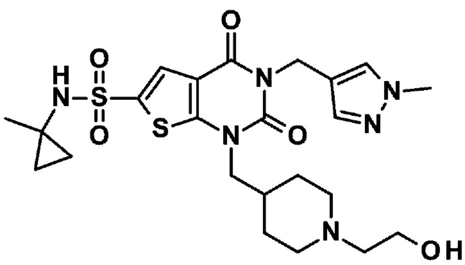 |

[Table 43]

| Ex | Syn | Chemical Structure |
|---|---|---|
| 100 | 100 | 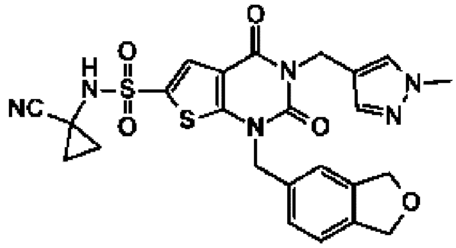 |
| 101 | 100 | 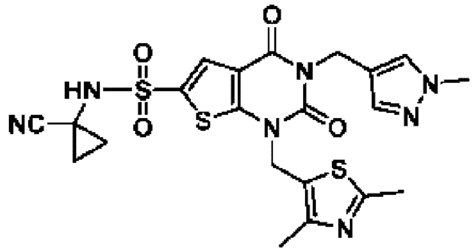 |
| 102 | 100 | 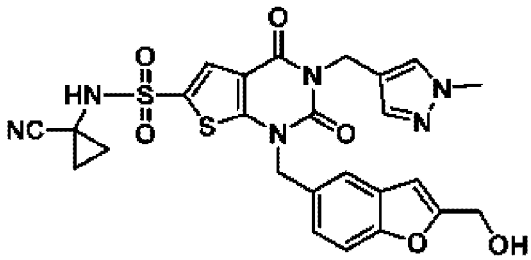 |
| 103 | 100 | 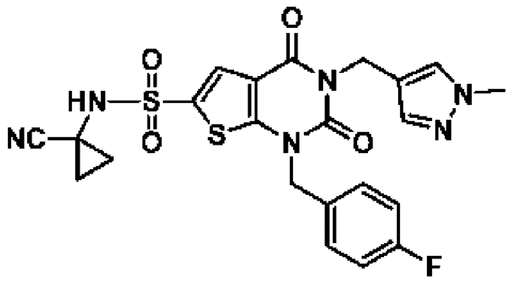 |
| 104a | 104 | 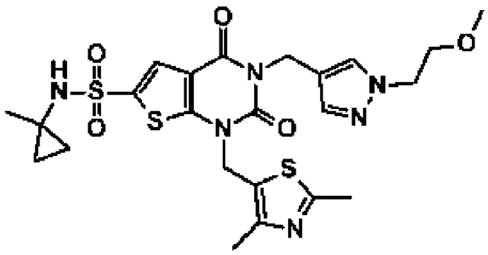 |

[Table 44]

| Ex | Syn | Chemical Structure |
|---|---|---|
| 104b | 104 | 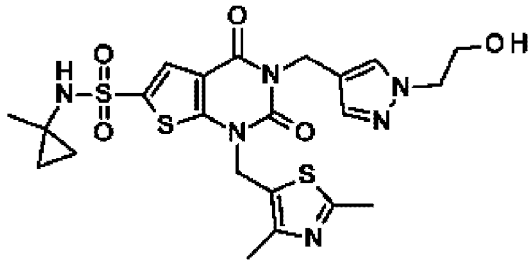 |
| 105 | 105 | 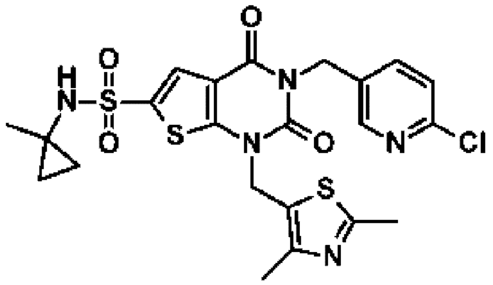 |
| 106 | 106 | 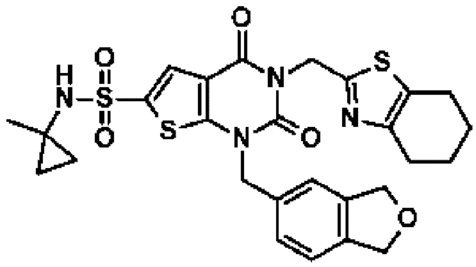 |
| 107 | 107 | 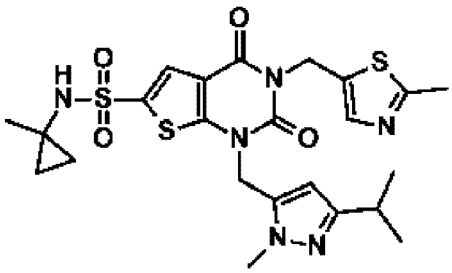 |
| 108 | 108 | 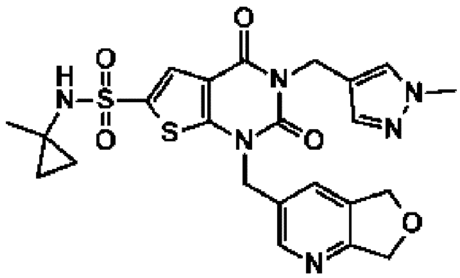 |

[Table 45]

| Ex | Syn | Chemical Structure |
|---|---|---|
| 109 | 108 | 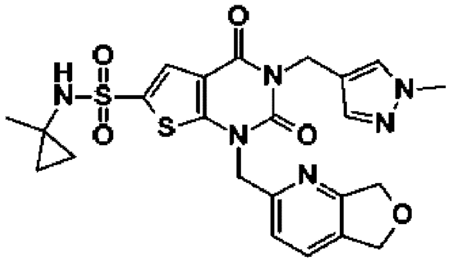 |
| 110 | 108 | 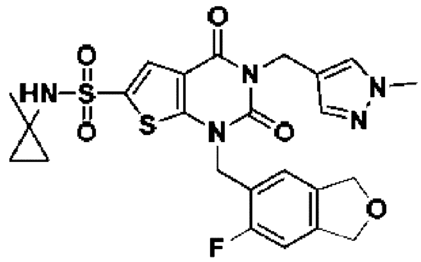 |
| 111 | 108 | 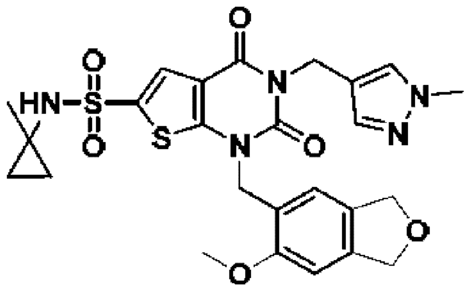 |
| 112 | 108 | 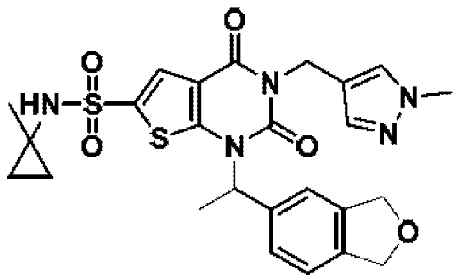 |

[Table 46]

| Ex | Syn | Chemical Structure |
|---|---|---|
| 113 | 108 | 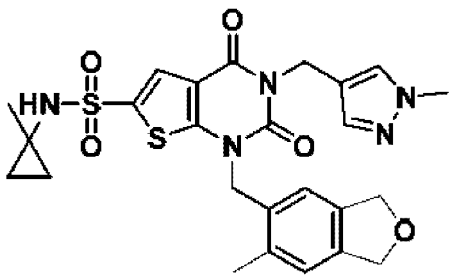 |

(continued)

| Ex | Syn | Chemical Structure |
|---|---|---|
| 114 | 108 | 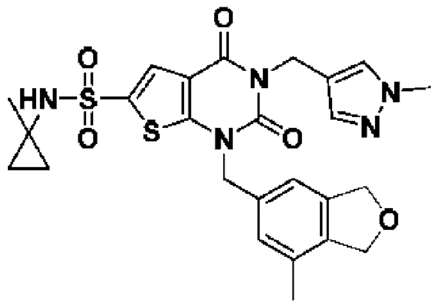 |
| 115 | 108 | 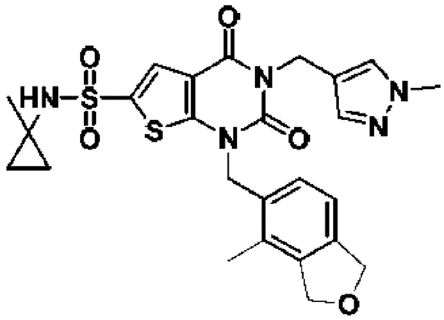 |
| 116 | 108 | 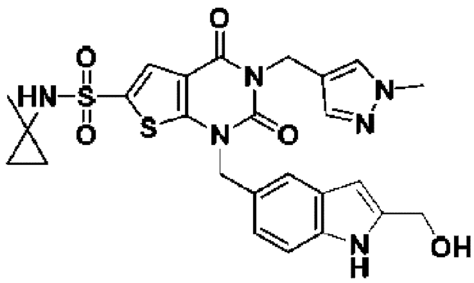 |

[Table 47]

| Ex | Syn | Chemical Structure |
|---|---|---|
| 117 | 108 | 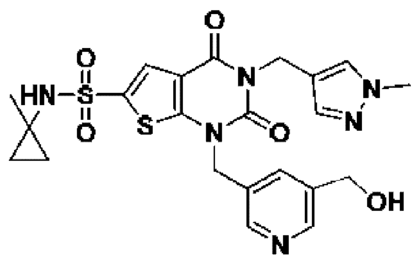 |

(continued)

| Ex | Syn | Chemical Structure |
|---|---|---|
| 118 | 108 | 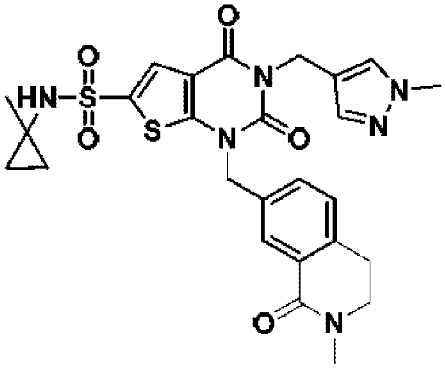 |
| 119 | 108 | 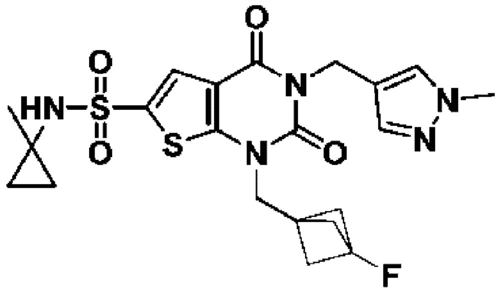 |
| 120 | 108 | 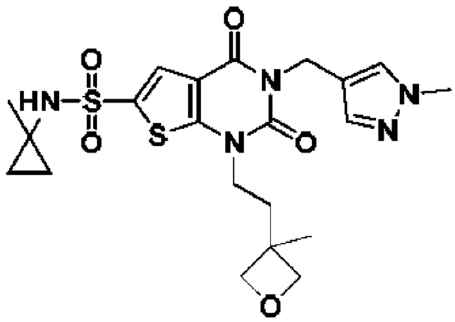 |

[Table 48]

| Ex | Syn | Chemical Structure |
|---|---|---|
| 121 | 108 | 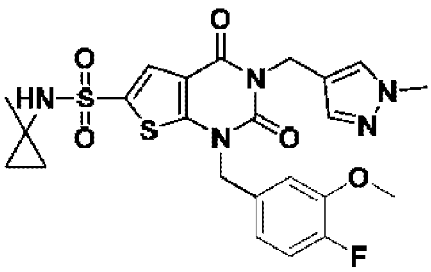 |

(continued)

| Ex | Syn | Chemical Structure |
|---|---|---|
| 122 | 108 | 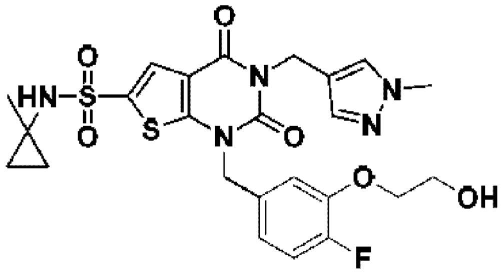 |
| 123 | 108 | 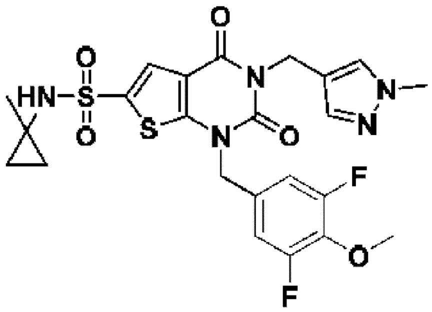 |
| 124 | 108 | 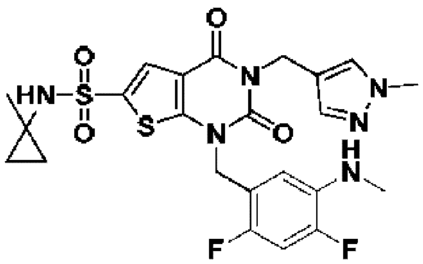 |

[Table 49]

| Ex | Syn | Chemical Structure |
|---|---|---|
| 125 | 108 | 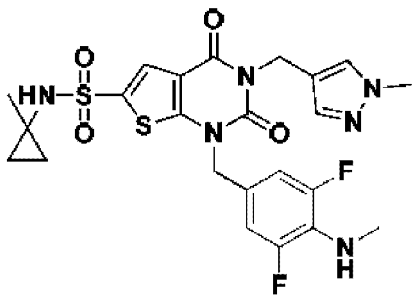 |

(continued)

| Ex | Syn | Chemical Structure |
|---|---|---|
| 126 | 108 | 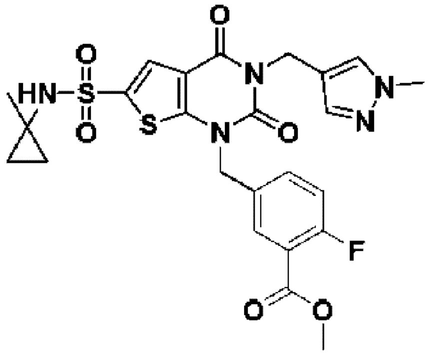 |
| 127 | 108 | 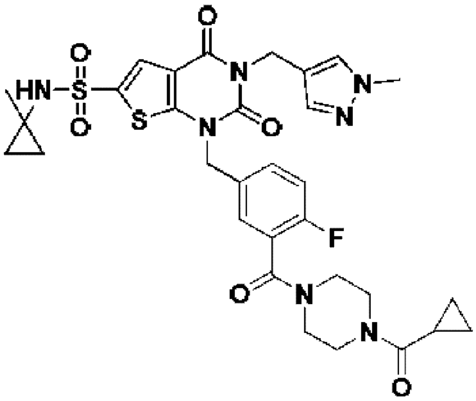 |
| 128 | 108 | 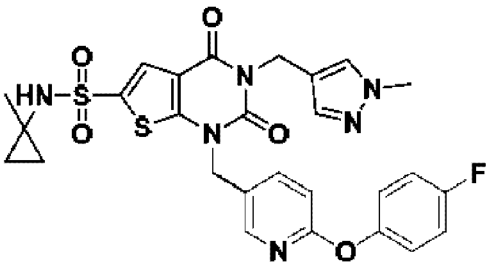 |

[Table 50]

| Ex | Syn | Chemical Structure |
|---|---|---|
| 129 | 108 | 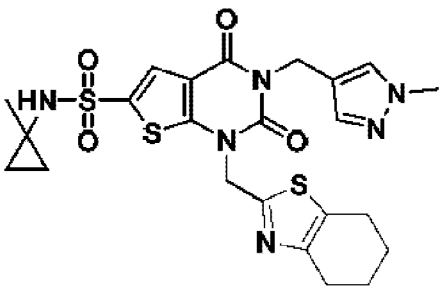 |

(continued)

| Ex | Syn | Chemical Structure |
|---|---|---|
| 130 | 130 | 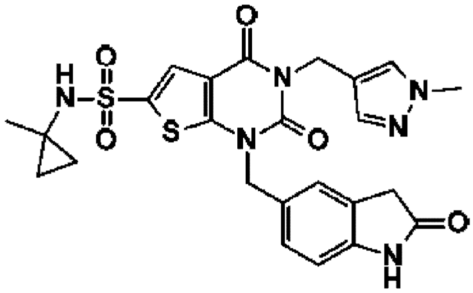 |
| 131 | 130 | 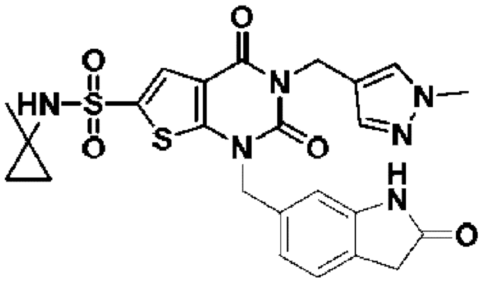 |
| 132 | 130 | 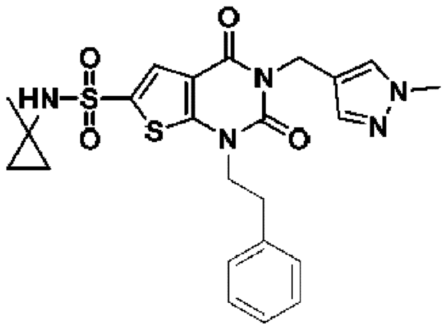 |

[Table 51]

| Ex | Syn | Chemical Structure |
|---|---|---|
| 133 | 130 | 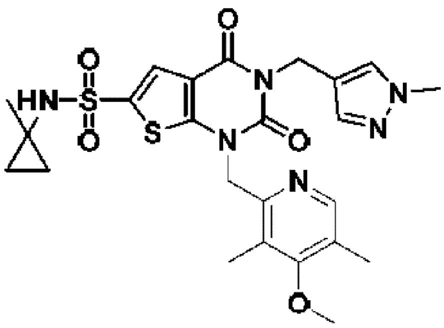 |

(continued)

| Ex | Syn | Chemical Structure |
|---|---|---|
| 134 | 130 | 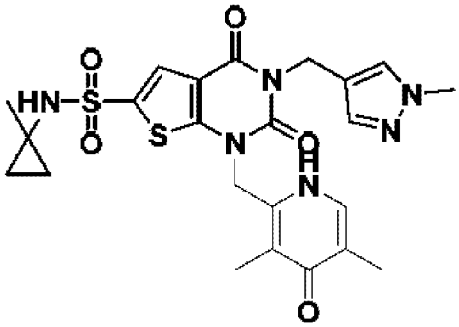 |
| 135 | 130 | 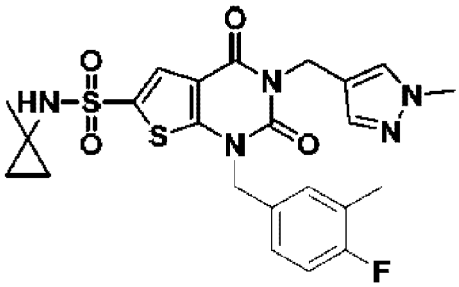 |
| 136 | 136 | 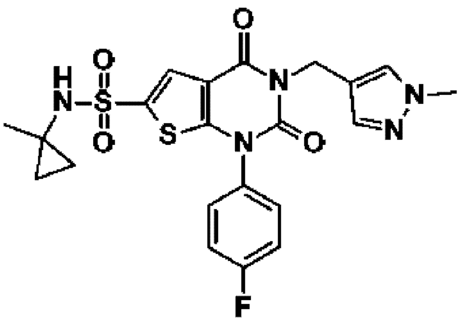 |

[Table 52]

| Ex | Data |
|---|---|
| 1 | ESI+: 413.0<br>NMR: δ 12.57(brs,1H), 8.34(brs,1H), 7.56(s,1H), 7.50(s,1H), 5.10(s,2H), 2.56(s,3H), 1.16(s,3H), 0.70(m, 2H), 0.44(m,2H) |
| 2 | ESI+: 467.0<br>NMR: δ 8.47(s,1H), 7.64(s,1H), 7.59(s,1H), 5.17(s,2H), 3.88(d, J=6.88Hz,2H), 2.56(s,3H), 1.23(m, 1H), 1.18(s,3H), 0.71(m,2H), 0.55(m,2H), 0.48(m, 4H) |
| 3 | ESI+: 521.2<br>NMR: δ 8.45 (s, 1H), 7.61-7.60 (m, 2H), 6.03 (s, 1H), 5.24 (s, 2H), 5.18 (s, 2H), 3.77 (s, 3H), 3.47-3.42 (m, 1H), 2.57 (s, 3H), 2.05 (s, 3H), 1.15 (s, 3H), 1.07 (m, 1H), 0.69 (m, 2H), 0.44 (m, 2H) |
| 4 | ESI+: 543.1<br>NMR: δ 13.13 (s, 1H), 8.38 (s, 1H), 8.06 (s, 1H), 7.83 (s, 1H), 7.63 (s, 1H), 7.58 (s, 1H), 7.55 (d, *J* = 8.64 Hz, 1H), 7.37 (d, *J* = 8.76 Hz, 1H), 5.31 (s, 2H), 5.23 (s, 2H), 2.59 (s, 3H), 1.08 (s, 3H), 0.62-0.59 (m, 2H), 0.38-0.35 (m, 3H) |

(continued)

| Ex | Data |
|---|---|
| 5 | ESI+: 543.2<br>NMR: δ 13.09 (s, 1H), 8.29 (brs, 1H), 8.06 (s, 1H), 7.77 (d, J= 8.36 Hz, 1H), 7.63 (s, 1H), 7.59 (d, *J* = 5.76 Hz, 2H), 7.12 (d, *J* = 8.52 Hz, 2H), 5.35 (s, 2H), 5.23 (s, 2H), 2.59 (s, 3H), 1.08 (s, 3H), 0.63 (m, 2H), 0.38 (m, 2H) |
| 5 | ESI+: 521.1<br>NMR: δ 8.50(s,1H), 7.61-7.60(m,2H), 5.28(s,2H), 5.18(s,2H), 2.57(s,3H), 2.50(s,3H), 2.43(s,3H), 1.10(s, 3H), 0.69-0.68(m,2H), 0.46(s,2H) |
| 7 | ESI+: 512.2<br>NMR: δ 7.59 (s, 2H), 5.16 (s, 2H), 4.25-4.19 (m, 1H), 4.09 (m, 2H), 3.65 (m, 2H), 3.37 (s, 2H), 3.21 (m, 2H), 2.56 (s, 3H), 1.17 (s, 3H), 0.71 (m, 2H), 0.47 (m, 2H) |

[Table 53]

| Ex | Data |
|---|---|
| 8 | ESI+: 496.0<br>NMR: δ 7.59 (s, 1H), 7.55 (brs, 1H), 5.15 (s, 2H), 3.95 (t, J= 7.04 Hz, 2H), 2.56 (s, 3H), 2.28 (m, 2H), 2.08 (s, 6H), 1.84-1.79 (m, 2H), 1.18 (s, 3H), 0.69 (m, 2H), 0.45 (m, 2H) |
| 9 | ESI+: 469.8<br>NMR: δ 7.60 (s, 1H), 7.58 (s, 1H), 6.78 (brs, 1H), 5.15 (s, 2H), 3.99 (m, 1H), 3.93 (m, 1H), 3.05-3.01 (m, 1H), 2.62 (s, 1H), 2.56 (s, 3H), 1.83 (m, 1H), 1.78 (m, 1H), 1.19 (s, 3H), 0.72 (t, *J* = 6.32 Hz, 2H), 0.48 (t, *J* = 4.76 Hz, 2H) |
| 10 | ESI+: 543.1<br>NMR: δ 11.54 (s, 1H), 8.38 (brs, 1H), 8.31 (d, *J* = 2.08 Hz, 1H), 8.01 (d, *J* = 1.88 Hz, 1H), 7.63 (s, 1H), 7.58 (s, 1H), 7.50 (t, *J* = 2.88 Hz, 1H), 6.43-6.42 (m, 1H), 5.31 (s, 2H), 5.22 (s, 2H), 2.58 (s, 3H), 1.09 (s, 3H), 0.64 (t, J = 6.32 Hz, 2H), 0.38 (t, *J* = 5.08 Hz, 2H) |
| 11 | ESI+: 542.1<br>NMR: δ 11.14 (s, 1H), 8.39 (s, 1H), 7.63 (s, 1H), 7.58 (s, 1H), 7.53 (d, *J* = 8.08 Hz, 1H), 7.44 (s, 1H), 7.36 (t, *J*= 2.72 Hz, 1H), 7.02 (dd, J= 8.08 Hz, 1H), 6.40 (s, 1H), 5.29 (s, 2H), 5.23 (s, 2H), 2.59 (s, 3H), 1.08 (s, 3H), 0.63 (t, *J* = 6.20 Hz, 2H), 0.39 (t, *J* = 4.68 Hz, 2H) |
| 12 | ESI+: 497.0<br>NMR: δ 8.37 (brs, 1H), 7.59 (s, 1H), 5.57 (s, 1H), 5.16 (s, 2H), 4.24-4.13 (m, 2H), 3.77-3.71 (m, 4H), 3.64-3.58 (m, 1H), 2.56 (s, 3H), 2.05-1.97 (m, 1H), 1.94-1.97 (m, 2H), 1.72-1.63 (m, 1H), 1.17 (s, 3H), 0.71 (t, *J*= 6.4 Hz, 2H), 0.47 (t, *J* = 4.76 Hz, 2H) |
| 13 | ESI+: 545.2<br>NMR: δ 8.41 (brs, 1H), 7.62 (s, 1H), 7.59 (s, 1H), 7.21 (d, *J* = 7.60 Hz, 1H), 6.83 (d, *J* = 8.40 Hz, 1H) 6.78 (s, 1H), 5.20 (s, 2H), 5.13 (s, 2H), 4.50 (m, 2H), 3.13 (m, 2H), 2.58 (s, 3H), 1.12 9s, 3H), 0.64 (m, 2H) and 0.43 (m, 2H) |

[Table 54]

| Ex | Data |
|---|---|
| 14 | ESI+: 511.0<br>NMR: δ 8.45 (s, 1H), 7.60 (d, *J*= 5.56 Hz, 1H), 5.15 (s, 2H), 3.84 (d, *J*= 7.4 Hz, 4H), 3.26 (t, *J*= 11.16 Hz, 2H), 2.56 (s, 3H), 2.10-2.09 (m, 1H), 1.58 (d, *J*= 11.84 Hz, 2H), 1.36-1.30 (m, 2H), 1.24 (s, 3H), 0.87 (t, *J*= 14.04 Hz, 1H), 0.72 (t, *J*= 5.64 Hz, 2H), 0.48 (t, *J*= 6.16 Hz, 2H) |

(continued)

| Ex | Data |
|---|---|
| 15 | ESI+: 497.0<br>NMR: δ 8.37 (brs, 1H), 7.59 (s, 1H), 5.57 (s, 1H), 5.16 (s, 2H), 4.24-4.13 (m, 2H), 3.77-3.71 (m, 4H), 3.64-3.58 (m, 1H), 2.56 (s, 3H), 2.05-1.97 (m, 1H), 1.94-1.97 (m, 2H), 1.72-1.63 (m, 1H), 1.17 (s, 3H), 0.71 (t, *J*= 6.4 Hz, 2H), and 0.47 (t, *J* = 4.76 Hz, 2H) |
| 16 | ESI+: 497.1<br>NMR: δ 8.26 (brs, 1H), 7.61 (s, 1H), 7.59 (s, 1H), 5.16 (s, 2H), 3.98-3.87 (m, 2H), 3.84-3.78 (m, 1H), 3.68-3.60 (m, 2H), 3.53-3.50 (m, 1H), 2.74 (t, *J*= 5.64 Hz, 1H), 2.57 (s, 3H), 2.02-1.94 (m, 1H), 1.71-1.63 (m, 1H), 1.19 (s, 3H), 0.72 (t, *J*= 6.24 Hz, 2H), and 0.48 (t, *J*= 4.76 Hz, 2H) |
| 17 | ESI+: 510.8<br>NMR: δ 8.44 (brs, 1H), 7.60 (d, *J* = 6.6 Hz, 2H), 5.15 (s, 2H), 3.87-3.67 (m, 4H), 3.26 (t, *J*= 8.96 Hz, 2H), 3.17 (d, *J* = 5.24 Hz, 1H), 2.56 (s, 3H), 2.08 (brs, 1H), 1.78-1.76 (m, 1H), 1.63-1.60 (m, 1H), 1.46-1.32 (m, 2H), 1.18 (s, 3H), 0.72 (t, *J* = 5.96 Hz, 2H), and 0.48 (t, *J*= 4.68 Hz, 2H) |
| 18 | ESI+: 543.1<br>NMR: δ 12.60 (s, 1H), 8.41 (s, 1H), 7.63 (d, *J* = 6.48 Hz, 2H), 7.52 (brs, 2H), 7.17 (s, 2H), 5.44 (s, 2H), 5.22 (s, 2H), 2.57 (s, 3H), 1.25 (s, 1H), 1.16 (s, 3H), 0.87 (t, *J*= 6.84 Hz, 1H), 0.65 (t, *J*= 5.64 Hz, 2H), and 0.39 (t, *J*= 4.8 Hz, 2H); LCMS: 543.08 $(M+H)^+$ |
| 19 | ESI+: 548.8<br>NMR: δ 8.30 (brs, 1H), 7.60 (d, *J*= 3.04 Hz, 2H), 6.07 (s, 1H), 5.18 (s, 2H), 5.09 (s, 2H), 4.73 (s, 2H), 4.01 (s, 4H), 2.57 (s, 3H), 1.15 (s, 3H), 0.69 (t, *J*= 5.52 Hz, 2H), and 0.46 (t, *J*= 4.76 Hz, 2H) |

[Table 55]

| Ex | Data |
|---|---|
| 20 | ESI+: 548.1<br>NMR: δ 8.48 (s, 1H), 7.62 (d, *J*= 13.36 Hz, 2H), 5.28 (s, 2H), 5.17 (s, 2H), 2.57 (s, 3H), 2.36 (s, 4H), 1.76 (d, *J*= 5.08 Hz, 2H), 1.69 (d, *J*= 4.16 Hz, 2H), 1.16 (s, 3H), 0.68 (s, 2H), and 0.45 (s, 2H) |
| 21 | ESI+: 441.1<br>NMR: δ 8.02 (t, *J*= 5.52 Hz, 1H), 7.66 (s, 1H), 7.59 (s, 1H), 5.18 (s, 2H), 3.88 (d, *J* = 6.96 Hz, 2H), 2.95-2.88 (m, 2H), 2.57 (s, 3H), 1.29-1.20 (m, 1H), 1.06 t, *J*= 7.2 Hz, 3H), 0.57 (d, *J*= 5.44 Hz, 2H) and 0.51 (t, *J* = 4.76 Hz, 2H) |
| 22 | ESI+: 455.1<br>NMR: δ 8.05 (d, *J*= 7.20 Hz, 1H), 7.65 (s, 1H), 7.59 (s, 1H), 5.17 (s, 2H), 3.88 (d, *J*= 7.04 Hz, 2H), 3.42-3.36 (m, 1H), 2.56 (s, 3H), 1.23 (s, 1H), 1.04 (s, 6H), 0.56 (m, 2H), 0.45 and (m 2H) |
| 23 | ESI+: 427.1<br>NMR: δ 7.90 (brs, 1H), 7.65 (s, 1H), 7.59 (s, 1H), 5.18 (s, 2H), 3.88 (d, *J*= 7.0 Hz, 2H), 2.56 (s, 3H), 2.49 (s, 3H), 1.24 (s, 1H), 0.55 (t, *J*= 8.04 Hz, 2H) and 0.47 (t, *J*= 4.72 Hz, 2H) |
| 24 | ESI+: 453.1<br>NMR: δ 8.34 (d, *J* = 2.68 Hz, 1H), 7.66 (s, 1H), 7.59 (s, 1H), 5.18 (s, 2H), 3.88 (d, *J* = 7.0 Hz, 2H), 2.57 (s, 3H), 2.29 (m, 1H), 1.24 (m, 1H), 0.59-0.52 (m, 4H) and 0.47-.45 (m, 4H) |
| 25 | ESI+: 521.1<br>NMR: δ 8.48(s,1H), 7.66(s,1H), 7.59(s,1H), 7.37(s,1H), 5.27(s,2H), 4.88(s,2H), 3.76(s,3H), 2.52(m,3H), 2.49(m,3H), 1.15(s,3H), 0.66(s,2H) and 0.44(s,2H) |
| 26 | ESI+: 450.1<br>NMR: δ 8.40 (brs, 1H), 7.65 (s, 1H), 7.60 (s, 1H), 7.36 (s, 1H), 4.87 (s, 2H), 3.87 (m, 2H), 3.75 (s, 3H), 1.24 (m, 1H), 1.18 (s, 3H), 0.72 (m, 2H), 0.56 (m, 2H) and 0.48 (m, 4H) |

[Table 56]

| Ex | Data |
|---|---|
| 27 | ESI+: 448.1<br>NMR: δ 8.48 (brs, 1H), 7.65 (s, 1H), 7.62 (s, 1H), 7.36 (s, 1H), 4.86 (s, 2H), 4.81 (s, 2H), 3.75 (s, 3H), 1.81 (s, 3H), 1.18 (s, 3H), 0.72 (m, 2H), and 0.46 (m, 2H) |
| 28 | ESI+: 528.2<br>NMR: δ 8.33(s,1H), 7.67(s,1H), 7.58(s,1H), 7.38(s,1H), 7.30(m,3H), 5.22(s,2H), 4.96-4.92(m,6H), 3.76(s, 3H), 1.26(s,3H), 0.65(s,2H) and 0.41(s,2H) |
| 29 | ESI+: 490.1<br>NMR: δ 8.43(s,1H), 7.78(s,1H), 7.66(s,1H), 7.57(s,1H), 7.46(s,1H), 7.37(s,1H), 4.99(s,2H), 4.91(s,2H), 3.78(s,3H), 3.75(s,3H), 1.57(s,3H), 0.68(s,2H) and 0.46(s,2H) |
| 30 | ESI+: 503.8<br>NMR: δ 8.35 (brs, 1H), 7.65 (s, 1H), 7.60 (s, 1H), 7.36 (s, 1H), 5.99 (s, 1H), 5.23 (s, 2H), 4.88 (s, 2H), 3.76 (m, 6H), 2.05 (s, 3H), 1.15 (s, 3H), 0.69 (m, 2H), and 0.45 (m, 2H) |
| 31 | ESI+: 490.1<br>NMR: δ 12.48 (brs, 1H), 8.33 (brs, 1H), 7.66 (s, 1H), 7.56 (s, 1H), 7.37 (s, 1H), 5.97 (s, 1H), 5.05 (s, 2H), 4.89 (s, 2H), 3.75 (m, 3H), 2.17 (s, 3H), 1.15 (s, 3H), 0.69 (m, 2H), and 0.45 (m, 2H) |
| 32 | ESI+: 504.2<br>NMR: δ 8.42 (brs, 1H), 8.20 (s, 1H), 7.86 (s, 1H), 7.51 (s, 1H), 7.28 (s, 1H), 5.46 (s, 2H), 4.85 (s, 2H), 4.05 (s, 3H), 3.75 (m, 3H), 2.05 (s, 3H), 1.14 (s, 3H), 0.69 (m, 2H), and 0.45 (m, 2H) |
| 33 | ESI+: 532.2<br>NMR: δ 8.42 (brs, 1H), 7.65 (s, 1H), 7.58 (s, 1H), 7.37 (s, 1H), 6.07 (s, 1H), 5.09 (s, 2H), 4.88 (s, 2H), 4.73 (s, 2H), 4.01 (m, 4H), 3.75 (s, 3H), 1.15 (s, 3H), 0.69 (m, 2H), and 0.45 (m, 2H) |

[Table 57]

| Ex | Data |
|---|---|
| 34 | ESI+: 488.1<br>NMR: δ 9.15 (s, 1H), 8.88 (s, 2H), 8.43 (s, 1H), 7.66 (s, 1H), 7.60 (s, 1H), 7.37 (s, 1H), 5.25 (s, 2H), 4.88 (s, 2H), 3.76 (s, 3H), 1.14 (s, 3H), 0.68-065 (m, 2H) and 0.44-0.41 (m, 2H) |
| 35 | ESI+: 518.2<br>NMR: δ 8.46(brs,1H), 8.36(s,1H), 7.66(s,1H), 7.60(s,1H), 7.38(s,1H), 5.49(s,2H), 4.88(s,2H), 3.76(s,3H), 1.17(s,3H), 0.69(s,2H) and 0.47(s,2H) |
| 36 | ESI+: 525.8<br>NMR: δ 13.11 (s, 1H), 8.39 (brs, 1H), 8.06 (s, 1H), 7.76 (d, *J* = 8.36 Hz, 1H), 7.69 (s, 1H), 7.57 (s, 1H), 7.53 (brs, 1H), 7.40 (s, 1H), 7.10 (d, *J* = 8.4 Hz, 1H), 5.34 (s, 2H), 4.94 (s, 2H), 3.77 (s, 3H), 1.08 (s, 3H), 0.58 (s, 2H) and 0.33 (s, 2H) |
| 37 | ESI+: 525.8<br>NMR: δ 11.74 (s, 1H), 8.37 (brs, 1H), 8.30 (d, *J* = 2.08 Hz, 1H), 8.00 (d, *J* = 1.8 Hz, 1H), 7.69 (s, 1H), 7.57 (s, 1H), 7.50-7.48 (m, 1H), 7.40 (s, 1H), 6.43-6.42 (m, 1H), 5.30 (s, 2H), 4.92 (s, 2H), 3.76 (s, 3H), 1.09 (s, 3H), 0.64-0.61 (m, 2H) and 0.40-0.37 (m, 2H) |
| 38 | ESI+: 526.1<br>NMR: δ 13.14 (brs, 1H), 8.06 (s, 1H), 7.81 (s, 1H), 7.69 (s, 1H), 7.56-7.53 (m, 2H), 7.40 (s, 1H), 7.36-7.33 (dd, *J* = 8.68 Hz, 1H), 5.30 (s, 2H), 4.93 (s, 2H), 3.77 (s, 3H), 1.07 (s, 3H), 0.62-0.59 (m, 2H) and 0.38-0.35 (m, 2H) |
| 39 | ESI+: 543.2<br>NMR: δ 11.72(s,1H), 8.41(s,1H), 7.68(s,1H), 7.58(s,1H), 7.39(s,1H), 7.36(s,1H), 7.19(d,J=8.0Hz,1H), 7.09 (d,J= 8.0Hz,1H), 5.20(s,2H), 4.91(s,2H), 3.76(s,3H), 1.28(s,3H), 0.65(s,2H) and 0.42(s,2H) |

[Table 58]

| Ex | Data |
|---|---|
| 40 | ESI+: 526.2<br>NMR: δ 8.48(s,1H), 8.35(s,1H), 7.68(s,1H), 7.60(s,1H), 7.57(m,2H), 7.39(s,1H), 7.35(s,1H), 6.74(d, J=10.0Hz,1H), 5.14(s,2H), 4.92(s,2H), 3.76(s,3H), 1.14(s,3H), 0.65(t,J=6.0Hz,2H) and 0.39(m,2H) |
| 41 | ESI-: 529.3<br>NMR: δ 8.43(m,2H), 7.64(s,1H), 7.59(s,1H), 7.48(s,1H), 7.35(s,1H), 5.29(s,2H), 5.01(m,4H), 4.89(s,2H), 3.75(s,3H), 1.13(s,3H), 0.65(s,2H) and 0.42(s,2H).<br>LCMS: 529.27$(M-H)^-$. |
| 42 | ESI+: 517.3<br>NMR: δ 8.42(s,1H), 8.26(d,2.40Hz,1H), 8.22(s,1H), 7.67(s,1H), 7.59(s,1H), 7.38(d,6.40Hz,2H), 5.23(s,2H), 4.91(s,2H), 3.79(m,3H), 3.32(m,3H), 1.12(s,3H), 0.65(s,2H) and 0.41(s,2H) |
| 43 | ESI+: 517.2<br>NMR: δ 8.41(s,1H), 8.14(m,1H), 7.67(s,1H), 7.61(d,J=2Hz,1H), 7.37(s,1H), 6.95(m,1H), 6.81(s,1H), 5.19 (s,2H), 4.90(s,2H), 3.82(s,3H), 3.76(s,3H), 1.12(s,3H), 0.65(s,2H) and 0.43(m,2H) |
| 44 | ESI+: 517.2<br>NMR: δ 8.41(s,1H), 8.26(s,1H), 7.73(m,1H), 7.67(s,1H), 7.58(s,1H), 7.38(s,1H), 6.83(d,J=7.6Hz,1H), 5.15 (s,2H), 4.90(s,2H), 3.83(s,3H), 3.76(s,3H), 1.13(s,3H), 0.65(s,2H) and 0.42(s,2H) |
| 45 | ESI+: 555.1<br>NMR: δ 8.39(s,1H), 7.68(m,2H), 7.60(d,J=10.0Hz,2H), 7.49(d,J=8.0Hz,1H), 7.39(s,1H), 5.31(s,2H), 4.92 (s,2H), 4.42(s,2H), 3.77(s,3H), 3.04(s,3H), 1.08(s,3H), 0.63(s,2H) and 0.40(s,2H) |
| 46 | ESI+: 503.8<br>NMR: δ 8.41 (s, 1H), 7.67 (s, 1H), 7.58 (s, 1H), 7.46-7.42 (m, 2H), 7.38 (s, 1H), 7.23-7.19 (m, 2H), 5.19 (s, 2H), 4.91 (s, 2H), 3.76 (s, 3H), 1.11 (s, 3H), 0.65-0.63 (m, 2H) and 0.42-0.40 (m, 2H) |

[Table 59]

| Ex | Data |
|---|---|
| 47 | ESI+: 538.2<br>NMR: δ 8.41(s,1H), 7.67(s,1H), 7.62-7.58(m,2H), 7.52(d,J=10.40Hz,1H), 7.38(s,1H), 7.27(d,J=8.0Hz,1H), 5.22(s,2H), 4.90(s,2H), 3.76(s,3H), 1.12(s,3H), 0.64(s,2H) and 0.43-0.40(m,2H) |
| 48 | ESI+: 538.2<br>NMR: δ 8.42(s,1H), 7.69(m2H), 7.59(s,1H), 7.43(m,2H), 7.38(s,1H), 5.20(s,2H), 4.90(s,2H), 3.76(s,3H), 1.22 (s,3H), 0.66-0.65(m,2H) and 0.43-0.40(m,2H) |
| 49 | ESI+: 526.3<br>NMR: δ 8.40(s,1H) 7.67(s,1H), 7.58(s,1H), 7.38(s,1H), 7.25(d,J=7.6Hz,2H), 7.07(d,J=8Hz,2H), 5.14(s,2H), 4.91(s,2H), 3.76(s,3H), 1.98(m,1H), 1.08(s,3H), 0.95(d,J=8Hz,2H), 0.64(s,4H) and 0.40(s,2H) |
| 50 | ESI+: 518.2<br>NMR: δ 8.41(s,1H), 7.07(s,1H), 7.57(s,1H), 7.38(s,1H), 7.33(d,J=8.4,2H), 6.94(m,2H), 5.13(s,2H), 4.91(s, 2H), 3.68(s,3H), 3.72(s,3H), 1.2(s,3H), 0.64(s,2H) and 0.41(s,2H) |
| 51 | ESI+: 552.3<br>NMR: δ 8.41(s,1H), 7.67(s,1H), 7.59(s,1H), 7.46(m,2H), 7.41-7.05(m,4H), 5.13(s,2H), 4.91(s,2H), 3.76(s, 3H), 1.2(s,3H), 0.64(s,2H) and 0.42(s,2H) |
| 52 | ESI+: 570.2<br>NMR: δ 8.41(s,1H), 7.67(s,1H), 7.59(s,1H), 7.53(d,J=8.4Hz,2H), 7.38-7.37(m,3H), 5.24(s,2H), 4.91(s,2H), 3.76(s,3H), 1.2(s,3H), 0.63(s,2H) and 0.39(s,2H) |

(continued)

| Ex | Data |
|---|---|
| 53 | ESI+: 511.1<br>NMR: δ 8.40(s,1H), 7.83(d,J=8.0Hz,2H), 7.64(d,J=10.0Hz,2H), 7.55(d,J=7.60Hz,2H), 7.37(s,1H), 5.28(s, 2H), 4.89(s,2H), 3.74(s,3H), 1.01(s,3H), 0.62(s,2H) and 0.40(s,2H) |

[Table 60]

| Ex | Data |
|---|---|
| 54 | ESI+: 517.3<br>NMR: δ 8.40(s,1H), 8.3(s,2H), 7.6(s,1H), 7.58(s,1H), 7.38(s,1H), 7.27-7.23(m,1H), 4.99(s,2H), 4.8(s,2H), 3.75(s,3H), 2.7(m,3H), 1.15(s,3H), 0.67(s,2H) and 0.45(s,2H) |
| 55 | ESI+: 545.2<br>NMR: δ 8.41(brs,1H), 7.62(d,J=10.4Hz,2H), 7.21(d,J=7.6Hz,1H), 6.83(d,J=7.60Hz,1H), 6.78 (s,1H), 5.20(s, 2H), 5.13(s,2H), 4.52(t,J=8.80Hz,2H), 3.16(t,J=8.80Hz,2H), 2.58(s,3H), 1.12(s,3H), 0.66(t,J=6.4Hz,2H) and 0.43(t,J=4.80,2H) |
| 56 | ESI+: 540.2<br>NMR: δ 8.44(s,1H), 7.90(s,1H), 7.66(s,1H), 7.58(s,2H), 7.38(s,1H), 6.45-6.15(m,1H), 5.02(s,2H), 4.89(s, 2H), 4.63-4.54(m,2H), 3.75(s,3H), 1.14(s,3H), 0.67(s,2H) and 0.44(s,2H) |
| 57 | ESI+: 520.2<br>NMR: δ 8.04(brs,1H), 7.80(s,1H), 7.66(s, 1H), 7.58(s,1H), 7.49(s,1H), 7.38(s,1H), 4.99(s,2H), 4.89(s,2H), 4.83(m,1H), 4.09(m,2H), 3.75(s,3H), 3.67(m,2H), 1.15(s,3H), 0.68(s,2H) and 0.44(s,2H) |
| 58 | ESI+: 537.2<br>NMR: δ 8.45(s,1H), 7.65(d,J=8.8Hz,2H), 7.60(s,1H), 7.36(s,1H), 5.46(s,2H), 4.89(s,2H), 4.59(s,2H), 3.75 (s,3H), 3.25(s,3H), 1.15(s,3H), 0.67(s,2H) and 0.45(s,2H) |
| 59 | ESI+: 528.1<br>NMR: δ 8.25(brs,1H), 7.67(s,1H), 7.57(s,1H), 7.38(s,1H), 7.23(s,1H), 7.14(d,J=8.4Hz,1H), 6.75(d,J=8.01Hz, 1H), 5.10(s,2H), 4.91(s,2H), 4.52(t,J=8.8Hz,2H), 3.76(s,3H), 3.15(t,J=8.8Hz,2H), 1.12(s,3H), 0.65(s,2H) and 0.42(s,2H) |
| 60 | ESI+: 541.3<br>NMR: δ 8.56(s,1H), 8.39(s,1H), 7.70(m,2H), 7.60(s,1H), 7.55(s,1H), 7.50(d,J=8.0Hz,1H), 7.39(s,1H), 5.28 (s,2H), 4.91(s,2H), 4.32(s,2H), 3.72(s,3H), 1.05(s,3H), 0.62(s,2H) and 0.40(s,2H) |

[Table 61]

| Ex | Data |
|---|---|
| 61 | ESI+: 541.1<br>NMR: δ 8.60(s,1H), 8.40(s,1H), 7.58(s,2H), 7.62(m,3H), 7.39(s,1H), 5.31(s,2H), 4.92(s,2H), 4.35(s,2H), 3.77(s,3H), 1.15(s,3H), 0.64(s,2H) and 0.40(s,2H) |
| 62 | ESI+: 555.1<br>NMR: δ 8.40(s,1H), 7.68(d,J=3.2Hz,2H), 7.58(s,3H), 7.39(s,1H), 5.30(s,2H), 4.92(s,2H), 4.44(s,2H), 3.77 (s,3H), 3.05(s,3H), 1.15(s,3H), 0.64(s,2H) and 0.41(s,2H) |
| 63 | ESI+: 570.1<br>NMR: δ 8.37(s,1H), 7.69(s,1H), 7.65(s,1H), 7.57(d,J=6.8Hz,2H), 7.40(s,1H), 7.34(d,J=8.8Hz,1H), 6.89(s, 1H), 5.29(s,2H), 4.93(s,2H), 4.51(s,2H), 3.32-3.29(m,3H), 3.23(s,3H), 1.08(s,3H), 0.63(s,2H) and 0.39(s, 2H) |

(continued)

| Ex | Data |
|---|---|
| 64 | ESI+: 556.1<br>NMR: δ 8.37(brs,1H), 7.69(s,1H), 7.62(s,1H), 7.57(s,1H), 7.55(d,J=8Hz,1H), 7.40(s,1H), 7.30(d,J=8.0Hz, 1H), 6.73(s,1H), 5.48(t,J = 4.0Hz,1H), 5.28(s,2H), 4.93(s,2H), 4.55(d,J=5.6Hz,2H), 3.77(s,3H), 1.09(s,3H), 0.62(s,2H) and 0.38(s,2H) |
| 65 | ESI+: 545<br>NMR: δ 8.41(brs,1H), 7.83(s,1H), 7.67(s,1H), 7.58(s,1H), 7.38(s,1H), 7.31(s,1H), 5.12(s,2H), 4.89(s,2H), 4.38(s,2H), 4.22(s,2H), 3.76(s,3H), 1.14(s,3H), 0.66(s,2H) and 0.43(s,2H) |
| 66 | ESI+: 527.1<br>NMR: δ 7.67(s,1H), 7.55(s,1H), 7.38(s,1H), 7.38(s,1H), 7.28-7.17(m,3H), 5.18(s,2H), 4.91(s,2H), 4.48(s, 1H), 4.00(s,1H), 3.76(s,3H), 1.16(s,3H), 0.62(s,2H) and 0.40(s,2H) |
| 67 | ESI+: 541.1<br>NMR: δ 8.40(s,1H), 7.67(s,1H), 7.58(s,1H), 7.38(s,1H), 7.22-7.20(m,3H), 5.17(s,2H), 4.91(s,2H), 3.76(s, 8H), 2.50-2.46(m,3H), 1.14(s,3H), 0.64(s,2H) and 0.43(s,2H) |

[Table 62]

| Ex | Data |
|---|---|
| 68 | ESI-: 540.1<br>NMR: δ 8.39(s,1H), 7.67(s,1H), 7.58(s,1H), 7.38(s,1H), 7.33(d,J=8.40Hz,2H), 7.04(d,J=8.4Hz,2H), 5.13(s, 2H), 4.91(s,2H), 3.83-3.79(m,1H), 3.76(s,3H), 1.12(s,3H), 0.78-0.73(m,2H), 0.66-0.59(m,4H) and 0.41(s, 2H) |
| 69 | ESI+: 556.3<br>NMR: δ 8.41(s,1H), 7.67(s,1H), 7.57(s,1H), 7.38(s,1H), 7.30(d,J=8.8Hz,2H), 6.83(d,J=8.4Hz,2H), 5.11(s, 2H), 4.90(s,2H), 4.68-4.61(m,1H), 3.76(s,3H), 2.42-2.38(m,2H), 2.01-1.94(m,2H), 1.76-1.58(m,2H), 1.11 (s,3H), 0.63(s,2H) and 0.41(s,2H) |
| 70 | ESI+: 529.1<br>NMR: δ 8.37(s,1H), 8.01(d,J=4.4Hz,1H), 7.85-7.82(m,1H), 7.67(s,1H), 7.60-7.53(m,2H), 7.38(s,1H), 5.23 (s,2H), 4.90(s,2H), 3.76(s,3H), 1.12(s,5H), 0.65(s,2H) and 0.42(s,2H) |
| 71 | ESI+: 547.3<br>NMR: δ 8.39(s,1H), 7.67(s,1H), 7.57(s,1H), 7.38(s,1H), 7.17-7.13(m,1H), 7.10(d,J = 8.8Hz, 1H), 6.94(t, J=8.8Hz,1H), 5.11(s,2H), 4.91(s,2H), 3.76(s,3H), 2.75(s,6H), 1.12(s,3H), 0.64(s,2H) and 0.41(s,2H) |
| 72 | ESI+: 561.2<br>NMR: δ 8.40(s,1H), 7.67(s,1H), 7.57(s,1H), 7.38(s,1H), 7.16-7.12(m,1H), 7.09-7.07(m,1H), 6.93(m,1H), 5.10(s,2H), 4.91(s,2H), 3.76(s,3H), 3.17-3.11(m,3H), 1.11(s,3H), 1.02(m3H), 0.64(s,2H) and 0.40(s,2H) |
| 73 | ESI+: 533.1<br>NMR: δ 8.40(s,1H), 7.67(s,1H), 7.57(s,1H), 7.38(s,1H), 7.08-7.03(m,2H), 6.63(t,J=8.8Hz,1H), 5.66(s,1H), 5.05(s,2H), 4.91(s,2H), 3.76(s,3H), 2.69(d,J=4.8Hz,3H), 1.10(s,3H), 0.65(s,2H) and 0.43-0.40(m,2H) |
| 74 | ESI+: 573.1<br>NMR: δ 8.35(brs,1H), 7.67(s,1H), 7.58(s,1H), 7.38(s,1H), 7.42(t,J=8.4Hz,1H), 7.16-7.09(m,2H), 5.11(s,2H), 4.91(s,2H), 3.76(s,3H), 2.85(s,3H), 2.44(s,1H), 1.11(s,3H), 0.71-0.69(m,2H), 0.65(m,2H) and 0.42-0.34(m, 4H) |

[Table 63]

| Ex | Data |
|---|---|
| 75 | ESI+: 591.4<br>NMR: δ 8.40(s,1H), 7.67(s,1H), 7.57(s,1H), 7.38(s,1H), 7.15-7.12(m,1H), 7.08(d,J=8.40Hz,1H), 6.94(t, J=8.8Hz,1H), 5.10(s,2H), 4.91(s,2H), 3.76(s,3H), 3.46(t,J=6.0Hz,2H), 3.29(s,2H), 3.18(s,3H), 2.81(s,3H), 1.12(s,3H), 0.64(s,2H) and 0.41(s,2H) |
| 76 | ESI+: 577.2<br>NMR: δ 8.38(s,1H), 7.67(s,1H), 7.57(s,1H), 7.38(s,1H), 7.14(s,1H), 7.08(d,J=8.0Hz,1H), 6.93(t,J=9.2Hz, 1H), 5.09(s,2H), 4.91(s,2H), 4.58(m, 1H), 3.76(s,3H), 3.54-3.50(m,2H), 3.20(m,2H), 2.82(s,3H), 1.12(s,3H), 0.65(s,2H) and 0.41(s,2H) |
| 77 | ESI+: 590.2<br>NMR: δ 7.67(s,1H), 7.57(s,1H), 7.38(s,1H), 7.15-7.06(m,2H), 6.95(m,1H), 5.10(s,3H), 4.91(s,3H) 3.76(s, 3H), 3.17(m,2H), 2.70(s,3H), 2.60(m,2H), 2.23(s,3H), 1.12(s,3H),0.64(s,2H) and 0.42(s,2H) |
| 78 | ESI+: 604.0<br>NMR: δ 8.40(s,1H), 7.67(s,1H), 7.57(s,1H), 7.38(s,1H), 7.16(d,J=14.8Hz,1H), 7.08(d,J=8.4,1H), 6.93(t, J=8.8,1H), 5.10(s,2H), 4.91(s,2H), 3.76(s,3H), 3.21(t,J=6.8,2H), 2.79(s,3H), 2.3(s,2H), 2.13(s,6H), 1.12(s, 3H), 0.64(s,2H) and 0.40(s,2H) |
| 79 | ESI+: 561.2<br>NMR: δ 8.43(s,1H), 8.35(s,2H), 7.66(s,1H), 7.59(s,1H), 7.37(s,1H), 7.32(s,1H), 4.99(s,2H), 4.88(s,2H), 3.75 (s,3H), 3.40(s,4H), 3.22(s,3H), 1.15(s,3H), 0.67(s,2H) and 0.43(s,2H) |
| 80 | ESI+: 547.2<br>NMR: δ 8.44(s,1H), 8.35(s,2H), 7.66(s,1H), 7.58(s,1H), 7.37(s,1H), 7.25(brs,1H), 4.99(s,2H), 4.88(s,2H), 3.78(s,3H), 3.49-3.44(m,3H), 3.31-3.30(m,2H), 1.15(s,3H), 0.67(s,2H) and 0.45-0.42(m,2H) |
| 81 | ESI+: 572.2<br>NMR: δ 8.41(brs,1H), 8.21(s,1H), 7.67(s,1H), 7.58-7.55(m,2H), 7.38(s,1H), 6.83(d,J=8.8Hz,1H), 5.07(s, 2H), 4.90(s,2H), 3.76(s,3H), 3.67-3.65(m,4H), 3.43-3.40(m,4H), 1.13(s,3H), 0.66(s,2H) and 0.43(s,2H) |

[Table 64]

| Ex | Data |
|---|---|
| 82 | ESI+: 556.2<br>NMR: δ 8.42(brs,1H), 8.14(s,1H), 7.67(s,1H), 7.57(s,1H), 7.49-7.46(m,1H), 7.38(s,1H), 6.42(d,J=8.8Hz, 1H), 5.04(s,2H), 4.90(s,2H), 3.76(s,3H), 3.32(s,4H), 1.92(s,4H), 1.23(s,1H), 1.13(s,3H), 0.66(s,2H) and 0.43(s,2H) |
| 83 | ESI+: 572.2<br>NMR: δ 8.42(s,1H), 8.14(s,1H), 7.67(s,1H), 7.57(s,1H), 7.48(d,J=8.8Hz,1H), 7.38(s,1H), 6.42(d,J=8.8Hz, 1H), 5.04(s,2H), 4.90(s,3H), 4.35(s,1H), 3.76(s,3H), 3.45-3.40(m,3H), 3.27-3.24(m,1H), 2.00(brs,1H), 1.87 (brs,1H), 1.13(s,3H), 0.66(s,2H) and 0.42(s,2H) |
| 84 | ESI+: 586.2<br>NMR: δ 8.41(s,1H), 8.15-8.19(d,J=2.0Hz,1H), 7.67(s,1H), 7.57(s,1H), 7.50-7.47(m,1H), 7.38(s,1H), 6.44 (d,J=8.8Hz,1H), 5.05(s,2H), 4.90(s,2H), 4.04(s,1H), 3.76(s,3H), 3.44(d,J=2.8Hz,3H), 3.32(s,1H), 3.23(s, 3H), 2.01(s,2H), 1.13(s,3H), 0.66(s,2H) and 0.41(s,2H) |
| 85 | ESI+: 517<br>NMR: δ 8.42(s,1H), 7.69-7.66(m,2H), 7.61(s,1H), 7.37(s,1H), 6.33(s,1H), 6.17-6.14(m, 1H), 5.03(s,2H), 4.89(s,2H), 3.76(s,3H), 3.37(s,3H), 1.14(s,3H), 0.68-0.65(m,2H) and 0.45-0.42(m,2H) |
| 85 | ESI+: 535.2<br>NMR: δ 8.43(s,1H), 7.64(s,1H), 7.59(s,1H), 7.35(s,1H), 4.86(s,2H), 4.36(d,J=12.8Hz,1H), 3.87(s,3H), 3.75 (s,3H), 2.98(t,J=12.4Hz,1H), 2.46(s,1H), 2.13(brs,1H), 1.97(s,3H), 1.69(t,J = 16Hz,2H), 1.29-1.28(m,1H), 1.18(s,3H), 1.12-1.07(m,1H), 0.71(s,2H) and 0.46(s,2H) |

(continued)

| Ex | Data |
|---|---|
| 87 | ESI+: 521<br>NMR: δ 8.43(s,1H), 7.64(s,1H), 7.59(s,1H), 7.35(s,1H), 4.86(s,2H), 3.93-3.81(m,2H), 3.75(s,3H), 3.31-3.25 (m,2H), 2.77(s,3H), 2.35-2.31(m,2H), 2.13-2.06(m,1H), 1.88-1.85(d,J=12Hz,1H), 1.59(s,1H), 1.19(s,3H), 0.71(s,2H) and 0.46(s,2H) |

[Table 65]

| Ex | Data |
|---|---|
| 88 | ESI+: 517.1<br>NMR: δ 8.43 (s, 1H), 7.86 (s, 1H), 7.66 (s, 1H), 7.59 (s, 1H), 7.43-7.38 (m, 2H), 6.40 (d, J = 9.6 Hz, 1H), 4.93 (d, J = 14.4 Hz, 4H), 3.76 (s, 3H), 3.39 (s, 3H), 1.14 (s, 3H), 0.67 (s, 2H) and 0.43 (s, 2H) |
| 89 | ESI+: 569.1<br>NMR: δ 11.20 (s, 1H), 8.28 (brs, 1H), 7.69 (s, 1H), 7.56 (d, J = 9.2 Hz, 2H), 7.40 (s, 1H), 7.32 (d, J = 8.4 Hz, 1H), 7.08-7.06 (m, 1H), 6.35 (s, 1H), 5.24 (s, 2H), 4.94 (s, 2H), 4.49 (s, 2H), 3.77 (s, 3H), 3.26 (s, 3H), 1.08 (s, 3H), 0.63-0.60 (m, 2H) and 0.38-0.35 (m, 2H) |
| 90 | ESI+: 558<br>NMR: δ 8.49(brs,1H), 8.13[s,1H), 7.67(s,1H), 7.55(s,1H), 7.51-7.48(m,2H), 7.38(s,1H), 6.37-6.34(d, J=8.4Hz,1H), 5.01(s,2H), 4.88(s,2H), 4.54-4.51(m,1H), 4.18-4.05(m,2H), 3.71(s,3H), 3.63-3.59(m,2H), 1.11(s,3H), 0.61-0.60(s,2H) and 0.43(s,2H) |
| 91 | ESI+: 479<br>NMR: δ 8.41(s,1H), 7.66(s,1H), 7.62(s,1H), 7.39(s,1H), 5.21(s,1H), 4.97(s,2H), 3.76(s,3H), 2.95-2.92(m, 2H), 2.63-2.58(m,2H), 1.98-1.95(m,2H), 1.67-1.64(m,2H), 1.15(s,3H), 0.70(s,2H) and 0.45(s,2H) |
| 92 | ESI+: 521.2<br>NMR: δ 8.42 (s, 1H), 7.67 (s, 1H), 7.63 (s, 1H), 7.39 (s, 1H), 5.39-5.38 (m, 1H), 4.99 (s, 2H), 3.76 (s, 3H), 3.70-3.60 (m, 2H), 3.43-3.38 (m, 2H), 2.03 (s, 4H), 1.94-1.72 (m, 3H), 1.16 (s, 3H), 0.69 (s, 2H) and 0.46-0.45 (m, 2H) |
| 93 | ESI+: 535.1<br>NMR: δ 8.42(s,1H), 7.66-7.63(d,J=12Hz,2H), 7.39(s1H), 5.38(s,1H), 4.99(s,2H), 3.76(s,3H), 3.62-3.58(m, 2H), 3.43-3.38(m,2H), 2.36-2.30(m,2H), 1.99-1.74(m,4H), 1.16(s,3H), 0.99-0.95(t,1=8Hz,3H), 0.69(s,2H) and 0.43(s,2H) |

[Table 66]

| Ex | Data |
|---|---|
| 94 | ESI+: 565.2<br>NMR: δ 8.43(s,1H), 7.66(s,1H), 7.63(s,1H), 7.39(s,1H), 5.38(s,1H), 4.99(s,2H), 3.76(s,3H), 3.70-3.65(m, 2H), 3.68(m,2H), 3.45-3.43(m,2H), 3.23(s,3H), 2.62(m,2H), 2.00(m,2H), 1.83(m,2H), 1.16(s,3H), 0.69(s, 2H) and 0.45(s,2H) |
| 95 | ESI+: 564.2<br>NMR: δ 7.67(s,1H), 7.63(s,1H), 7.39(s,1H), 5.38(m,1H), 4.99(s,2H), 3.76(s,3H), 3.70-3.42(m,4H), 2.67(m, 2H), 2.46(m,2H), 2.27(s,3H), 2.10-1.74(m,4H), 1.16(s,3H), 0.69(s,2H) and 0.45(s,2H) |
| 96 | ESI+: 578.2<br>NMR: δ 7.67(d,J=12.8Hz,1H), 7.39(s,1H), 5.38(brs,1H), 4.99(s,2H), 3.76(s,3H), 3.63-3.75(m,2H), 3.42(brs, 4H), 2.50(s,3H), 2.39(t,J=12.8Hz,2H), 2.27(s,3H), 1.99-1.72(m,4H), 1.65(t,J=6.8Hz,2H), 1.16(s,3H), 0.69 (s,2H) and 0.45(s,2H) |

(continued)

| Ex | Data |
|---|---|
| 97 | ESI+: 593<br>NMR: δ 8.71(brs,1H), 8.49(s,1H), 7.64(s,1H), 7.61(s,1H), 7.35(s,1H), 4.86(s,2H), 3.87-3.85(d,J=8Hz,2H), 3.75(s,3H), 3.27-3.24(d,1=12Hz,2H), 2.84(m,2H), 2.17(s,1H), 1.83(d,J=12Hz,2H), 1.50-1.41(m,2H), 1.23 (s,3H), 0.73(s,2H) and 0.48(s,2H) |
| 98 | ESI+: 507.1<br>NMR: δ 8.47(s,1H), 7.63(s,1H), 7.60(s,1H), 7.35(s,1H), 4.8(s,2H), 3.8(m,2H), 3.75(s,3H), 2.8(m,2H), 2.17 (m,1H), 1.85(m,3H), 1.4(m,3H), 1.19(m,3H), 0.93(m,3H), 0.71(s,2H) and 0.48(s,2H) |
| 99 | ESI+: 537<br>NMR: δ 8.39(brs,1H), 7.63(s,1H), 7.46(s,1H), 7.34(s,1H),7.35(s,1H), 4.86(s,2H), 4.33-4.30(m,1H), 3.80-3.75(m,5H), 3.45-3.42(m,2H), 3.18-3.13(m,2H), 2.84-2.81(d,J = 12Hz,2H), 1.89-1.84(m,3H), 1.58-1.5s(d,J=12Hz,4H), 1.19(s,3H), 0.63(s,2H) and 0.37(s,2H) |

[Table 67]

| Ex | Data |
|---|---|
| 100 | ESI+:539.1<br>NMR:59.50(brs,1H),7.72(s,1H),7.67(s,1H),7.37(s,1H),7.3 0-7.28(m,3H),5.22(s,2H),4.96-4.91(m,6H),3.76 (s,3H),1. 42(s,2H),1.26-1.24(m,2H) |
| 101 | ESI+:532.3<br>NMR:δ9.56(brs,1H),7.72(s,1H),7.66(s,1H),7.36(s,1H),5.2 8(s,2H),4.89(s,2H),3.76(s,3H),2.50-2.44(m,6H), 1.36(rn,2 H), 1.20(m,2H) |
| 102 | ESI+:567.4<br>NMR:δ9.46(brs,1H),7.74(s,1H),7.68(s,1H),7.61(s,1H),7.5 2(d,J=7.6Hz,1H),7.38(s,1H),7.30(d,J=8.4Hz, 1H),6.72(s,1 H),5.47(t,*J*=6.0Hz,1H),5.29(s,2H),4.93(s,2H),4.55(d,*J*=5. 6Hz,2H),3.77(s,3H),1.46-1.44(m, 2H),1.28-1.25(m,2H) |
| 103 | ESI+:515.2<br>NMR:δ7.66(s,1H),7.43-7.40(m,3H),7.37(s,1H),7.21-7.10 (m,3H),5.16(s,2H),4.90(s,2H),3.76(s,3H),1.14(m, 2H),1.0 1 (m,2H) |
| 104a | ESI+:564.2 |
| 104b | ESI+:551.3<br>NMR:δ8.46(brs,1H),7.68(s,1H),7.60(s,1H),7.39(s,1H),5.2 8(s,2H),4.89(s,2H),4.86-4.83(m,1H)/4.06(t, J=5.6Hz,2H), 3.69-3.65(m,2H),2.52(s,3H),2.43(s,3H),1.15(s,3H),0.67 (m,2H),0.45-0.43(m,2H) |
| 105 | ESI+:552.0<br>NMR:δ8.42(s,2H),7.83(dd,J=8.4and2.4Hz,1H),7.61(s,1H), 7.49(d,J=8.0Hz,1H),5.27(s,2H),5.08(s,2H),2.53 (s,3H),2. 43(m,3H),1.17(s,3H),0.71-0.68(m,2H),0.47-0.44(m,2H) |

[Table 68]

| Ex | Data |
|---|---|
| 106 | ESI+ :585.3<br>NMR:δ8.44(brs,1H),7.61(s,1H),7.31-7.30(m,3H),5.31(s,2 H),5.26(s,2H),4.97-4.96(m,4H),2.69-2.66(m, 4H),1.75-1. 74(m,4H),1.13(s,3H),0.67-0.64(m,2H),0.44-0.43(m,2H) |
| 107 | ESI+:539.3<br>NMR:δ8.44(brs,1H),7.61(s,2H),6.05(s,1H),5.25(s,2H),5.1 9(s,2H),3.78(s,3H),2.79-2.72(m,1H),2.57(s,3H), 1.14(s,3 H),1.10(d,J=6.8Hz,6H),0.67-0.64(m,2H),0.44-0.41(m,2 H) |

(continued)

| Ex | Data |
|---|---|
| 108 | ESI+:529.1<br>NMR:68.54(brs,1H),8.41(s,1H),7.74(s,1H),7.67(s,1H),7.6 0(s,1H),7.38(s,1H),5.25(m,2H),5.03(s,2H),4.90 (s,4H),3. 76(s,3H),1.13(s,3H),0.67-0.64(s,2H),0.44-0.41(m,2H) |
| 109 | ESI+:529.0<br>NMR:δ8.38(s,2H),7.78(d,J=7.6Hz,1H),7.64(s,1H),7.59(s, 1H),7.40(d,J=8.0Hz,1H),7.35(5,1H),5.29(s,2H), 5.05(s,2 H),4.89(s,2H),4.82(s,2H),3.75(s,3H),1.12(s,3H),0.67-0.6 3 (m, 2H), 0.43 - 0.40 (m, 2 H) |
| 110 | ESI+ :546.0<br>NMR:δ8.38(brs,1H),7.66(s,1H),7.60(s,1H),7.37(s,1H),7.3 1(d,*J*=6.8Hz,1H),7.26(d,*J*=10.0Hz,1H),5.22(s, 2H),4.96(s, 2H),4.90-4.89(m,4H),3.76(s,3H),1.12(s,3H),0.66-0.64 (m,2H),0.43-0.40(m,2H) |
| 111 | ESI+:558.4<br>NMR:δ8.38(brs,1H),7.66(s,1H),7.58(s,1H),7.37(s,1H),7.1 3(s,1H),7.03(s,1H),5.11(s,2H),4.96(s,2H),4.89 (s,2H),4.8 6(s,2H),3.77(s,3H),3.76(s,3H),1.11(s,3H),0.68-0.64(m,2 H),0.43-0.41(m,2H) |

[Table 69]

| Ex | Data |
|---|---|
| 112 | ESI+:542.2<br>NMR:δ8.30(brs,1H),7.68(s,1H),7.53(s,1H),7.39-7.38(m,2 H),7.34(s,2H),6.26-6.24(m,1H),4.99-4.88(m, 6H),3.77(s, 3H),1.80(d,*J*=7.2Hz,3H),1.04(s,3H),0.61-0.52(m,2H),0.3 8-0.35(m,2H) |
| 113 | ESI+: 542.4<br>NMR:δ8.36(brs,1H),7.66(s,1H),7.60(s,1H),7.37(s,1H),7.1 9(s,1H),6.98(s,1H),5.19(s,2H),4.94-4.92(m, 4H),4.83(s,2 H),3.76(s,3H),2.34(s,3H),1.10(s,3H),0.64-0.61(m,2H),0. 42-0.39(m,2H) |
| 114 | ESI+:542.4<br>NMR:δ8.35(brs,1H),7.68(s,1H),7.58(s,1H),7.33(s,1H),7.1 0(s,1H),7.07(s,1H),5.18(s,2H),4.95-4.92(m, 6H),3.76(s,3 H),2.17(s,3H),1.12(s,3H),0.64-0.61(m,2H),0.42-0.40(m, 2H) |
| 115 | ESI+:542.4<br>NMR:68.36(brs,1H),7.67(s,1H),7.58(s,1H),7.37(s,1H),7.0 5(d,*J*=7.6Hz,1H),6.97(d,*J*=8.0Hz,1H),5.18(s, 2H),5.02-4.9 5(m,4H),4.92(s,2H),3.76(s,3H),2.17(s,3H),1.10(s,3H),0. 64-0.63(m,2H),0.41-0.39(m,2H) |
| 116 | ESI+:555.0<br>NMR:δ11.09(brs,1H),8.29(brs,1H),7.69(s,1H),7.55(s,1H), 7.50(s,1H),7.40(s,1H),7.31(d,*J*=8.4Hz,1H), 7.04(dd,*J*=1.6 &8.4Hz,1H),6.25(s,1H),5.24-5.21(m,3H),4.94(s,2H),4.58 -4.56(m,2H),3.77(s,3H),1.09(s, 3H),0.63-0.61(m,2H),0.4 0-0.37(m,2H) |
| 117 | ESI+:516.8<br>NMR:δ8.53(s,1H),8.47(s,1H),8.36(s,1H),7.70(s,1H),7.66 (s,1H),7.59(s,1H),7.38(s,1H),5.33(t,*J*=6.0Hz, 1H),5.24(s, 2H),4.91(s,2H),4.49(s,2H),3.76(s,3H),1.13(s,3H),0.67-0. 64(m,2H),0.43-0.40(m,2H) |

[Table 70]

| Ex | Data |
|---|---|
| 118 | ESI+: 569.2<br>NMR:δ8.42(brs,1H),7.87(s,1H),7.67(s,1H),7.58(s,1H),7.4 7(dd,*J*=8.0&2.0Hz,1H),7.38(s,1H),7.30(d, *J*=8.0Hz,1H),5. 24(s,2H),4.92(s,2H),3.77(s,3H),3.51(t,*J*=6.8Hz,2H),3.00 (s,3H),2.95(t,*J*=6.8Hz,2H),1.11(s, 3H),0.65-0.63(m,2H), 0.42-0.39(m,2H) |
| 119 | ESI+:494.2<br>NMR:δ8.44(brs,1H),7.63(s,1H),7.59-7.58(m,1H),7.35(s,1 H),4.86(s,2H),4.27(s,2H),3.75(s,3H),2.03(s,6H), 1.17(s,3 H),0.71-0.68(m,2H),0.47-0.44(m,2H) |

(continued)

| Ex | Data |
|---|---|
| 120 | ESI+:494.2<br>NMR:δ8.39(brs,1H),7.66(s,1H),7.62(s,1H),7.36(s,1H),4.8 6(s,2H),4.35-4.34(m,2H),4.26-4.25(m,2H), 3.92-3.88(m,2 H),3.75(s,3H),2.04-2.00(m,2H),1.32(s,3H),1.19(s,3H),0. 72-0.69(m,2H),0.48-0.45(m,2H) |
| 121 | ESI+:533.9<br>NMR:δ8.39(brs,1H),7.67(s,1H),7.59(s,1H),7.39(s,1H),7.2 1-7.16(m,2H),6.94-6.90(m,1H),5.17(s,2H),4.92(s, 2H),3. 78(s,3H),3.76(s,3H),1.18(s,3H),0.66-0.63(m,2H),0.42-0. 39(m,2H) |
| 122 | ESI+:564.0<br>NMR:δ8.38(brs,1H),7.67(s,1H),7.59(s,1H),7.39(s,1H),7.2 1-7.17(m,2H),6.94-6.91(m,1H),5.16(s,2H), 4.92-4.88(m,3 H),4.01-3.98(m,2H),3.76(s,3H),3.73-3.69(m,2H),1.11(s, 3H),0.66-0.63(m,2H)and0.42-0.40 (m,2H) |
| 123 | ESI+:551.9<br>NMR:δ8.35(brs,1H),7.67(s,1H),7.59(s,1H),7.38(s,1H),7.2 7-7.21(m,2H),5.15(s,2H),4.90(s,2H),3.90(m,3H), 3.76(s,3 H),1.12(s,3H),0.66-0.64(m,2H),0.43-0.40(m,2H) |

[Table 71]

| Ex | Data |
|---|---|
| 124 | ESI+:550.9<br>NMR:δ8.31(brs,1H),7.66(s,1H),7.59(5,1H),7.37(s,1H),7.1 6-7.11(m,1H),6.54-6.50(m,1H),5.54-5.53(m,1H), 5.16(s,2 H),4.91(s,2H),3.75(s,3H),2.56(d,*J*=8.8Hz,3H),1.11(s,3H), 0.66-0.63(m,2H),0.42-0.40(m,2H) |
| 125 | ESI+:551.0<br>NMR:δ8.40(brs,1H),7.67(s,1H),7.58(s,1H),7.38(s,1H),7.0 5-6.97(m,2H),5.30-5.29(m,1H),5.06(s,2H),4.90(s, 2H),3. 76(s,3H),2.87-2.85(m,3H),1.12(s,3H),0.67-0.64(m,2H), 0.43-0.40(m,2H) |
| 126 | ESI+:562.0<br>NMR:68.35(brs,1H),7.94-7.92(m,1H),7.71-7.66(m,2H),7. 59(s,1H),7.39-7.34(m,2H),5.25(s,2H),4.91(s,2H), 3.84(s, 3H),3.76(s,3H),1.11(s,3H),0.66-0.63(m,2H),0.42-0.40 (m,2H) |
| 127 | ESI+:684.3<br>NMR:δ8.39(brs,1H),7.66(s,1H),7.59(s,1H),7.55-7.49(m,2 H), 7.38- 7.3 2 (m, 2 H), 5.2 2 (s, 2H),4.9 1 (s, 2 H), 3.76(s,4H), 3. 68- 3.59(m,4H),3.32-3.29 (m, 1H),3.22-3.16(m,2H), 1.99-1.88(m,1H),1.12(s,3H),0.74-0.73(m, 4H),0.66-0.63(m,2 H),0.43-0.40(m,2H) |
| 128 | ESI+:597.0<br>NMR:δ8.22-8.21(m,2H),7.88(dd,*J*=8.4&2.4Hz,1H),7.66(s, 1H),7.59(s,1H),7.37(s,1H),7.26-7.20(m,2H), 7.18-7.14 (m,2H),7.06(d,*J*=8.8Hz,1H),5.17(s,2H),4.89(s,2H),3.75 (s,3H),1.13(s,3H),0.67-0.64(m,2H), 0.44-0.41(m,2H) |
| 129 | ESI+:547.2<br>NMR:δ8.44(brs,1H),7.65(s,1H),7.60(s,1H),7.36(s,1H),5.3 7(s,2H),4.88(s,2H),3.75(s,3H),2.71-2.60(m,4H), 1.75-1.7 3(m,4H),1.15(s,3H),0.69-0.66(m,2H),0.46-0.43(m,2H) |

[Table 72]

| Ex | Data |
|---|---|
| 130 | ESI+:541.0<br>NMR:δ10.41(brs,1H),8.39(s,1H),7.67(s,1H),7.58(s,1H),7. 38(s,1H),7.22(s,2H),6.80(d,J=8.4Hz,1H),5.13(s, 2H),4.91 (s,2H),3.76(s,3H),3.44(s,2H),1.12(s,3H),0.66-0.63(m,2 H),0.43-0.40(m,2H) |

(continued)

| Ex | Data |
|---|---|
| 131 | ESI+:541.0<br>NMR:δ10.37(s,1H),8.38(s,1H),7.67(s,1H),7.59(s,1H),7.39 (s,1H),7.19(d,*J*=7.6Hz,1H),6.95(d,*J*=7.6Hz,1H), 7.81(s,1 H),5.16(s,2H),4.92(s,2H),3.77(s,3H),3.44(m,2H),1.13(s, 3H),0.67-0.64(m,2H),0.43-0.40(m,2H) |
| 132 | ESI+:500.0<br>NMR:38.39(s,1H),7.62(s,1H),7.53(s,1H),7.35(s,1H),7.26-7.22(m,2H),7.19-7.16(m,3H),4.86(s,2H),4.11(t, *J*=7.2Hz, 2H),3.77(s,3H),3.02(t,*J*=7.2Hz,2H),1.13(s,3H),0.67-0.64 (m,2H),0.45-0.43(m,2H) |
| 133 | ESI+ :545.4<br>NMR:δ8.36(s,1H),8.02(s,1H),7.61(s,1H),7.58(s,1H),7.33 (s,1H),5.25(s,2H),4.88(s,2H),3.75(s,6H),2.26(s, 3H),2.15 (s,3H),1.12(s,3H),0.66-0.63(m,2H),0.43-0.40(m,2H) |
| 134 | ESI+:531.4<br>NMR:δ10.90(brs,1H),8.47(brs,1H),7.65(s,1H),7.60(s,1H), 7.54(s,1H),7.37(s,1H),5.20(s,2H),4.90(s,2H),3.76 (s,3H), 2.02 (s,3H), 1.92 (5, 3H), 1.12 (5,3 H),O. 65-0.63(m,2 H),0.42-0.40(m,2H) |
| 135 | ESI+:518.0<br>NMR:δ8.39(brs,1H),7.67(s,1H),7.58(s,1H),7.38(s,1H),7.3 0-7.28(m,1H),7.26-7.22(m,1H),7.15-7.11(m,1H), 5.15(s,2 H),4.91(s,2H),3.76(s,3H),2.19(s,3H),1.12(s,3H),0.66-0.6 3(m,2H)and0.43-0.40(m,2H) |
| 136 | ESI+:489.9<br>NMR:δ8.34(brs,1H),7.75-7.72(m,2H),7.67(s,1H),7.63(s,1 H),7.48(t,J=8.8Hz,2H),7.38(s,1H),4.89(s,2H),3.76 (s,3H), 1.16(s,3H),0.71-0.67(m,2H),0.45-0.42(m,2H) |

Industrial Applicability

The tetrahydrothienopyrimidinesulfonamide compound or a salt thereof of the present invention is useful as the PARG inhibitor, and can be used as the active ingredient of a pharmaceutical composition, for example, a pharmaceutical composition for treating cancer, a pharmaceutical composition for enhancing an effect of an anticancer agent, and/or a pharmaceutical composition for enhancing an effect of radiotherapy.

## Claims

1. A compound of Formula (I) or a salt thereof.

[Chem. 1]

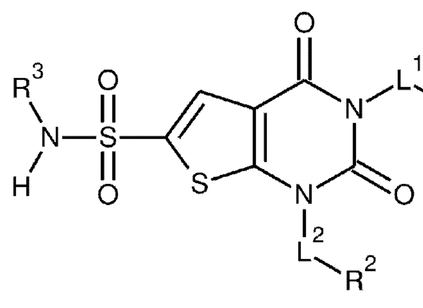

(I)

(In Formula,

$L^1$ is $C_{1-4}$ alkylene;
$L^2$ is a single bond, $C_{1-4}$ alkylene, $C_{2-4}$ alkenylene, $C_{2-4}$ alkynylene, or $C_{1-4}$ alkylene-O-;
$R^1$ is an aromatic heterocyclic group which may have a substituent, or a non-aromatic heterocyclic group which may have a substituent;
$R^2$ is -H, amino, $C_{3-6}$ cycloalkyl which may have a substituent, an aryl which may have a substituent, a non-aromatic heterocyclic group which may have a substituent, or an aromatic heterocyclic group which may have a substituent; and

$R^3$ is $C_{3-6}$ cycloalkyl which may be substituted with one or more groups selected from the group consisting of $C_{1-4}$ alkyl and cyano, or $C_{1-4}$ alkyl.)

**2.** The compound or a salt thereof according to claim 1, wherein $R^1$ is an aromatic heterocyclic group which may have a substituent, and $R^3$ is $C_{3-6}$ cycloalkyl which may be substituted with $C_{1-4}$ alkyl, or $C_{1-4}$ alkyl.

**3.** The compound or a salt thereof according to claim 1 or 2, wherein $R^3$ is 1-methylcyclopropyl.

**4.** The compound or a salt thereof according to any one of claims 1 to 3, wherein $L^1$ is methylene.

**5.** The compound or a salt thereof according to any one of claims 1 to 4, wherein $L^2$ is methylene.

**6.** The compound or a salt thereof according to any one of claims 1 to 5, wherein $R^1$ is 1-methylpyrazol-4-yl or 2-methylthiazol-5-yl.

**7.** The compound or a salt thereof according to claim 6, wherein $R^1$ is 1-methylpyrazol-4-yl.

**8.** The compound or a salt thereof according to any one of claims 1 to 7, wherein $R^2$ is a non-aromatic heterocyclic group which may have a substituent or an aromatic heterocyclic group which may have a substituent.

**9.** 1-((2,4-Dimethylthiazol-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,

1-((1,3-Dihydroisobenzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,
1,3-Bis((1-methyl-1H-pyrazol-4-yl)methyl)-N-(1-methylcyclopropyl)-2,4-dioxo-l,2,3,4-tetrahydrothieno[2,3-d]pyrimidine-6-sulfonamide or a salt thereof,
1-((1H-Indazol-6-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,
1-((Imidazo[1,5-a]pyridin-6-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof,
1-((2-(Methoxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof, or
1-((2-(hydroxymethyl)benzofuran-5-yl)methyl)-3-((1-methyl-1H-pyrazol-4-yl)methyl)-N-(1-methylcyclopropyl)-2,4-dioxo-1,2,3,4-tetrahydrothieno[2,3-d]pyrimidine-6-sulfonamide or a salt thereof.

**10.** A pharmaceutical composition comprising the compound or a salt thereof according to claim 1 or 9, and a pharmaceutically acceptable excipient.

**11.** The pharmaceutical composition according to claim 10, wherein the pharmaceutical composition is a pharmaceutical composition for treating cancer, a pharmaceutical composition for enhancing an effect of an anticancer agent, or a pharmaceutical composition for enhancing an effect of radiotherapy.

**12.** Use of the compound or a salt thereof according to claim 1 or 9 for manufacturing a pharmaceutical composition for treating cancer, a pharmaceutical composition for enhancing an effect of an anticancer agent, or a pharmaceutical composition for enhancing an effect of radiotherapy.

**13.** Use of the compound or a salt thereof according to claim 1 or 9 for treating cancer, enhancing an effect of an anticancer agent, or enhancing an effect of radiotherapy.

**14.** The compound or a salt thereof according to claim 1 or 9 for treating cancer, enhancing an effect of an anticancer agent, or enhancing an effect of radiotherapy.

**15.** A method for treating cancer, enhancing an effect of an anticancer agent, or enhancing an effect of radiotherapy by administering an effective amount of the compound or a salt thereof according to claim 1 or 9 to a subject.

**INTERNATIONAL SEARCH REPORT**

International application No.
**PCT/JP2021/047849**

**A. CLASSIFICATION OF SUBJECT MATTER**

***C07D 495/04***(2006.01)i; ***A61K 31/519***(2006.01)i; ***A61K 31/5377***(2006.01)i; ***A61K 31/5383***(2006.01)i; ***A61P 35/00***(2006.01)i; ***C07D 519/00***(2006.01)i
FI: C07D495/04 105Z; A61P35/00; A61K31/519; C07D519/00 301; A61K31/5377; C07D519/00 CSP; A61K31/5383

According to International Patent Classification (IPC) or to both national classification and IPC

**B. FIELDS SEARCHED**

Minimum documentation searched (classification system followed by classification symbols)

C07D495/04; A61K31/519; A61K31/5377; A61K31/5383; A61P35/00; C07D519/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MARPAT (STN)

**C. DOCUMENTS CONSIDERED TO BE RELEVANT**

| Category* | Citation of document, with indication, where appropriate, of the relevant passages | Relevant to claim No. |
|---|---|---|
| A | JAMES, Dominic I. et al. First-in-Class Chemical Probes against Poly (ADP-ribose) Glycohydrolase (PARC) Inhibit DNA Repair with Differential Pharmacology to Olaparib. ACS Chem. Biol. 30 September 2016, vol. 11, pp. 3179-3190<br>entire text, all drawings | 1-15 |
| A | WASZKOWYCZ, Bohdan et al. Cell-Active Small Molecule Inhibitors of the DNA-Damage Repair Enzyme Poly (ADP-ribose) Glycohydrolase (PARG): Discovery and Optimization of Orally Bioavailable Quinazolinedione Sulfonamides. J. Med. Chem. 07 November 2018, vol. 61, pp. 10767-10792<br>entire text, all drawings | 1-15 |
| A | JP 2017-537137 A (CANCER RESEARCH TECHNOLOGY LIMITED) 14 December 2017 (2017-12-14)<br>entire text, all drawings | 1-15 |
| A | WO 2016/130271 A1 (DUQUESNE UNIVERSITY OF THE HOLY GHOST) 18 August 2016 (2016-08-18)<br>entire text, all drawings | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2022** | **15 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2021/047849**

| Patent document cited in search report | Publication date (day/month/year) | Patent family member(s) | Publication date (day/month/year) |
|---|---|---|---|
| JP 2017-537137 A | 14 December 2017 | US 2018/0016242 A1 | |
| | | WO 2016/092326 A1 | |
| | | EP 3230273 A1 | |
| | | KR 10-2017-0093967 A | |
| | | CN 107207467 A | |
| WO 2016/130271 A1 | 18 August 2016 | US 2016/0229857 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018237296 A
- WO 2016092326 A
- WO 2017075056 A
- WO 2017147161 A
- WO 2018171698 A


### Non-patent literature cited in the description


- *Prog. Med,* 1985, vol. 5, 2157-2161
- Pharmaceutical Research and Development. Drug Design. Hirokawa Publishing Company, 1990, vol. 7, 163-198
- **P. G. M. WUTS ; T. W. GREENE.** Greene's Protective Groups in Organic Synthesis. 2006
- Greene's Protective Groups in Organic Synthesis. 2006